# EUROPEAN PATENT APPLICATION

(11) **EP 2 168 603 A1**
(43) Date of publication of application: **31.03.2010**
(21) Application number: 10150655.8
(22) Date of filing: 20.10.2004
(51) Int. Cl.: A61K 48/00, C12N 15/861, C07K 14/525

(54) **Therapeutic regimen for treating cancer**

(30) Priority: 14.11.2003 US 520127 P
(62) Divisional of application: 04795677.6
(71) Applicant: GenVec, Inc., Gaithersburg, MD 20878 (US)
(72) Inventor: Kessler, Paul D., Hagerstown, MD 21742 (US); Rasmussen, Henrik S., Arnold, MD 21012 (US); Chu, Karen W., White Plains, NY 10607 (US)
(74) Representative: Boehme, Ulrich

(57) **Abstract**

The invention provides a method for treating cancer in a human comprising (a) administering to the human a dose of a pharmaceutical composition comprising (i) a pharmaceutically acceptable carrier and (ii) an adenoviral vector comprising a nucleic acid sequence encoding a human TNF-α and operably linked to a promoter, wherein the dose comprises about 4 x 10⁷ to about 4 x 10¹² particle units (pu) of adenoviral vector, at least once in a therapeutic period comprising up to about 10 weeks, (b) administering a dose of ionizing radiation to the human over the duration of the therapeutic period, and (c) administering a dose of one or more chemotherapeutics to the human over the duration of the therapeutic period, whereby the cancer in the human is treated.

SEQUENCE LISTING

<110>  GENVEC, INC.

<120>  THERAPEUTIC REGIMEN FOR TREATING CANCER

<130>  A 6 287 g

<150>  US 60/520,127
<151>  2003-11-14

<160>  2

<170>  PatentIn version 3.2

<210>  1
<211>  32798
<212>  DNA
<213>  Artificial

<220>
<223>  Synthetic construct

<400>  1
catcatcata atatacctta ttttggattg aagccaatat gataatgagg gggtggagtt      60

tgtgacgtgg cgcggggcgt gggaacgggg cgggtgacgt agtagtgtgg cggaagtgtg     120

atgttgcaag tgtggcggaa cacatgtaag cgccggatgt ggtaaaagtg acgtttttgg     180

tgtgcgccgg tgtatacggg aagtgacaat tttcgcgcgg ttttaggcgg atgttgtagt     240

aaatttgggc gtaaccaagt aatatttggc cattttcgcg ggaaaactga ataagaggaa     300

gtgaaatctg aataattctg tgttactcat agcgcgtaat atttgtctag ggccgcgggg     360

actttgaccg tttacgtgga gactcgccca ggtgtttttc tcaggtgttt tccgcgttcc     420

gggtcaaagt tggcgtttta ttattatagt cagctctaga ctagatgcgc cgacccggaa     480

acgccatata aggagcagga aggatccccc gccggaacag accttatttg ggcagcgcct     540

tatatggagt ggcccaatat ggccctgccg cttccggctc tgggaggagg ggcgagcggg     600

ggttggggcg ggggcaagct gggaactcca ggcgcctggc ccgggaggcc actgctgctg     660

ttccaatact aggctttcca ggagcctgag cgctcgcgat gccggagcgg gtcgcagggt     720

ggaggtgccc accactcttg gatgggaggg cttcacgtca ctccgggtcc tcccggccgg     780

tccttccata ttagggcttc ctgcttccca tatatggcca tgtacgtcac ggcggaggcg     840

ggcccgtgct gttccagacc cttgaaatag aggccgattc ggggagtcgc gagagatccc     900

agcgcgcaga acttggggag ccgccgccgc gattcgccgc cgccgccagc ttccggtcga     960

ggaactgaaa aaccagaaag ttaactgggt aagtttagtc tttttgtctt ttatttcagg    1020

tcccggatcc ggtggtggtg caaatcaaag aactgctcct cagtggatgt tgcctttact    1080

tctaggcctg tacggaagtg ttacttctgc tctaaaagct gcggaattgt acccgcggcc    1140

gcaaagggaa caaaagctgg gtaccgagct cgaatggggg gggggggggg gtactgaccc    1200

acggctccac cctctctccc ctggaaagga caccatgagc actgaaagca tgatccggga    1260

cgtggagctg gccgaggagg cgctccccaa gaagacaggg gggccccagg gctccaggcg    1320

gtgcttgttc ctcagcctct tctccttcct gatcgtggca ggcgccacca cgctcttctg    1380

cctgctgcac tttggagtga tcggccccca gagggaagag tcccccaggg acctctctct 1440
aatcagccct ctggcccagg cagtcagatc atcttctcga accccgagtg acaagcctgt 1500
agcccatgtt gtagcaaacc ctcaagctga ggggcagctc cagtggctga accgccgggc 1560
caatgccctc ctggccaatg gcgtggagct gagagataac cagctggtgg tgccatcaga 1620
gggcctgtac ctcatctact cccaggtcct cttcaagggc caaggctgcc cctccaccca 1680
tgtgctcctc acccacacca tcagccgcat cgccgtctcc taccagacca aggtcaacct 1740
cctctctgcc atcaagagcc cctgccagag ggagacccca gagggggctg aggccaagcc 1800
ctggtatgag cccatctatc tgggaggggt cttccagctg gagaagggtg accgactcag 1860
cgctgagatc aatcggcccg actatctcga ctttgccgag tctgggcagg tctactttgg 1920
gatcattgcc ctgtgaggag gacgaacatc caaccttccc aaacgcctcc cctgccccaa 1980
tccctttatt accccctcct tcagacaccc tcaacctctt ctggctcaaa aagagaattg 2040
ggggcttagg gtcggaaccc aagcttgata tcgaattcct gcagcccggg ggatccacta 2100
gttctagagc ggccgcgggg atccagacat gataagatac attgatgagt ttggacaaac 2160
cacaactaga atgcagtgaa aaaaatgctt tatttgtgaa atttgtgatg ctattgcttt 2220
atttgtaacc attataagct gcaataaaca agttaacaac aacaattgca ttcattttat 2280
gtttcaggtt cagggggagg tgtgggaggt tttttcggat cctctagagt cgactagagt 2340
ggaaggtgct gaggtacgat gagacccgca ccaggtgcag accctgcgag tgtggcggta 2400
aacatattag gaaccagcct gtgatgctgg atgtgaccga ggagctgagg cccgatcact 2460
tggtgctggc ctgcacccgc gctgagtttg gctctagcga tgaagataca gattgaggta 2520
ctgaaatgtg tgggcgtggc ttaagggtgg gaaagaatat ataaggtggg ggtcttatgt 2580
agttttgtat ctgttttgca gcagccgccg ccgccatgag caccaactcg tttgatggaa 2640
gcattgtgag ctcatatttg acaacgcgca tgcccccatg ggccggggtg cgtcagaatg 2700
tgatgggctc cagcattgat ggtcgccccg tcctgcccgc aaactctact accttgacct 2760
acgagaccgt gtctggaacg ccgttggaga ctgcagcctc cgccgccgct tcagccgctg 2820
cagccaccgc ccgcgggatt gtgactgact ttgctttcct gagcccgctt gcaagcagtg 2880
cagcttcccg ttcatccgcc cgcgatgaca agttgacggc tcttttggca caattggatt 2940
ctttgacccg ggaacttaat gtcgtttctc agcagctgtt ggatctgcgc cagcaggttt 3000
ctgccctgaa ggcttcctcc cctcccaatg cggtttaaaa cataaataaa aaaccagact 3060
ctgtttggat ttggatcaag caagtgtctt gctgtcttta tttaggggtt ttgcgcgcgc 3120
ggtaggcccg ggaccagcgg tctcggtcgt tgagggtcct gtgtattttt tccaggacgt 3180
ggtaaaggtg actctggatg ttcagataca tgggcataag cccgtctctg gggtggaggt 3240
agcaccactg cagagcttca tgctgcgggg tggtgttgta gatgatccag tcgtagcagg 3300
agcgctgggc gtggtgccta aaaatgtctt tcagtagcaa gctgattgcc aggggcaggc 3360
ccttggtgta agtgtttaca aagcggttaa gctgggatgg gtgcatacgt ggggatatga 3420
gatgcatctt ggactgtatt tttaggttgg ctatgttccc agccatatcc ctccggggat 3480

tcatgttgtg cagaaccacc agcacagtgt atccggtgca cttgggaaat ttgtcatgta 3540
gcttagaagg aaatgcgtgg aagaacttgg agacgccctt gtgacctcca agattttcca 3600
tgcattcgtc cataatgatg gcaatgggcc cacgggcggc ggcctgggcg aagatatttc 3660
tgggatcact aacgtcatag ttgtgttcca ggatgagatc gtcataggcc atttttacaa 3720
agcgcgggcg gagggtgcca gactgcggta taatggttcc atccggccca ggggcgtagt 3780
taccctcaca gatttgcatt tcccacgctt tgagttcaga tggggggatc atgtctacct 3840
gcggggcgat gaagaaaacg gtttccgggg taggggagat cagctgggaa gaaagcaggt 3900
tcctgagcag ctgcgactta ccgcagccgg tgggcccgta aatcacacct attaccgggt 3960
gcaactggta gttaagagag ctgcagctgc cgtcatccct gagcaggggg gccacttcgt 4020
taagcatgtc cctgactcgc atgttttccc tgaccaaatc cgccagaagg cgctcgccgc 4080
ccagcgatag cagttcttgc aaggaagcaa agtttttcaa cggtttgaga ccgtccgccg 4140
taggcatgct tttgagcgtt tgaccaagca gttccaggcg gtcccacagc tcggtcacct 4200
gctctacggc atctcgatcc agcatatctc ctcgtttcgc gggttggggc ggctttcgct 4260
gtacggcagt agtcggtgct cgtccagacg ggccagggtc atgtctttcc acgggcgcag 4320
ggtcctcgtc agcgtagtct gggtcacggt gaaggggtgc gctccgggct gcgcgctggc 4380
cagggtgcgc ttgaggctgg tcctgctggt gctgaagcgc tgccggtctt cgccctgcgc 4440
gtcggccagg tagcatttga ccatggtgtc atagtccagc ccctccgcgg cgtggccctt 4500
ggcgcgcagc ttgcccttgg aggaggcgcc gcacgagggg cagtgcagac ttttgagggc 4560
gtagagcttg ggcgcgagaa ataccgattc cggggagtag gcatccgcgc cgcaggcccc 4620
gcagacggtc tcgcattcca cgagccaggt gagctctggc cgttcggggt caaaaaccag 4680
gtttccccca tgctttttga tgcgtttctt acctctggtt tccatgagcc ggtgtccacg 4740
ctcggtgacg aaaaggctgt ccgtgtcccc gtatacagac ttgagaggcc tgtcctcgag 4800
cggtgttccg cggtcctcct cgtatagaaa ctcggaccac tctgagacaa aggctcgcgt 4860
ccaggccagc acgaaggagg ctaagtggga ggggtagcgg tcgttgtcca ctagggggtc 4920
cactcgctcc agggtgtgaa gacacatgtc gccctcttcg gcatcaagga aggtgattgg 4980
tttgtaggtg taggccacgt gaccgggtgt tcctgaaggg gggctataaa agggggtggg 5040
ggcgcgttcg tcctcactct cttccgcatc gctgtctgcg agggccagct gttggggtga 5100
gtactccctc tgaaaagcgg gcatgacttc tgcgctaaga ttgtcagttt ccaaaaacga 5160
ggaggatttg atattcacct ggcccgcggt gatgcctttg agggtggccg catccatctg 5220
gtcagaaaag acaatctttt tgttgtcaag cttggtggca aacgacccgt agagggcgtt 5280
ggacagcaac ttggcgatgg agcgcagggt ttggtttttg tcgcgatcgg cgcgctcctt 5340
ggccgcgatg tttagctgca cgtattcgcg cgcaacgcac cgccattcgg gaaagacggt 5400
ggtgcgctcg tcgggcacca ggtgcacgcg ccaaccgcgg ttgtgcaggg tgacaaggtc 5460
aacgctggtg gctacctctc cgcgtaggcg ctcgttggtc cagcagaggc ggccgccctt 5520
gcgcgagcag aatggcggta gggggtctag ctgcgtctcg tccggggggt ctgcgtccac 5580

ggtaaagacc ccgggcagca ggcgcgcgtc gaagtagtct atcttgcatc cttgcaagtc 5640
tagcgcctgc tgccatgcgc gggcggcaag cgcgcgctcg tatgggttga gtgggggacc 5700
ccatggcatg gggtgggtga gcgcggaggc gtacatgccg caaatgtcgt aaacgtagag 5760
gggctctctg agtattccaa gatatgtagg gtagcatctt ccaccgcgga tgctggcgcg 5820
cacgtaatcg tatagttcgt gcgagggagc gaggaggtcg ggaccgaggt tgctacgggc 5880
gggctgctct gctcggaaga ctatctgcct gaagatggca tgtgagttgg atgatatggt 5940
tggacgctgg aagacgttga agctggcgtc tgtgagacct accgcgtcac gcacgaagga 6000
ggcgtaggag tcgcgcagct tgttgaccag ctcggcggtg acctgcacgt ctagggcgca 6060
gtagtccagg gtttccttga tgatgtcata cttatcctgt cccttttttt tccacagctc 6120
gcggttgagg acaaactctt cgcggtcttt ccagtactct tggatcggaa acccgtcggc 6180
ctccgaacgg taagagccta gcatgtagaa ctggttgacg gcctggtagg cgcagcatcc 6240
cttttctacg ggtagcgcgt atgcctgcgc ggccttccgg agcgaggtgt gggtgagcgc 6300
aaaggtgtcc ctgaccatga ctttgaggta ctggtatttg aagtcagtgt cgtcgcatcc 6360
gccctgctcc cagagcaaaa agtccgtgcg ctttttggaa cgcggatttg gcagggcgaa 6420
ggtgacatcg ttgaagagta tctttcccgc gcgaggcata aagttgcgtg tgatgcggaa 6480
gggtcccggc acctcggaac ggttgttaat tacctgggcg gcgagcacga tctcgtcaaa 6540
gccgttgatg ttgtggccca caatgtaaag ttccaagaag cgcgggatgc ccttgatgga 6600
aggcaatttt ttaagttcct cgtaggtgag ctcttcaggg gagctgagcc cgtgctctga 6660
aagggcccag tctgcaagat gagggttgga agcgacgaat gagctccaca ggtcacgggc 6720
cattagcatt tgcaggtggt cgcgaaaggt cctaaactgg cgacctatgg ccattttttc 6780
tggggtgatg cagtagaagg taagcgggtc ttgttcccag cggtcccatc caaggttcgc 6840
ggctaggtct cgcgcggcag tcactagagg ctcatctccg ccgaacttca tgaccagcat 6900
gaagggcacg agctgcttcc caaaggcccc catccaagta taggtctcta catcgtaggt 6960
gacaaagaga cgctcggtgc gaggatgcga gccgatcggg aagaactgga tctcccgcca 7020
ccaattggag gagtggctat tgatgtggtg aaagtagaag tccctgcgac gggccgaaca 7080
ctcgtgctgg cttttgtaaa aacgtgcgca gtactggcag cggtgcacgg gctgtacatc 7140
ctgcacgagg ttgacctgac gaccgcgcac aaggaagcag agtgggaatt tgagcccctc 7200
gcctggcggg tttggctggt ggtcttctac ttcggctgct tgtccttgac cgtctggctg 7260
ctcgagggga gttacggtgg atcggaccac cacgccgcgc gagcccaaag tccagatgtc 7320
cgcgcgcggc ggtcggagct tgatgacaac atcgcgcaga tgggagctgt ccatggtctg 7380
gagctcccgc ggcgtcaggt caggcgggag ctcctgcagg tttacctcgc atagacgggt 7440
cagggcgcgg gctagatcca ggtgatacct aatttccagg ggctggttgg tggcggcgtc 7500
gatggcttgc aagaggccgc atccccgcgg cgcgactacg gtaccgcgcg gcgggcggtg 7560
ggccgcgggg gtgtccttgg atgatgcatc taaaagcggt gacgcgggcg agcccccgga 7620
ggtagggggg gctccggacc cgccgggaga gggggcaggg gcacgtcggc gccgcgcgcg 7680

ggcaggagct ggtgctgcgc gcgtaggttg ctggcgaacg cgacgacgcg gcggttgatc 7740
tcctgaatct ggcgcctctg cgtgaagacg acgggcccgg tgagcttgag cctgaaagag 7800
agttcgacag aatcaatttc ggtgtcgttg acggcggcct ggcgcaaaat ctcctgcacg 7860
tctcctgagt tgtcttgata ggcgatctcg gccatgaact gctcgatctc ttcctcctgg 7920
agatctccgc gtccggctcg ctccacggtg gcggcgaggt cgttggaaat gcgggccatg 7980
agctgcgaga aggcgttgag gcctccctcg ttccagacgc ggctgtagac cacgccccct 8040
tcggcatcgc gggcgcgcat gaccacctgc gcgagattga gctccacgtg ccgggcgaag 8100
acggcgtagt ttcgcaggcg ctgaaagagg tagttgaggg tggtggcggt gtgttctgcc 8160
acgaagaagt acataaccca gcgtcgcaac gtggattcgt tgatatcccc caaggcctca 8220
aggcgctcca tggcctcgta gaagtccacg gcgaagttga aaaactggga gttgcgcgcc 8280
gacacggtta actcctcctc cagaagacgg atgagctcgg cgacagtgtc gcgcacctcg 8340
cgctcaaagg ctacaggggc ctcttcttct tcttcaatct cctcttccat aagggcctcc 8400
ccttcttctt cttctggcgg cggtggggga ggggggacac ggcggcgacg acggcgcacc 8460
gggaggcggt cgacaaagcg ctcgatcatc tccccgcggc gacggcgcat ggtctcggtg 8520
acggcgcggc cgttctcgcg ggggcgcagt tggaagacgc cgcccgtcat gtcccggtta 8580
tgggttggcg gggggctgcc atgcggcagg gatacggcgc taacgatgca tctcaacaat 8640
tgttgtgtag gtactccgcc gccgagggac ctgagcgagt ccgcatcgac cggatcggaa 8700
aacctctcga gaaaggcgtc taaccagtca cagtcgcaag gtaggctgag caccgtggcg 8760
ggcggcagcg ggcggcggtc ggggttgttt ctggcggagg tgctgctgat gatgtaatta 8820
aagtaggcgg tcttgagacg gcggatggtc gacagaagca ccatgtcctt gggtccggcc 8880
tgctgaatgc gcaggcggtc ggccatgccc caggcttcgt tttgacatcg gcgcaggtct 8940
ttgtagtagt cttgcatgag cctttctacc ggcacttctt cttctccttc ctcttgtcct 9000
gcatctcttg catctatcgc tgcggcggcg gcggagtttg gccgtaggtg gcgccctctt 9060
cctcccatgc gtgtgacccc gaagcccctc atcggctgaa gcagggctag gtcggcgaca 9120
acgcgctcgg ctaatatggc ctgctgcacc tgcgtgaggg tagactggaa gtcatccatg 9180
tccacaaagc ggtggtatgc gcccgtgttg atggtgtaag tgcagttggc cataacggac 9240
cagttaacgg tctggtgacc cggctgcgag agctcggtgt acctgagacg cgagtaagcc 9300
ctcgagtcaa atacgtagtc gttgcaagtc cgcaccaggt actggtatcc caccaaaaag 9360
tgcggcggcg gctggcggta gaggggccag cgtagggtgg ccggggctcc gggggcgaga 9420
tcttccaaca taaggcgatg atatccgtag atgtacctgg acatccaggt gatgccggcg 9480
gcggtggtgg aggcgcgcgg aaagtcgcgg acgcggttcc agatgttgcg cagcggcaaa 9540
aagtgctcca tggtcgggac gctctggccg gtcaggcgcg cgcaatcgtt gacgctctag 9600
ccgtgcaaaa ggagagcctg taagcgggca ctcttccgtg gtctggtgga taaattcgca 9660
agggtatcat ggcggacgac cggggttcga gccccgtatc cggccgtccg ccgtgatcca 9720
tgcggttacc gcccgcgtgt cgaacccagg tgtgcgacgt cagacaacgg gggagtgctc 9780

cttttggctt ccttccaggc gcggcggctg ctgcgctagc ttttttggcc actggccgcg 9840
cgcagcgtaa gcggttaggc tggaaagcga aagcattaag tggctcgctc cctgtagccg 9900
gagggttatt ttccaagggt tgagtcgcgg gacccccggt tcgagtctcg gaccggccgg 9960
actgcggcga acgggggttt gcctccccgt catgcaagac cccgcttgca aattcctccg 10020
gaaacaggga cgagcccctt ttttgctttt cccagatgca tccggtgctg cggcagatgc 10080
gcccccctcc tcagcagcgg caagagcaag agcagcggca gacatgcagg gcaccctccc 10140
ctcctcctac cgcgtcagga ggggcgacat ccgcggttga cgcggcagca gatggtgatt 10200
acgaaccccc gcggcgccgg gcccggcact acctggactt ggaggagggc gagggcctgg 10260
cgcggctagg agcgccctct cctgagcggt acccaagggt gcagctgaag cgtgatacgc 10320
gtgaggcgta cgtgccgcgg cagaacctgt ttcgcgaccg cgagggagag gagcccgagg 10380
agatgcggga tcgaaagttc cacgcagggc gcgagctgcg gcatggcctg aatcgcgagc 10440
ggttgctgcg cgaggaggac tttgagcccg acgcgcgaac cgggattagt cccgcgcgcg 10500
cacacgtggc ggccgccgac ctggtaaccg catacgagca gacggtgaac caggagatta 10560
actttcaaaa aagctttaac aaccacgtgc gtacgcttgt ggcgcgcgag gaggtggcta 10620
taggactgat gcatctgtgg gactttgtaa gcgcgctgga gcaaaaccca aatagcaagc 10680
cgctcatggc gcagctgttc cttatagtgc agcacagcag ggacaacgag gcattcaggg 10740
atgcgctgct aaacatagta gagcccgagg gccgctggct gctcgatttg ataaacatcc 10800
tgcagagcat agtggtgcag gagcgcagct tgagcctggc tgacaaggtg gccgccatca 10860
actattccat gcttagcctg ggcaagtttt acgcccgcaa gatataccat accccttacg 10920
ttcccataga caaggaggta aagatcgagg ggttctacat gcgcatggcg ctgaaggtgc 10980
ttaccttgag cgacgacctg ggcgtttatc gcaacgagcg catccacaag gccgtgagcg 11040
tgagccggcg gcgcgagctc agcgaccgcg agctgatgca cagcctgcaa agggccctgg 11100
ctggcacggg cagcggcgat agagaggccg agtcctactt tgacgcgggc gctgacctgc 11160
gctgggcccc aagccgacgc gccctggagg cagctggggc cggacctggg ctggcggtgg 11220
cacccgcgcg cgctggcaac gtcggcggcg tggaggaata tgacgaggac gatgagtacg 11280
agccagagga cggcgagtac taagcggtga tgtttctgat cagatgatgc aagacgcaac 11340
ggacccggcg gtgcgggcgg cgctgcagag ccagccgtcc ggccttaact ccacggacga 11400
ctggcgccag gtcatggacc gcatcatgtc gctgactgcg cgcaatcctg acgcgttccg 11460
gcagcagccg caggccaacc ggctctccgc aattctggaa gcggtggtcc cggcgcgcgc 11520
aaaccccacg cacgagaagg tgctggcgat cgtaaacgcg ctggccgaaa acagggccat 11580
ccggcccgac gaggccggcc tggtctacga cgcgctgctt cagcgcgtgg ctcgttacaa 11640
cagcggcaac gtgcagacca acctggaccg gctggtgggg gatgtgcgcg aggccgtggc 11700
gcagcgtgag cgcgcgcagc agcagggcaa cctgggctcc atggttgcac taaacgcctt 11760
cctgagtaca cagcccgcca acgtgccgcg gggacaggag gactacacca actttgtgag 11820
cgcactgcgg ctaatggtga ctgagacacc gcaaagtgag gtgtaccagt ctgggccaga 11880

ctattttttc cagaccagta gacaaggcct gcagaccgta aacctgagcc aggctttcaa 11940
aaacttgcag gggctgtggg gggtgcgggc tcccacaggc gaccgcgcga ccgtgtctag 12000
cttgctgacg cccaactcgc gcctgttgct gctgctaata gcgcccttca cggacagtgg 12060
cagcgtgtcc cgggacacat acctaggtca cttgctgaca ctgtaccgcg aggccatagg 12120
tcaggcgcat gtggacgagc atactttcca ggagattaca agtgtcagcc gcgcgctggg 12180
gcaggaggac acgggcagcc tggaggcaac cctaaactac ctgctgacca accggcggca 12240
gaagatcccc tcgttgcaca gtttaaacag cgaggaggag cgcattttgc gctacgtgca 12300
gcagagcgtg agccttaacc tgatgcgcga cggggtaacg cccagcgtgg cgctggacat 12360
gaccgcgcgc aacatggaac cgggcatgta tgcctcaaac cggccgttta tcaaccgcct 12420
aatggactac ttgcatcgcg cggccgccgt gaaccccgag tatttcacca atgccatctt 12480
gaacccgcac tggctaccgc cccctggttt ctacaccggg ggattcgagg tgcccgaggg 12540
taacgatgga ttcctctggg acgacataga cgacagcgtg ttttccccgc aaccgcagac 12600
cctgctagag ttgcaacagc gcgagcaggc agaggcggcg ctgcgaaagg aaagcttccg 12660
caggccaagc agcttgtccg atctaggcgc tgcggccccg cggtcagatg ctagtagccc 12720
atttccaagc ttgatagggt ctcttaccag cactcgcacc acccgcccgc gcctgctggg 12780
cgaggaggag tacctaaaca actcgctgct gcagccgcag cgcgaaaaaa acctgcctcc 12840
ggcatttccc aacaacggga tagagagcct agtggacaag atgagtagat ggaagacgta 12900
cgcgcaggag cacagggacg tgccaggccc gcgcccgccc acccgtcgtc aaaggcacga 12960
ccgtcagcgg ggtctggtgt gggaggacga tgactcggca gacgacagca gcgtcctgga 13020
tttgggaggg agtggcaacc cgtttgcgca ccttcgcccc aggctgggga gaatgtttta 13080
aaaaaaaaaa agcatgatgc aaaataaaaa actcaccaag gccatggcac cgagcgttgg 13140
ttttcttgta ttccccttag tatgcggcgc gcggcgatgt atgaggaagg tcctcctccc 13200
tcctacgaga gtgtggtgag cgcggcgcca gtggcggcgg cgctgggttc tcccttcgat 13260
gctcccctgg acccgccgtt tgtgcctccg cggtacctgc ggcctaccgg ggggagaaac 13320
agcatccgtt actctgagtt ggcaccccta ttcgacacca cccgtgtgta cctggtggac 13380
aacaagtcaa cggatgtggc atccctgaac taccagaacg accacagcaa ctttctgacc 13440
acggtcattc aaaacaatga ctacagcccg ggggaggcaa gcacacagac catcaatctt 13500
gacgaccggt cgcactgggg cggcgacctg aaaaccatcc tgcataccaa catgccaaat 13560
gtgaacgagt tcatgtttac caataagttt aaggcgcggg tgatggtgtc gcgcttgcct 13620
actaaggaca atcaggtgga gctgaaatac gagtgggtgg agttcacgct gcccgagggc 13680
aactactccg agaccatgac catagacctt atgaacaacg cgatcgtgga gcactacttg 13740
aaagtgggca gacagaacgg ggttctggaa agcgacatcg gggtaaagtt tgacacccgc 13800
aacttcagac tggggtttga ccccgtcact ggtcttgtca tgcctggggt atatacaaac 13860
gaagccttcc atccagacat cattttgctg ccaggatgcg gggtggactt cacccacagc 13920
cgcctgagca acttgttggg catccgcaag cggcaaccct tccaggaggg ctttaggatc 13980

acctacgatg atctggaggg tggtaacatt cccgcactgt tggatgtgga cgcctaccag 14040
gcgagcttga aagatgacac cgaacagggc gggggtggcg caggcggcag caacagcagt 14100
ggcagcggcg cggaagagaa ctccaacgcg gcagccgcgg caatgcagcc ggtggaggac 14160
atgaacgatc atgccattcg cggcgacacc tttgccacac gggctgagga gaagcgcgct 14220
gaggccgaag cagcggccga agctgccgcc cccgctgcgc aacccgaggt cgagaagcct 14280
cagaagaaac cggtgatcaa acccctgaca gaggacagca agaaacgcag ttacaaccta 14340
ataagcaatg acagcacctt cacccagtac cgcagctggt accttgcata caactacggc 14400
gaccctcaga ccggaatccg ctcatggacc ctgctttgca ctcctgacgt aacctgcggc 14460
tcggagcagg tctactggtc gttgccagac atgatgcaag accccgtgac cttccgctcc 14520
acgcgccaga tcagcaactt tccggtggtg ggcgccgagc tgttgcccgt gcactccaag 14580
agcttctaca acgaccaggc cgtctactcc caactcatcc gccagtttac ctctctgacc 14640
cacgtgttca atcgctttcc cgagaaccag attttggcgc gcccgccagc ccccaccatc 14700
accaccgtca gtgaaaacgt tcctgctctc acagatcacg ggacgctacc gctgcgcaac 14760
agcatcggag gagtccagcg agtgaccatt actgacgcca gacgccgcac ctgcccctac 14820
gtttacaagg ccctgggcat agtctcgccg cgcgtcctat cgagccgcac tttttgagca 14880
agcatgtcca tccttatatc gcccagcaat aacacaggct ggggcctgcg cttcccaagc 14940
aagatgtttg gcggggccaa gaagcgctcc gaccaacacc cagtgcgcgt gcgcgggcac 15000
taccgcgcgc cctggggcgc gcacaaacgc ggccgcactg ggcgcaccac cgtcgatgac 15060
gccatcgacg cggtggtgga ggaggcgcgc aactacacgc ccacgccgcc accagtgtcc 15120
acagtggacg cggccattca gaccgtggtg cgcggagccc ggcgctatgc taaaatgaag 15180
agacggcgga ggcgcgtagc acgtcgccac cgccgccgac ccggcactgc cgcccaacgc 15240
gcggcggcgg ccctgcttaa ccgcgcacgt cgcaccggcc gacgggcggc catgcgggcc 15300
gctcgaaggc tggccgcggg tattgtcact gtgcccccca ggtccaggcg acgagcggcc 15360
gccgcagcag ccgcggccat tagtgctatg actcagggtc gcaggggcaa cgtgtattgg 15420
gtgcgcgact cggttagcgg cctgcgcgtg cccgtgcgca cccgcccccc gcgcaactag 15480
attgcaagaa aaaactactt agactcgtac tgttgtatgt atccagcggc ggcggcgcgc 15540
aacgaagcta tgtccaagcg caaaatcaaa gaagagatgc tccaggtcat cgcgccggag 15600
atctatggcc ccccgaagaa ggaagagcag gattacaagc cccgaaagct aaagcgggtc 15660
aaaaagaaaa agaaagatga tgatgatgaa cttgacgacg aggtggaact gctgcacgct 15720
accgcgccca ggcgacgggt acagtggaaa ggtcgacgcg taaaacgtgt tttgcgaccc 15780
ggcaccaccg tagtctttac gcccggtgag cgctccaccc gcacctacaa gcgcgtgtat 15840
gatgaggtgt acggcgacga ggacctgctt gagcaggcca acgagcgcct cggggagttt 15900
gcctacggaa agcggcataa ggacatgctg gcgttgccgc tggacgaggg caacccaaca 15960
cctagcctaa agcccgtaac actgcagcag gtgctgcccg cgcttgcacc gtccgaagaa 16020
aagcgcggcc taaagcgcga gtctggtgac ttggcaccca ccgtgcagct gatggtaccc 16080

aagcgccagc gactggaaga tgtcttggaa aaaatgaccg tggaacctgg gctggagccc 16140
gaggtccgcg tgcggccaat caagcaggtg gcgccgggac tgggcgtgca gaccgtggac 16200
gttcagatac ccactaccag tagcaccagt attgccaccg ccacagaggg catggagaca 16260
caaacgtccc cggttgcctc agcggtggcg gatgccgcgg tgcaggcggt cgctgcggcc 16320
gcgtccaaga cctctacgga ggtgcaaacg gacccgtgga tgtttcgcgt ttcagccccc 16380
cggcgcccgc gcggttcgag gaagtacggc gccgccagcg cgctactgcc cgaatatgcc 16440
ctacatcctt ccattgcgcc tacccccggc tatcgtggct acacctaccg ccccagaaga 16500
cgagcaacta cccgacgccg aaccaccact ggaacccgcc gccgccgtcg ccgtcgccag 16560
cccgtgctgg ccccgatttc cgtgcgcagg gtggctcgcg aaggaggcag gaccctggtg 16620
ctgccaacag cgcgctacca ccccagcatc gtttaaaagc cggtctttgt ggttcttgca 16680
gatatggccc tcacctgccg cctccgtttc ccggtgccgg gattccgagg aagaatgcac 16740
cgtaggaggg gcatggccgg ccacggcctg acgggcggca tgcgtcgtgc gcaccaccgg 16800
cggcggcgcg cgtcgcaccg tcgcatgcgc ggcggtatcc tgcccctcct tattccactg 16860
atcgccgcgg cgattggcgc cgtgcccgga attgcatccg tggccttgca ggcgcagaga 16920
cactgattaa aaacaagttg catgtggaaa aatcaaaata aaaagtctgg actctcacgc 16980
tcgcttggtc ctgtaactat tttgtagaat ggaagacatc aactttgcgt ctctggcccc 17040
gcgacacggc tcgcgcccgt tcatgggaaa ctggcaagat atcggcacca gcaatatgag 17100
cggtggcgcc ttcagctggg gctcgctgtg gagcggcatt aaaaatttcg gttccaccgt 17160
taagaactat ggcagcaagg cctggaacag cagcacaggc cagatgctga gggataagtt 17220
gaaagagcaa aatttccaac aaaaggtggt agatggcctg gcctctggca ttagcggggt 17280
ggtggacctg gccaaccagg cagtgcaaaa taagattaac agtaagcttg atccccgccc 17340
tcccgtagag gagcctccac cggccgtgga gacagtgtct ccagaggggc gtggcgaaaa 17400
gcgtccgcgc cccgacaggg aagaaactct ggtgacgcaa atagacgagc ctccctcgta 17460
cgaggaggca ctaaagcaag gcctgcccac cacccgtccc atcgcgccca tggctaccgg 17520
agtgctgggc cagcacacac ccgtaacgct ggacctgcct ccccccgccg acacccagca 17580
gaaacctgtg ctgccaggcc cgaccgccgt tgttgtaacc cgtcctagcc gcgcgtccct 17640
gcgccgcgcc gccagcggtc cgcgatcgtt gcggcccgta gccagtggca actggcaaag 17700
cacactgaac agcatcgtgg gtctgggggt gcaatccctg aagcgccgac gatgcttctg 17760
aatagctaac gtgtcgtatg tgtgtcatgt atgcgtccat gtcgccgcca gaggagctgc 17820
tgagccgccg cgcgcccgct ttccaagatg gctacccctt cgatgatgcc gcagtggtct 17880
tacatgcaca tctcgggcca ggacgcctcg gagtacctga gccccgggct ggtgcagttt 17940
gcccgcgcca ccgagacgta cttcagcctg aataacaagt ttagaaaccc cacggtggcg 18000
cctacgcacg acgtgaccac agaccggtcc cagcgtttga cgctgcggtt catccctgtg 18060
gaccgtgagg atactgcgta ctcgtacaag gcgcggttca ccctagctgt gggtgataac 18120
cgtgtgctgg acatggcttc cacgtacttt gacatccgcg gcgtgctgga caggggccct 18180

acttttaagc cctactctgg cactgcctac aacgccctgg ctcccaaggg tgccccaaat 18240
ccttgcgaat gggatgaagc tgctactgct cttgaaataa acctagaaga agaggacgat 18300
gacaacgaag acgaagtaga cgagcaagct gagcagcaaa aaactcacgt atttgggcag 18360
gcgccttatt ctggtataaa tattacaaag gagggtattc aaataggtgt cgaaggtcaa 18420
acacctaaat atgccgataa aacatttcaa cctgaacctc aaataggaga atctcagtgg 18480
tacgaaactg aaattaatca tgcagctggg agagtcctta aaaagactac cccaatgaaa 18540
ccatgttacg gttcatatgc aaaacccaca aatgaaaatg gagggcaagg cattcttgta 18600
aagcaacaaa atggaaagct agaaagtcaa gtggaaatgc aatttttctc aactactgag 18660
gcgaccgcag gcaatggtga taacttgact cctaaagtgg tattgtacag tgaagatgta 18720
gatatagaaa ccccagacac tcatatttct tacatgccca ctattaagga aggtaactca 18780
cgagaactaa tgggccaaca atctatgccc aacaggccta attacattgc ttttagggac 18840
aattttattg gtctaatgta ttacaacagc acgggtaata tgggtgttct ggcgggccaa 18900
gcatcgcagt tgaatgctgt tgtagatttg caagacagaa acacagagct ttcataccag 18960
cttttgcttg attccattgg tgatagaacc aggtactttt ctatgtggaa tcaggctgtt 19020
gacagctatg atccagatgt tagaattatt gaaaatcatg gaactgaaga tgaacttcca 19080
aattactgct ttccactggg aggtgtgatt aatacagaga ctcttaccaa ggtaaaacct 19140
aaaacaggtc aggaaaatgg atgggaaaaa gatgctacag aattttcaga taaaaatgaa 19200
ataagagttg gaaataattt tgccatggaa atcaatctaa atgccaacct gtggagaaat 19260
ttcctgtact ccaacatagc gctgtatttg cccgacaagc taaagtacag tccttccaac 19320
gtaaaaattt ctgataaccc aaacacctac gactacatga acaagcgagt ggtggctccc 19380
gggttagtgg actgctacat taaccttgga gcacgctggt cccttgacta tatggacaac 19440
gtcaacccat ttaaccacca ccgcaatgct ggcctgcgct accgctcaat gttgctgggc 19500
aatggtcgct atgtgccctt ccacatccag gtgcctcaga agttctttgc cattaaaaac 19560
ctccttctcc tgccgggctc atacacctac gagtggaact tcaggaagga tgttaacatg 19620
gttctgcaga gctccctagg aaatgaccta agggttgacg gagccagcat taagtttgat 19680
agcatttgcc tttacgccac cttcttcccc atggcccaca acaccgcctc cacgcttgag 19740
gccatgctta gaaacgacac caacgaccag tcctttaacg actatctctc cgccgccaac 19800
atgctctacc ctatacccgc caacgctacc aacgtgccca tatccatccc ctcccgcaac 19860
tgggcggctt tccgcggctg ggccttcacg cgccttaaga ctaaggaaac cccatcactg 19920
ggctcgggct acgaccctta ttacacctac tctggctcta taccctacct agatggaacc 19980
ttttacctca accacacctt taagaaggtg gccattacct ttgactcttc tgtcagctgg 20040
cctggcaatg accgcctgct tacccccaac gagtttgaaa ttaagcgctc agttgacggg 20100
gagggttaca acgttgccca gtgtaacatg accaaagact ggttcctggt acaaatgcta 20160
gctaactaca acattggcta ccagggcttc tatatcccag agagctacaa ggaccgcatg 20220
tactccttct ttagaaactt ccagcccatg agccgtcagg tggtggatga tactaaatac 20280

aaggactacc aacaggtggg catcctacac caacacaaca actctggatt tgttggctac 20340
cttgccccca ccatgcgcga aggacaggcc taccctgcta acttccccta tccgcttata 20400
ggcaagaccg cagttgacag cattacccag aaaaagtttc tttgcgatcg caccctttgg 20460
cgcatcccat tctccagtaa ctttatgtcc atgggcgcac tcacagacct gggccaaaac 20520
cttctctacg ccaactccgc ccacgcgcta gacatgactt ttgaggtgga tcccatggac 20580
gagcccaccc ttctttatgt tttgtttgaa gtctttgacg tggtccgtgt gcaccggccg 20640
caccgcggcg tcatcgaaac cgtgtacctg cgcacgccct tctcggccgg caacgccaca 20700
acataaagaa gcaagcaaca tcaacaacag ctgccgccat gggctccagt gagcaggaac 20760
tgaaagccat tgtcaaagat cttggttgtg ggccatattt tttgggcacc tatgacaagc 20820
gctttccagg ctttgtttct ccacacaagc tcgcctgcgc catagtcaat acggccggtc 20880
gcgagactgg gggcgtacac tggatggcct ttgcctggaa cccgcactca aaaacatgct 20940
acctctttga gccctttggc ttttctgacc agcgactcaa gcaggtttac cagtttgagt 21000
acgagtcact cctgcgccgt agcgccattg cttcttcccc cgaccgctgt ataacgctgg 21060
aaaagtccac ccaaagcgta caggggccca actcggccgc ctgtggacta ttctgctgca 21120
tgtttctcca cgcctttgcc aactggcccc aaactcccat ggatcacaac cccaccatga 21180
accttattac cggggtaccc aactccatgc tcaacagtcc ccaggtacag cccaccctgc 21240
gtcgcaacca ggaacagctc tacagcttcc tggagcgcca ctcgccctac ttccgcagcc 21300
acagtgcgca gattaggagc gccacttctt tttgtcactt gaaaaacatg taaaaataat 21360
gtactagaga cactttcaat aaaggcaaat gcttttattt gtacactctc gggtgattat 21420
ttacccccac ccttgccgtc tgcgccgttt aaaaatcaaa ggggttctgc cgcgcatcgc 21480
tatgcgccac tggcagggac acgttgcgat actggtgttt agtgctccac ttaaactcag 21540
gcacaaccat ccgcggcagc tcggtgaagt tttcactcca caggctgcgc accatcacca 21600
acgcgtttag caggtcgggc gccgatatct tgaagtcgca gttggggcct ccgccctgcg 21660
cgcgcgagtt gcgatacaca gggttgcagc actggaacac tatcagcgcc gggtggtgca 21720
cgctggccag cacgctcttg tcggagatca gatccgcgtc caggtcctcc gcgttgctca 21780
gggcgaacgg agtcaacttt ggtagctgcc ttcccaaaaa gggcgcgtgc ccaggctttg 21840
agttgcactc gcaccgtagt ggcatcaaaa ggtgaccgtg cccggtctgg gcgttaggat 21900
acagcgcctg cataaaagcc ttgatctgct taaaagccac ctgagccttt gcgccttcag 21960
agaagaacat gccgcaagac ttgccggaaa actgattggc cggacaggcc gcgtcgtgca 22020
cgcagcacct tgcgtcggtg ttggagatct gcaccacatt tcggccccac cggttcttca 22080
cgatcttggc cttgctagac tgctccttca gcgcgcgctg cccgttttcg ctcgtcacat 22140
ccatttcaat cacgtgctcc ttatttatca taatgcttcc gtgtagacac ttaagctcgc 22200
cttcgatctc agcgcagcgg tgcagccaca acgcgcagcc cgtgggctcg tgatgcttgt 22260
aggtcacctc tgcaaacgac tgcaggtacg cctgcaggaa tcgccccatc atcgtcacaa 22320
aggtcttgtt gctggtgaag gtcagctgca acccgcggtg ctcctcgttc agccaggtct 22380

tgcatacggc cgccagagct tccacttggt caggcagtag tttgaagttc gcctttagat 22440
cgttatccac gtggtacttg tccatcagcg cgcgcgcagc ctccatgccc ttctcccacg 22500
cagacacgat cggcacactc agcgggttca tcaccgtaat ttcactttcc gcttcgctgg 22560
gctcttcctc ttcctcttgc gtccgcatac cacgcgccac tgggtcgtct tcattcagcc 22620
gccgcactgt gcgcttacct cctttgccat gcttgattag caccggtggg ttgctgaaac 22680
ccaccatttg tagcgccaca tcttctcttt cttcctcgct gtccacgatt acctctggtg 22740
atggcgggcg ctcgggcttg ggagaagggc gcttcttttt cttcttgggc gcaatggcca 22800
aatccgccgc cgaggtcgat ggccgcgggc tgggtgtgcg cggcaccagc gcgtcttgtg 22860
atgagtcttc ctcgtcctcg gactcgatac gccgcctcat ccgctttttt gggggcgccc 22920
ggggaggcgg cggcgacggg gacggggacg acacgtcctc catggttggg ggacgtcgcg 22980
ccgcaccgcg tccgcgctcg ggggtggttt cgcgctgctc ctcttcccga ctggccattt 23040
ccttctccta taggcagaaa aagatcatgg agtcagtcga gaagaaggac agcctaaccg 23100
ccccctctga gttcgccacc accgcctcca ccgatgccgc caacgcgcct accaccttcc 23160
ccgtcgaggc acccccgctt gaggaggagg aagtgattat cgagcaggac ccaggttttg 23220
taagcgaaga cgacgaggac cgctcagtac caacagagga taaaaagcaa gaccaggaca 23280
acgcagaggc aaacgaggaa caagtcgggc ggggggacga aaggcatggc gactacctag 23340
atgtgggaga cgacgtgctg ttgaagcatc tgcagcgcca gtgcgccatt atctgcgacg 23400
cgttgcaaga gcgcagcgat gtgcccctcg ccatagcgga tgtcagcctt gcctacgaac 23460
gccacctatt ctcaccgcgc gtacccccca aacgccaaga aaacggcaca tgcgagccca 23520
acccgcgcct caacttctac cccgtatttg ccgtgccaga ggtgcttgcc acctatcaca 23580
tctttttcca aaactgcaag atacccctat cctgccgtgc caaccgcagc cgagcggaca 23640
agcagctggc cttgcggcag ggcgctgtca tacctgatat cgcctcgctc aacgaagtgc 23700
caaaaatctt tgagggtctt ggacgcgacg agaagcgcgc ggcaaacgct ctgcaacagg 23760
aaaacagcga aaatgaaagt cactctggag tgttggtgga actcgagggt gacaacgcgc 23820
gcctagccgt actaaaacgc agcatcgagg tcacccactt tgcctacccg gcacttaacc 23880
taccccccaa ggtcatgagc acagtcatga gtgagctgat cgtgcgccgt gcgcagcccc 23940
tggagaggga tgcaaatttg caagaacaaa cagaggaggg cctacccgca gttggcgacg 24000
agcagctagc gcgctggctt caaacgcgcg agcctgccga cttggaggag cgacgcaaac 24060
taatgatggc cgcagtgctc gttaccgtgg agcttgagtg catgcagcgg ttctttgctg 24120
acccggagat gcagcgcaag ctagaggaaa cattgcacta cacctttcga cagggctacg 24180
tacgccaggc ctgcaagatc tccaacgtgg agctctgcaa cctggtctcc taccttggaa 24240
ttttgcacga aaaccgcctt gggcaaaacg tgcttcattc cacgctcaag ggcgaggcgc 24300
gccgcgacta cgtccgcgac tgcgtttact tatttctatg ctacacctgg cagacggcca 24360
tgggcgtttg gcagcagtgc ttggaggagt gcaacctcaa ggagctgcag aaactgctaa 24420
agcaaaactt gaaggaccta tggacggcct tcaacgagcg ctccgtggcc gcgcacctgg 24480

cggacatcat tttccccgaa cgcctgctta aaaccctgca acagggtctg ccagacttca 24540
ccagtcaaag catgttgcag aactttagga actttatcct agagcgctca ggaatcttgc 24600
ccgccacctg ctgtgcactt cctagcgact ttgtgcccat taagtaccgc gaatgccctc 24660
cgccgctttg gggccactgc taccttctgc agctagccaa ctaccttgcc taccactctg 24720
acataatgga agacgtgagc ggtgacggtc tactggagtg tcactgtcgc tgcaacctat 24780
gcaccccgca ccgctccctg gtttgcaatt cgcagctgct taacgaaagt caaattatcg 24840
gtacctttga gctgcagggt ccctcgcctg acgaaaagtc cgcggctccg gggttgaaac 24900
tcactccggg gctgtggacg tcggcttacc ttcgcaaatt tgtacctgag gactaccacg 24960
cccacgagat taggttctac gaagaccaat cccgcccgcc aaatgcggag cttaccgcct 25020
gcgtcattac ccagggccac attcttggcc aattgcaagc catcaacaaa gcccgccaag 25080
agtttctgct acgaaaggga cggggggttt acttggaccc ccagtccggc gaggagctca 25140
acccaatccc cccgccgccg cagccctatc agcagcagcc gcgggccctt gcttcccagg 25200
atggcaccca aaaagaagct gcagctgccg ccgccaccca cggacgagga ggaatactgg 25260
gacagtcagg cagaggaggt tttggacgag gaggaggagg acatgatgga agactgggag 25320
agcctagacg aggaagcttc cgaggtcgaa gaggtgtcag acgaaacacc gtcaccctcg 25380
gtcgcattcc cctcgccggc gccccagaaa tcggcaaccg gttccagcat ggctacaacc 25440
tccgctcctc aggcgccgcc ggcactgccc gttcgccgac ccaaccgtag atgggacacc 25500
actggaacca gggccggtaa gtccaagcag ccgccgccgt tagcccaaga gcaacaacag 25560
cgccaaggct accgctcatg gcgcgggcac aagaacgcca tagttgcttg cttgcaagac 25620
tgtgggggca acatctcctt cgcccgccgc tttcttctct accatcacgg cgtggccttc 25680
ccccgtaaca tcctgcatta ctaccgtcat ctctacagcc catactgcac cggcggcagc 25740
ggcagcggca gcaacagcag cggccacaca gaagcaaagg cgaccggata gcaagactct 25800
gacaaagccc aagaaatcca cagcggcggc agcagcagga ggaggagcgc tgcgtctggc 25860
gcccaacgaa cccgtatcga cccgcgagct tagaaacagg atttttccca ctctgtatgc 25920
tatatttcaa cagagcaggg gccaagaaca agagctgaaa ataaaaaaca ggtctctgcg 25980
atccctcacc cgcagctgcc tgtatcacaa aagcgaagat cagcttcggc gcacgctgga 26040
agacgcggag gctctcttca gtaaatactg cgcgctgact cttaaggact agtttcgcgc 26100
cctttctcaa atttaagcgc gaaaactacg tcatctccag cggccacacc cggcgccagc 26160
acctgtcgtc agcgccatta tgagcaagga aattcccacg ccctacatgt ggagttacca 26220
gccacaaatg ggacttgcgg ctggagctgc ccaagactac tcaacccgaa taaactacat 26280
gagcgcggga ccccacatga tatcccgggt caacggaatc cgcgcccacc gaaaccgaat 26340
tctcttggaa caggcggcta ttaccaccac acctcgtaat aaccttaatc cccgtagttg 26400
gcccgctgcc ctggtgtacc aggaaagtcc cgctcccacc actgtggtac ttcccagaga 26460
cgcccaggcc gaagttcaga tgactaactc aggggcgcag cttgcgggcg gctttcgtca 26520
cagggtgcgg tcgcccgggc agggtataac tcacctgaca atcagagggc gaggtattca 26580

gctcaacgac gagtcggtga gctcctcgct tggtctccgt ccggacggga catttcagat 26640
cggcggcgcc ggccgctctt cattcacgcc tcgtcaggca atcctaactc tgcagacctc 26700
gtcctctgag ccgcgctctg gaggcattgg aactctgcaa tttattgagg agtttgtgcc 26760
atcggtctac tttaacccct tctcgggacc tcccggccac tatccggatc aatttattcc 26820
taactttgac gcggtaaagg actcggcgga cggctacgac tgaatgttaa gtggagaggc 26880
agagcaactg cgcctgaaac acctggtcca ctgtcgccgc cacaagtgct ttgcccgcga 26940
ctccggtgag ttttgctact ttgaattgcc cgaggatcat atcgagggcc cggcgcacgg 27000
cgtccggctt accgcccagg gagagcttgc ccgtagcctg attcgggagt ttacccagcg 27060
ccccctgcta gttgagcggg acaggggacc ctgtgttctc actgtgattt gcaactgtcc 27120
taaccctgga ttacatcaag atctttgttg ccatctctgt gctgagtata ataaatacag 27180
aaattaaaat atactggggc tcctatcgcc atcctgtaaa cgccaccgtc ttcacccgcc 27240
caagcaaacc aaggcgaacc ttacctggta cttttaacat ctctccctct gtgatttaca 27300
acagtttcaa cccagacgga gtgagtctac gagagaacct ctccgagctc agctactcca 27360
tcagaaaaaa caccaccctc cttacctgcc gggaacgtac gagtgcgtca ccggccgctg 27420
caccacacct accgcctgac cgtaaaccag actttttccg gacagacctc aataactctg 27480
tttaccagaa caggaggtga gcttagaaaa cccttagggt attaggccaa aggcgcagct 27540
actgtggggt ttatgaacaa ttcaagcaac tctacgggct attctaattc aggtttctct 27600
agaaatggac ggaattatta cagagcagcg cctgctagaa agacgcaggg cagcggccga 27660
gcaacagcgc atgaatcaag agctccaaga catggttaac ttgcaccagt gcaaaagggg 27720
tatcttttgt ctggtaaagc aggccaaagt cacctacgac agtaatacca ccggacaccg 27780
ccttagctac aagttgccaa ccaagcgtca gaaattggtg gtcatggtgg gagaaaagcc 27840
cattaccata actcagcact cggtagaaac cgaaggctgc attcactcac cttgtcaagg 27900
acctgaggat ctctgcaccc ttattaagac cctgtgcggt ctcaaagatc ttattccctt 27960
taactaataa aaaaaaataa taaagcatca cttacttaaa atcagttagc aaatttctgt 28020
ccagtttatt cagcagcacc tccttgccct cctcccagct ctggtattgc agcttcctcc 28080
tggctgcaaa ctttctccac aatctaaatg gaatgtcagt ttcctcctgt tcctgtccat 28140
ccgcacccac tatcttcatg ttgttgcaga tgaagcgcgc aagaccgtct gaagatacct 28200
tcaaccccgt gtatccatat gacacggaaa ccggtcctcc aactgtgcct tttcttactc 28260
ctccctttgt atcccccaat gggtttcaag agagtccccc tggggtactc tctttgcgcc 28320
tatccgaacc tctagttacc tccaatggca tgcttgcgct caaaatgggc aacggcctct 28380
ctctggacga ggccggcaac cttacctccc aaaatgtaac cactgtgagc ccacctctca 28440
aaaaaaccaa gtcaaacata aacctggaaa tatctgcacc cctcacagtt acctcagaag 28500
ccctaactgt ggctgccgcc gcacctctaa tggtcgcggg caacacactc accatgcaat 28560
cacaggcccc gctaaccgtg cacgactcca aacttagcat tgccacccaa ggacccctca 28620
cagtgtcaga aggaaagcta gccctgcaaa catcaggccc cctcaccacc accgatagca 28680

gtacccttac tatcactgcc tcaccccctc taactactgc cactggtagc ttgggcattg 28740
acttgaaaga gcccatttat acacaaaatg gaaaactagg actaaagtac ggggctcctt 28800
tgcatgtaac agacgaccta aacactttga ccgtagcaac tggtccaggt gtgactatta 28860
ataatacttc cttgcaaact aaagttactg gagccttggg ttttgattca caaggcaata 28920
tgcaacttaa tgtagcagga ggactaagga ttgattctca aaacagacgc cttatacttg 28980
atgttagtta tccgtttgat gctcaaaacc aactaaatct aagactagga cagggccctc 29040
tttttataaa ctcagcccac aacttggata ttaactacaa caaaggcctt tacttgttta 29100
cagcttcaaa caattccaaa aagcttgagg ttaacctaag cactgccaag gggttgatgt 29160
ttgacgctac agccatagcc attaatgcag gagatgggct tgaatttggt tcacctaatg 29220
caccaaacac aaatcccctc aaaacaaaaa ttggccatgg cctagaattt gattcaaaca 29280
aggctatggt tcctaaacta ggaactggcc ttagttttga cagcacaggt gccattacag 29340
taggaaacaa aaataatgat aagctaactt tgtggaccac accagctcca tctcctaact 29400
gtagactaaa tgcagagaaa gatgctaaac tcactttggt cttaacaaaa tgtggcagtc 29460
aaatacttgc tacagtttca gttttggctg ttaaaggcag tttggctcca atatctggaa 29520
cagttcaaag tgctcatctt attataagat ttgacgaaaa tggagtgcta ctaaacaatt 29580
ccttcctgga cccagaatat tggaacttta gaaatggaga tcttactgaa ggcacagcct 29640
atacaaacgc tgttggattt atgcctaacc tatcagctta tccaaaatct cacggtaaaa 29700
ctgccaaaag taacattgtc agtcaagttt acttaaacgg agacaaaact aaacctgtaa 29760
cactaaccat tacactaaac ggtacacagg aaacaggaga cacaactcca agtgcatact 29820
ctatgtcatt ttcatgggac tggtctggcc acaactacat taatgaaata tttgccacat 29880
cctcttacac tttttcatac attgcccaag aataaagaat cgtttgtgtt atgtttcaac 29940
gtgtttattt ttcaattgcc cgggatcggt gatcaccgat ccagacatga taagatacat 30000
tgatgagttt ggacaaacca caactagaat gcagtgaaaa aaatgcttta tttgtgaaat 30060
ttgtgatgct attgctttat ttgtaaccat tataagctgc aataaacaag ttcccggatc 30120
gcgatccggc ccgaggctgt agccgacgat ggtgcgccag gagagttgtt gattcattgt 30180
ttgcctccct gctgcggttt ttcaccgaag ttcatgccag tccagcgttt ttgcagcaga 30240
aaagccgccg acttcggttt gcggtcgcga gtgaagatcc ctttcttgtt accgccaacg 30300
cgcaatatgc cttgcgaggt cgcaaaatcg gcgaaattcc atacctgttc accgacgacg 30360
gcgctgacgc gatcaaagac gcggtgatac atatccagcc atgcacactg atactcttca 30420
ctccacatgt cggtgtacat tgagtgcagc ccggctaacg tatccacgcc gtattcggtg 30480
atgataatcg gctgatgcag tttctcctgc caggccagaa gttctttttc cagtaccttc 30540
tctgccgttt ccaaatcgcc gctttggaca taccatccgt aataacggtt caggcacagc 30600
acatcaaaga gatcgctgat ggtatcggtg tgagcgtcgc agaacattac attgacgcag 30660
gtgatcggac gcgtcgggtc gagtttacgc gttgcttccg ccagtggcgc gaaatattcc 30720
cgtgcacctt gcggacgggt atccggttcg ttggcaatac tccacatcac cacgcttggg 30780

tggtttttgt cacgcgctat cagctcttta atcgcctgta agtgcgcttg ctgagtttcc 30840
ccgttgactg cctcttcgct gtacagttct ttcggcttgt tgcccgcttc gaaaccaatg 30900
cctaaagaga ggttaaagcc gacagcagca gtttcatcaa tcaccacgat gccatgttca 30960
tctgcccagt cgagcatctc ttcagcgtaa gggtaatgcg aggtacggta ggagttggcc 31020
ccaatccagt ccattaatgc gtggtcgtgc accatcagca cgttatcgaa tcctttgcca 31080
cgcaagtccg catcttcatg acgaccaaag ccagtaaagt agaacggttt gtggttaatc 31140
aggaactgtt cgcccttcac tgccactgac cggatgccga cgcgaagcgg gtagatatca 31200
cactctgtct ggcttttggc tgtgacgcac agttcataga gataaccttc acccggttgc 31260
cagaggtgcg gattcaccac ttgcaaagtc ccgctagtgc cttgtccagt tgcaaccacc 31320
tgttgatccg catcacgcag ttcaacgctg acatcaccat tggccaccac ctgccagtca 31380
acagacgcgt ggttacagtc ttgcgcgaca tgcgtcacca cggtgatatc gtccacccag 31440
gtgttcggcg tggtgtagag cattacgctg cgatggattc cggcatagtt aaagaaatca 31500
tggaagtaag actgcttttt cttgccgttt tcgtcggtaa tcaccattcc cggcgggata 31560
gtctgccagt tcagttcgtt gttcacacaa acggtgatac gtacactttt cccggcaata 31620
acatacggcg tgacatcggc ttcaaatggc gtatagccgc cctgatgctc catcacttcc 31680
tgattattga cccacacttt gccgtaatga gtgaccgcat cgaaacgcag cacgatacgc 31740
tggcctgccc aacctttcgg tataaagact tcgcgctgat accagacgtt gcccgcataa 31800
ttacgaatat ctgcatcggc gaactgatcg ttaaaactgc ctggcacagc aattgcccgg 31860
ctttcttgta acgcgctttc ccaccaacgc tgatcaattc cacagttttc gcgatccaga 31920
ctgaatgccc acaggccgtc gagttttttg atttcacggg ttggggtttc tacaggacgg 31980
accatgcgtt cgacctttct cttctttttt gggcccatga tggcagatcc gtatagtgag 32040
tcgtattagc tggttctttc cgcctcagaa gccatagagc ccaccgcatc cccagcatgc 32100
ctgctattgt cttcccaatc ctcccccttg ctgtcctgcc ccaccccacc ccccagaata 32160
gaatgacacc tactcagaca atgcgatgca atttcctcat tttattagga aaggacagtg 32220
ggagtggcac cttccagggt caaggaaggc acgggggagg ggcaaacaac agatggctgg 32280
caactagaag gcacagtcga ggctgatcag cgagctctag atgcatgctc gagcggccgc 32340
cagtgtgatg gatatctgca gaattccagc acactggcgg ccgttactag tggatccgag 32400
ctcggtaccc ggccgttata acaccactcg acacggcacc agctcaatca gtcacagtgt 32460
aaaaaagggc caagtgcaga gcgagtatat ataggactaa aaaatgacgt aacggttaaa 32520
gtccacaaaa aacacccaga aaaccgcacg cgaacctacg cccagaaacg aaagccaaaa 32580
aacccacaac ttcctcaaat cgtcacttcc gttttcccac gttacgtcac ttcccatttt 32640
aagaaaacta caattcccaa cacatacaag ttactccgcc ctaaaaccta cgtcacccgc 32700
cccgttccca cgccccgcgc cacgtcacaa actccacccc ctcattatca tattggcttc 32760
aatccaaaat aaggtatatt attgatgatg ggtcgtta 32798

<210> 2

<211>  465
<212>  DNA
<213>  Artificial

<220>
<223>  DNA encoding tumor necrosis factor-alpha


<220>
<221>  misc_feature
<223>  SEQ ID NO:2 represents the nucleotides of positions 1469 through
       1933 of SEQ ID NO:1

<400>  2
tcatcttctc gaaccccgag tgacaagcct gtagcccatg ttgtagcaaa ccctcaagct      60

gaggggcagc tccagtggct gaaccgccgg gccaatgccc tcctggccaa tggcgtggag     120

ctgagagata accagctggt ggtgccatca gagggcctgt acctcatcta ctcccaggtc     180

ctcttcaagg gccaaggctg cccctccacc catgtgctcc tcacccacac catcagccgc     240

atcgccgtct cctaccagac caaggtcaac ctcctctctg ccatcaagag cccctgccag     300

agggagaccc cagagggggc tgaggccaag ccctggtatg agcccatcta tctgggaggg     360

gtcttccagc tggagaaggg tgaccgactc agcgctgaga tcaatcggcc cgactatctc     420

gactttgccg agtctgggca ggtctacttt gggatcattg ccctg                     465

## Description

### FIELD OF THE INVENTION

This invention pertains to a method and a composition for treating cancer in a human.

### BACKGROUND OF THE INVENTION

The desire of cancer research is the identification of a therapy effective on one or several different types of cancers. The American Cancer Society, alone, distributed approximately $1 billion last year to cancer researchers working to elucidate the mechanisms of a multitude of cancer types. Yet, despite extensive research into the disease, effective cancer therapeutics remain elusive for the medical community. Clinicians have realized limited success with the current standard therapies: chemotherapy, radiation therapy, and surgery. However, each therapy has inherent limitations. Chemotherapy and radiation therapy cause extensive damage to normal, healthy tissue, despite efforts to target such therapy to abnormal tissue (e.g., tumors). Surgery can be effective in removing masses of cancerous cells; however, even the most talented surgeon cannot ensure complete removal of affected tissue nor are all tumors in an anatomical location amenable to surgical removal. The limitations of existing therapies are reflected in the 60% 5-year relative survival rate for all cancers combined (Cancer Facts & Figures 2001, The American Cancer Society, New York, NY).

Clinicians have looked to the delivery of therapeutic nucleic acid sequences as a possible alternative to existing cancer therapies. The local production of therapeutic agents at biologically-significant levels in target sites *in vivo*, thereby reducing the toxicity to normal tissues, addresses some of the limitations associated with conventional therapy. Numerous genes have been examined for anti-tumor effects. One of the most promising anti-tumor agents is tumor necrosis factor (TNF), in particular TNF-α, which has displayed activity with respect to a number of cancer cell lines. TNF-α is a 17 kDa polypeptide secreted by macrophages and monocytes. TNF-α has been shown to selectively destroy tumor vasculature and activate a myriad of immune cells, as well as induce apoptosis of some tumor cell types (Baher et al., Anticancer Research, 19, 2917-2924 (1999), and Mauceri et al., C. R. Acad. Sci. III, 322, 225-228 (1998)). However, the use of TNF in humans as an anti-cancer agent has been limited by its severe systemic effects, including hypotension and respiratory insufficiency (Mauceri et al., *supra*). In addition, many cancer types are refractory to treatment with TNF-α protein such as, for instance, pancreatic cancer (Brown et al., J. Immunotherapy, 10, 376-378 (1991)), gastric cancer (Muggia, Anticancer Drugs, 3, 211-217 (1992)), metastatic breast cancer (Budd et al., Cancer, 68, 1694-1695 (1991)), and colorectal cancer (Heim et al., Onkologie, 13, 444-447 (1990)).

Accordingly, there remains a need for a composition suitable for use in treating a variety of cancer types in a patient, as well as a method for delivering the composition to treat cancer. In particular, there remains a need for a composition and method that optimizes the local effects of anti-cancer agents, such as TNF, while minimizing toxicity. The invention provides such a composition and method. These and other advantages of the invention, as well as additional inventive features, will be apparent from the description of the invention provided herein.

### BRIEF SUMMARY OF THE INVENTION

The invention provides a method of treating unresectable, locally advanced pancreatic cancer (LAPC) cancer, locally advanced resectable esophageal cancer, and locally advanced rectal cancer in a human. The method comprises (a) administering to the human a dose of a pharmaceutical composition comprising (i) a pharmaceutically acceptable carrier and (ii) an adenoviral vector comprising a nucleic acid sequence encoding a human TNF-α and operably linked to a promoter, wherein the dose comprises about 4 x 10⁷ to about 4 x 10¹² particle units (pu) of adenoviral vector, at least once in a therapeutic period comprising up to about 10 weeks, (b) administering a dose of ionizing radiation to the human over the duration of the therapeutic period, and (c) administering a dose of one or more chemotherapeutics to the human over the duration of the therapeutic period, whereby the cancer in the human is treated.

The invention also provides a method of treating glioma in a human. The method comprises (a) administering to the human a dose of a pharmaceutical composition comprising (i) a pharmaceutically acceptable carrier and (ii) an adenoviral vector comprising a nucleic acid sequence encoding a human TNF-α and operably linked to a promoter, wherein the dose comprises about 4 x 10⁷ to about 4 x 10¹² particle units (pu) of adenoviral vector, at least once in a therapeutic period comprising up to about 10 weeks, and (b) administering a dose of ionizing radiation to the human over the duration of the therapeutic period, whereby the glioma in the human is treated.

### BRIEF DESCRIPTION OF THE DRAWINGS

Figure 1 is a schematic representation of an unmodified adenoviral vector genome.

Figure 2 is a schematic representation of an adenoviral vector containing a modified adenoviral genome and genetic elements in accordance with one embodiment of the invention.

Figure 3A is an end-on view of a solid tumor with an illustration of a preferred pattern for multiple applications of a single dose of pharmaceutical composition to the solid tumor. Figure 3B is a side-view of a solid tumor with an illustration of a preferred pattern for multiple applications of a single dose of pharmaceutical composition to the solid tumor.

Figure 4 is an end-on view of a soft tissue sarcoma with an illustration of a preferred pattern for multiple applications of a single dose of pharmaceutical composition to the soft tissue sarcoma.

### DETAILED DESCRIPTION OF THE INVENTION

The invention provides a method of treating cancer in a human (e.g., a patient in need of such treatment). The method preferably involves the delivery of TNF-α to a tumor, preferably directly to the tumor, in combination with chemotherapy and radiation therapy, whereby the size of the tumor is reduced. In particular, the method comprises administering to a human in need of treatment a pharmaceutical composition comprising an adenoviral vector, preferably a replication-deficient adenoviral vector, comprising a nucleic acid sequence encoding a human TNF-α in a pharmaceutically acceptable carrier. The method preferably further comprises administering a dose of ionizing radiation and a dose of one or more chemotherapeutics to the human. The delivery of the human TNF-α coding sequence, especially in combination with the delivery of radiation and chemotherapy, offers an improvement over previously described treatments using soluble TNF protein by optimizing local effect and minimizing systemic toxicity. Indeed, the invention is predicated, in part, on the surprising discovery that tumor size can be reduced without significant toxicity to the patient. In addition, the inventive method has been demonstrated to reduce the size of tumors that were refractory to treatment with TNF-α protein. Various aspects of the inventive method are discussed below. Although each parameter is discussed separately, the inventive method comprises combinations of the parameters set forth below to treat a human for cancer. Accordingly, any combination of parameters can be used according to the inventive method.

### Adenoviral Vector

The inventive method comprises administering to a human a pharmaceutical composition comprising an adenoviral vector comprising a nucleic acid sequence encoding a human TNF-α in a pharmaceutically acceptable carrier. Adenovirus (Ad) is a 36 kb double-stranded DNA virus that efficiently transfers DNA *in vivo* to a variety of different target cell types. The vector can be produced in high titers and can efficiently transfer DNA to replicating and non-replicating cells. Any subtype, mixture of subtypes, or chimeric adenovirus can be used as the source of the viral genome for the adenoviral vector. Adenoviral stocks that can be employed as a source of adenovirus can be amplified from the adenoviral serotypes 1 through 51, which are currently available from the American Type Culture Collection (ATCC, Manassas, VA), or from any other serotype of adenovirus available from any other source. For instance, an adenovirus can be of subgroup A (e.g., serotypes 12, 18, and 31), subgroup B (e.g., serotypes 3, 7, 11, 14, 16, 21, 34, 35, and 50), subgroup C (e.g., serotypes 1, 2, 5, and 6), subgroup D (e.g., serotypes 8, 9, 10, 13, 15, 17, 19, 20, 22-30, 32, 33, 36-39, and 42-48), subgroup E (serotype 4), subgroup F (serotypes 40 and 41), an unclassified serogroup (e.g., serotypes 49 and 51), or any other adenoviral serotype. Preferably, the adenoviral vector is of subgroup C, especially serotype 2 or 5.

The adenoviral vector comprising a nucleic acid sequence encoding TNF-α is ideally manipulated to limit replication of the vector within the target tissue. For example, the adenoviral vector of the pharmaceutical composition can be a conditionally-replicating adenoviral vector, which is engineered to replicate under conditions pre-determined by the practitioner. For example, replication-essential gene functions, e.g., gene functions encoded by the adenoviral early regions, can be operably linked to an inducible, repressible, or tissue-specific transcription control sequence, e.g., promoter. In this embodiment, replication requires the presence or absence of specific factors that interact with the transcription control sequence. Replication of the adenoviral vector can be limited to a target tissue, thereby allowing greater distribution of the vector throughout the tissue while exploiting adenovirus' natural ability to lyse cells during the replication cycle, thereby providing an additional mode of destroying tumor cells. Conditionally-replicating adenoviral vectors are described further in U.S. Patent 5,998,205.

Preferably, the adenoviral vector is replication-deficient. By "replication-deficient" is meant that the adenoviral vector comprises an adenoviral genome that lacks at least one replication-essential gene function (i.e., such that the adenoviral vector does not replicate in typical host cells, especially those in the human patient that could be infected by the adenoviral vector in the course of treatment in accordance with the invention). A deficiency in a gene, gene function, or gene or genomic region, as used herein, is defined as a deletion of sufficient genetic material of the viral genome to impair or obliterate the function of the gene whose nucleic acid sequence was deleted in whole or in part. Deletion of an entire gene region often is not required for disruption of a replication-essential gene function. However, for the purpose of providing sufficient space in the adenoviral genome for one or more transgenes, removal of a majority of a gene region may be desirable. Replication-essential gene functions are those gene functions that are required for replication (e.g., propagation) and are encoded by, for example, the adenoviral early regions (e.g., the E1, E2, and E4 regions), late regions (e.g., the L1-L5 regions), genes involved in viral packaging (e.g., the IVa2 gene), and virus-associated RNAs (e.g., VA-RNA1 and/or VA-RNA-2). Preferably, the replication deficient adenoviral vector comprises an adenoviral genome deficient in at least one replication-essential gene function of one or more regions of the adenoviral genome (such as deletions within or of the E1 region of the adenoviral genome), alone or in combination with deletions of the adenoviral genome that do not render the adenoviral vector replication-deficient (such as deletions of the adenoviral genome within or of the E3 region, which is not essential for adenoviral replication). Preferably, the adenoviral vector is deficient in at least one gene function of the E1 region of the adenoviral genome required for viral replication (denoted an E1-deficient adenoviral vector). In addition to such a deficiency in the E1 region, the recombinant adenovirus also can have a mutation in the major late promoter (MLP), as discussed in International Patent Application WO 00/00628. More preferably, the vector is deficient in at least one replication-essential gene function (desirably all replication-essential gene functions) of the E1 region and at least part of the nonessential E3 region (e.g., an Xba I deletion of the E3 region) (denoted an E1/E3-deficient adenoviral vector). With respect to the E1 region, the adenoviral vector can be deficient in part or all of the E1A region and part or all of the E1B region, e.g., in at least one replication-essential gene function of each of the E1A and E1B regions. When E1-deficient, the adenoviral vector genome can comprise a deletion beginning at any nucleotide between nucleotides 335 to 375 (e.g., nucleotide 356) and ending at any nucleotide between nucleotides 3,310 to 3,350 (e.g., nucleotide 3,329) or even ending at any nucleotide between 3,490 and 3,530 (e.g., nucleotide 3,510) (based on the adenovirus serotype 5 genome). When E2A-deficient the adenoviral vector genome can comprise a deletion beginning at any nucleotide between nucleotides 22,425 to 22,465 (e.g., nucleotide 22,443) and ending at any nucleotide between nucleotides 24,010 to 24,050 (e.g., nucleotide 24,032) (based on the adenovirus serotype 5 genome). When E3-deficient, the adenoviral vector genome can comprise a deletion beginning at any nucleotide between nucleotides 28,575 to 29,615 (e.g., nucleotide 28,594) and ending at any nucleotide between nucleotides 30,450 to 30,490 (e.g., nucleotide 30,469) (based on the adenovirus serotype 5 genome). When the adenoviral vector is deficient in at least one replication-essential gene function in one region of the adenoviral genome (e.g., an E1- or E1/E3-deficient adenoviral vector), the adenoviral vector is referred to as "singly replication-deficient."

Preferably, the adenoviral vector is "multiply deficient," meaning that the adenoviral vector is deficient in one or more gene functions required for viral replication in each of two or more regions of the adenoviral genome. For example, the aforementioned E1-deficient or E1/E3-deficient adenoviral vector can be further deficient in at least one replication-essential gene function of the E4 region (denoted an E1/E4- or E1/E3/E4-deficient adenoviral vector). An adenoviral vector deleted of the entire E4 region can elicit a lower host immune response. When E4-deficient, the adenoviral vector genome can comprise a deletion beginning at, for example, any nucleotide between nucleotides 32,805 to 32,845 (e.g., nucleotide 32,826) and ending at, for example, any nucleotide between nucleotides to 35,540 to 35,580 (e.g., nucleotide 35,561) (based on the adenovirus serotype 5 genome), optionally in addition to deletions in the E1 region (e.g., nucleotides 356 to 3,329 or nucleotides 356 to 3,510) and/or deletions in the E3 region (e.g., nucleotides 28,594 to 30,469 or nucleotides 28,593 to 30,470).

Alternatively, the adenoviral vector is deficient in at least one gene function of the E1 region and is deficient in at least one gene function of the E2 region (denoted an E1/E2A- or E1/E2A/E3-deficient adenoviral vector). If the adenoviral vector of the invention is deficient in a replication-essential gene function of the E2A region, the vector preferably does not comprise a complete deletion of the E2A region, which is less than about 230 base pairs in length. Generally, the E2A region of the adenovirus codes for a DBP (DNA binding protein), a polypeptide required for DNA replication. DBP is composed of 473 to 529 amino acids depending on the viral serotype. It is believed that DBP is an asymmetric protein that exists as a prolate ellipsoid consisting of a globular Ct with an extended Nt domain. Studies indicate that the Ct domain is responsible for DBP's ability to bind to nucleic acids, bind to zinc, and function in DNA synthesis at the level of DNA chain elongation. However, the Nt domain is believed to function in late gene expression at both transcriptional and post-transcriptional levels, is responsible for efficient nuclear localization of the protein, and also may be involved in enhancement of its own expression. Deletions in the Nt domain between amino acids 2 to 38 have indicated that this region is important for DBP function (Brough et al., Virology, 196, 269-281 (1993)). While deletions in the E2A region coding for the Ct region of the DBP have no effect on viral replication, deletions in the E2A region which code for amino acids 2 to 38 of the Nt domain of the DBP impair viral replication. It is preferable that any multiply replication-deficient adenoviral vector contains this portion of the E2A region of the adenoviral genome. In particular, for example, the desired portion of the E2A region to be retained is that portion of the E2A region of the adenoviral genome which is defined by the 5' end of the E2A region, specifically positions Ad5(23816) to Ad5(24032) of the E2A region of the adenoviral genome of serotype Ad5. This portion of the adenoviral genome desirably is included in the adenoviral vector because it is not complemented in current E2A cell lines so as to provide the desired level of viral propagation.

In a particularly preferred embodiment, the adenoviral vector comprises an adenoviral genome deficient in one or more replication-essential gene functions of each of the E1 and E4 regions (i.e., the adenoviral vector is an E1/E4-deficient adenoviral vector), preferably with the entire coding region of the E4 region having been deleted from the adenoviral genome. In other words, all the open reading frames (ORFs) of the E4 region have been removed. The E4 region of the adenoviral vector preferably retains the native E4 promoter, polyadenylation sequence, and/or the right-side inverted terminal repeat (ITR).

The adenoviral vector, when multiply replication-deficient, especially in replication-essential gene functions of the E1 and E4 regions, preferably includes a spacer element to provide viral growth in a complementing cell line similar to that achieved by singly replication-deficient adenoviral vectors, particularly an adenoviral vector comprising a deficiency in the E1 region. The spacer element can contain any sequence or sequences which are of a desired length, such as sequences at least about 15 base pairs (e.g., between about 15 base pairs and about 12,000 base pairs), preferably about 100 base pairs to about 10,000 base pairs, more preferably about 500 base pairs to about 8,000 base pairs, even more preferably about 1,500 base pairs to about 6,000 base pairs, and most preferably about 2,000 to about 3,000 base pairs in length. The spacer element sequence can be coding or non-coding and native or non-native with respect to the adenoviral genome, but does not restore the replication-essential function to the deficient region. In the absence of a spacer, production of fiber protein and/or viral growth of the multiply replication-deficient adenoviral vector is reduced by comparison to that of a singly replication-deficient adenoviral vector. However, inclusion of the spacer in at least one of the deficient adenoviral regions, preferably the E4 region, can counteract this decrease in fiber protein production and viral growth. The use of a spacer in an adenoviral vector is described in U.S. Patent 5,851,806.

Desirably, the adenoviral vector requires, at most, complementation of replication-essential gene functions of the E1, E2A, and/or E4 regions of the adenoviral genome for replication (i.e., propagation). However, the adenoviral genome can be modified to disrupt one or more replication-essential gene functions as desired by the practitioner, so long as the adenoviral vector remains deficient and can be propagated using, for example, complementing cells and/or exogenous DNA (e.g., helper adenovirus) encoding the disrupted replication-essential gene functions. In this respect, the adenoviral vector can be deficient in replication-essential gene functions of only the early regions of the adenoviral genome, only the late regions of the adenoviral genome, and both the early and late regions of the adenoviral genome. The adenoviral vector also can have essentially the entire adenoviral genome removed, in which case it is preferred that at least either the viral inverted terminal repeats (ITRs) and one or more promoters or the viral ITRs and a packaging signal are left intact (i.e., an adenoviral amplicon). The larger the region of the adenoviral genome that is removed, the larger the piece of exogenous nucleic acid sequence that can be inserted into the genome. For example, given that the adenoviral genome is 36 kb, by leaving the viral ITRs and one or more promoters intact, the exogenous insert capacity of the adenovirus is approximately 35 kb. Alternatively, a multiply deficient adenoviral vector that contains only an ITR and a packaging signal effectively allows insertion of an exogenous nucleic acid sequence of approximately 37-38 kb (in that adenovirus can package DNA up to about 105% the size of the wild-type adenoviral genome). Of course, the inclusion of a spacer element in any or all of the deficient adenoviral regions will decrease the capacity of the adenoviral vector for large inserts. Suitable replication-deficient adenoviral vectors, including multiply replication-deficient adenoviral vectors, are disclosed in U.S. Patents 5,837,511, 5,851,806, 5,994,106, and 6,579,522, U.S. Patent Application Publication Nos. 2001/0043922 A1 2002/0004040 A1, 2002/0031831 A1, and 2002/01 10545 A1, and International Patent Applications WO 95/34671, WO 97/12986, and WO 97/21826.

A particularly preferred adenoviral vector for use in the context of the invention contains a deletion beginning at any nucleotide between nucleotides 335 to 375 (e.g., nucleotide 356) and ending at any nucleotide between nucleotides 3,310 to 3,350 (e.g., nucleotide 3,329), and a deletion beginning at any nucleotide between nucleotides 28,575 to 29,615 (e.g., nucleotide 28,594) and ending at any nucleotide between nucleotides 30,450 to 30,490 (e.g., nucleotide 30,469) (based on the adenovirus serotype 5 genome). Alternatively, the adenoviral vector genome preferably contains a deletion beginning at any nucleotide between nucleotides 335 to 375 (e.g., nucleotide 356) and ending at any nucleotide between nucleotides 3,490 and 3,530 (e.g., nucleotide 3,510), a deletion beginning at any nucleotide between nucleotides 28,575 an 29,615 (e.g., nucleotide 28,593) and ending at any nucleotide between nucleotides 30,450 and 30,490 (e.g., nucleotide 30,470), and a deletion beginning at any nucleotide between nucleotides 32,805 to 32,845 (e.g., nucleotide 32,826) and ending at, for example, any nucleotide between nucleotides to 35,540 to 35,580 (e.g., nucleotide 35,561) (based on the adenovirus serotype 5 genome). The endpoints defining the deleted nucleotide portions can be difficult to preciously determine and typically will not significantly affect the nature of the adenoviral vector, i.e., each of the aforementioned nucleotide numbers can be +/- 1, 2, 3, 4, 5, 10, or even 20 nucleotides. An especially preferred adenoviral vector for use in the inventive method is that described in U.S. Patent 6,579,522 and International Patent Application WO 02/00906. The adenoviral vector, for example, can comprise the nucleotide sequence of SEQ ID NO:1, i.e., the adenoviral genome of the adenoviral vector, for example, can be the adenoviral portion of the human TNF-α-encoding adenoviral vector whose nucleotide sequence is set forth in SEQ ID NO:1.

Ideally, the pharmaceutical composition is virtually free of replication-competent adenovirus (RCA) contamination (e.g., the pharmaceutical composition comprises less than about 1% of RCA contamination). Most desirably, the pharmaceutical composition is RCA-free. Adenoviral vector compositions and stocks that are RCA-free are described in U.S. Patents 5,944,106 and 6,482,616, U.S. Patent Application Publication No. 2002/0110545 A1, and International Patent Application WO 95/34671. Ideally, the pharmaceutical composition also is free of E1-revertants when the adenoviral vector is E1-deficient in combination with deficiencies in other replication-essential gene functions of another region of the adenoviral genome, as further described in International Patent Application WO 03/040314.

In addition to modification (e.g., deletion, mutation, or replacement) of adenoviral sequences encoding replication-essential gene functions, the adenoviral genome can contain benign or non-lethal modifications, i.e., modifications which do not render the adenovirus replication-deficient, or, desirably, do not adversely affect viral functioning and/or production of viral proteins, even if such modifications are in regions of the adenoviral genome that otherwise contain replication-essential gene functions. Such modifications commonly result from DNA manipulation or serve to facilitate expression vector construction. For example, it can be advantageous to remove or introduce restriction enzyme sites in the adenoviral genome. Such benign mutations often have no detectable adverse effect on viral functioning. For example, the adenoviral vector can comprise a deletion of nucleotides 10,594 and 10,595 (based on the adenoviral serotype 5 genome), which are associated with VA-1 transcription, but the deletion of which does not prohibit production of VA-1.

In the preferred E1/E4-deficient adenoviral vector for use in the inventive method, the L5 fiber region is retained, and a spacer is located between the L5 fiber region and the right-side ITR. More preferably, in such an adenoviral vector, the E4 polyadenylation sequence alone or, most preferably, in combination with another sequence, exists between the L5 fiber region and the right-side ITR, so as to sufficiently separate the retained L5 fiber region from the right-side ITR, such that viral production of such a vector approaches that of a singly replication-deficient adenoviral vector, particularly an E1-deficient adenoviral vector.

The spacer element inserted into the adenoviral genome can contain a promoter-variable expression cassette, which can include any promoter (cellular or viral), foreign nucleic acid sequence, and/or polyadenylation sequence. Preferably, in the case of a spacer element inserted into the E4 region (coding sequences for which have been removed), both the E4 polyadenylation sequence and the E4 promoter remain in the vector. In such an embodiment, the spacer element is located between the E4 polyadenylation site and the E4 promoter, or, if the E4 promoter is not present in the vector, the spacer element is proximal to the right-side (3') ITR.

The spacer element can comprise any suitable polyadenylation sequence. Examples of suitable polyadenylation sequences include synthetic optimized sequences, as well as the polyadenylation sequences of BGH (Bovine Growth Hormone), polyoma virus, TK (Thymidine Kinase), EBV (Epstein Barr Virus), and the papillomaviruses, including human papillomaviruses and BPV (Bovine Papilloma Virus). Preferably, the spacer element includes an SV40 (Human Sarcoma Virus-40) polyadenylation sequence. The SV40 polyadenylation sequence allows for higher virus production levels of multiply deficient adenoviral vectors. If the spacer element comprises all or part of a coding sequence and production of a gene product is not desired, the spacer element can be flanked by polyadenylation sequences.

A foreign coding sequence also can function as the spacer element in the E4-deficient region of the adenoviral genome. The foreign coding sequence is limited only by the size of the insert the vector can accommodate and can be any suitable coding sequence. Examples of suitable foreign coding sequences include, but are not limited to, coding sequences encoding marker proteins such as pGUS, secretory alkaline phosphatase, luciferase, β-galactosidase, and human anti-trypsin; therapeutic factors; potential immune modifiers such as B3-19K, E3-14.7, ICP47, fas ligand, and CTLA4; biologically inactive sequences (e.g., sequences that are (i) not transcribed to produce a product or (ii) encode a defective or biologically inactive product), and other innocuous sequences such as the glucuronidase gene.

It should be appreciated that the deletion of different regions of the adenoviral vector can alter the immune response of the mammal. In particular, the deletion of different regions can reduce the inflammatory response generated by the adenoviral vector. Furthermore, the adenoviral vector's coat protein can be modified so as to decrease the adenoviral vector's ability or inability to be recognized by a neutralizing antibody directed against the wild-type coat protein, as described in International Patent Application WO 98/40509.

Similarly, the coat protein of the adenoviral vector can be manipulated to alter the binding specificity or recognition of the adenoviral vector for a viral receptor on a potential host cell. Such manipulations can include deletion of regions of the fiber, penton, hexon, pIIIa, pVI, and/or pIX, insertions of various native or non-native ligands into portions of the coat protein, and the like. Manipulation of the coat protein can broaden the range of cells infected by the adenoviral vector or enable targeting of the adenoviral vector to a specific cell type.

For example, in one embodiment, the adenoviral vector comprises a chimeric coat protein (e.g., a fiber, hexon, pIX, pIIIa, or penton protein), which differs from the wild-type (i.e., native) coat protein by the introduction of a nonnative amino acid sequence, preferably at or near the carboxyl terminus. Preferably, the nonnative amino acid sequence is inserted into or in place of an internal coat protein sequence. The nonnative amino acid sequence can be inserted within the internal coat protein sequence or at the end of the internal coat protein sequence. The resultant chimeric viral coat protein is able to direct entry into cells of the adenoviral vector comprising the coat protein that is more efficient than entry into cells of an adenoviral vector that is identical except for comprising a wild-type viral coat protein rather than the chimeric viral coat protein. Preferably, the chimeric coat protein binds a novel endogenous binding site present on the cell surface that is not recognized, or is poorly recognized, by an adenoviral vector comprising a wild-type coat protein. One direct result of this increased efficiency of entry is that the adenoviral vector can bind to and enter cell types which an adenovirus comprising wild-type coat protein typically cannot enter or can enter with only a low efficiency. If desired, native binding of the adenoviral coat proteins, e.g., the fiber or penton base, can be ablated.

In another embodiment, the adenoviral vector comprises a chimeric virus coat protein not selective for a specific type of eukaryotic cell. The chimeric coat protein differs from the wild-type coat protein by an insertion of a nonnative amino acid sequence into or in place of an internal coat protein sequence. For example, a ligand comprising about five to about nine lysine residues (preferably seven lysine residues) is attached to the C-terminus of the adenoviral fiber protein via a non-coding spacer sequence. In this embodiment, the chimeric virus coat protein efficiently binds to a broader range of eukaryotic cells than a wild-type virus coat, such as described in International Patent Application WO 97/20051. In that a tumor does not comprise a homogenous population of cancer cells, such adenoviral vectors are particularly preferred.

The specificity of binding of the adenoviral vector to a given cell also can be adjusted by use of an adenovirus comprising a short-shafted adenoviral fiber gene, as discussed in U.S. Patent 5,962,311. Use of an adenovirus comprising a short-shafted adenoviral fiber gene reduces the level or efficiency of adenoviral fiber binding to its cell-surface receptor and increases adenoviral penton base binding to its cell-surface receptor, thereby increasing the specificity of binding of the adenoviral vector to a given cell. Alternatively, use of an adenovirus comprising a short-shafted fiber enables targeting of the adenovirus to a desired cell-surface receptor by the introduction of a nonnative amino acid sequence either into the penton base or the fiber knob.

Of course, the ability of an adenoviral vector to recognize a potential host cell can be modulated without genetic manipulation of the coat protein, i.e., through use of a bispecific molecule. For instance, complexing an adenovirus with a bispecific molecule comprising a penton base-binding domain and a domain that selectively binds a particular cell surface binding site enables the targeting of the adenoviral vector to a particular cell type.

Suitable modifications to an adenoviral vector are described in U.S. Patents 5,543,328, 5,559,099, 5,712,136, 5,731,190, 5,756,086, 5,770,442, 5,846,782, 5,871,727, 5,885,808, 5,922,315, 5,962,311, 5,965,541, 6,057,155, 6,127,525, 6,153,435, 6,329,190, 6,455,314, and 6,465,253, U.S. Patent Applications Publication Nos. 2001/0047081 A1, 2002/0099024 A1, and 2002/0151027 A1, and International Patent Applications WO 95/02697, WO 95/16772, WO 95/34671, WO 96/07734, WO 96/22378, WO 96/26281, WO 97/20051, WO 98/07865, WO 98/07877, WO 98/40509, WO 98/54346, WO 00/15823, WO 01/58940, and WO 01/92549. The construction and propagation of adenoviral vectors is well understood in the art. Moreover, numerous adenoviral vectors are available commercially.

Replication-deficient adenoviral vectors are typically produced in complementing cell lines that provide gene functions not present in the replication-deficient adenoviral vectors, but required for viral propagation, at appropriate levels in order to generate high titers of viral vector stock. A preferred cell line complements for at least one and preferably all replication-essential gene functions not present in a replication-deficient adenovirus. The complementing cell line can complement for a deficiency in at least one replication-essential gene function encoded by the early regions, late regions, viral packaging regions, virus-associated RNA regions, or combinations thereof, including all adenoviral functions (e.g., to enable propagation of adenoviral amplicons). Most preferably, the complementing cell line complements for a deficiency in at least one replication-essential gene function (e.g., two or more replication-essential gene functions) of the E1 region of the adenoviral genome, particularly a deficiency in a replication-essential gene function of each of the E1A and E1B regions. In addition, the complementing cell line can complement for a deficiency in at least one replication-essential gene function of the E2 (particularly as concerns the adenoviral DNA polymerase and terminal protein) and/or E4 regions of the adenoviral genome. Desirably, a cell that complements for a deficiency in the E4 region comprises the E4-ORF6 gene sequence and produces the E4-ORF6 protein. Such a cell desirably comprises at least ORF6 and no other ORF of the E4 region of the adenoviral genome. The cell line preferably is further **characterized in that** it contains the complementing genes in a non-overlapping fashion with the adenoviral vector, which minimizes, and practically eliminates, the possibility of the vector genome recombining with the cellular DNA. Accordingly, the presence of replication competent adenoviruses (RCA) is minimized if not avoided in the vector stock, which, therefore, is suitable for certain therapeutic purposes, especially gene therapy purposes. The lack of RCA in the vector stock avoids the replication of the adenoviral vector in non-complementing cells. Construction of such a complementing cell lines involve standard molecular biology and cell culture techniques, such as those described by Sambrook et al., Molecular Cloning, a Laboratory Manual, 2d edition, Cold Spring Harbor Press, Cold Spring Harbor, N.Y. (1989), and Ausubel et al., Current Protocols in Molecular Biology, Greene Publishing Associates and John Wiley & Sons, New York, N.Y. (1994).

Complementing cell lines for producing the adenoviral vector include, but are not limited to, 293 cells (described in, e.g., Graham et al., J. Gen. Virol., 36, 59-72 (1977)), PER.C6 cells (described in, e.g., International Patent Application WO 97/00326, and U.S. Patents 5,994,128 and 6,033,908), and 293-ORF6 cells (described in, e.g., International Patent Application WO 95/34671 and Brough et al., J. Virol., 71, 9206-9213 (1997)). In some instances, the complementing cell will not complement for all required adenoviral gene functions. Helper viruses can be employed to provide the gene functions in *trans* that are not encoded by the cellular or adenoviral genomes to enable replication of the adenoviral vector. Adenoviral vectors can be constructed, propagated, and/or purified using the materials and methods set forth, for example, in U.S. Patents 5,965,358, 5,994,128, 6,033,908, 6,168,941, 6,329,200, 6,383,795, 6,440,728, 6,447,995, and 6,475,757, U.S. Patent Application Publication No. 2002/0034735 A1 and International Patent Applications WO 98/53087, WO 98/56937, WO 99/15686, WO 99/54441, WO 00/12765, WO 01/77304, and WO 02/29388, as well as the other references identified herein.

### Nucleic Acid Sequence Encoding TNF

The adenoviral vector, preferably the replication-deficient adenoviral vector, comprises a nucleic acid sequence encoding TNF-α, preferably a human TNF-α. While other members of the TNF family of proteins, such as Fas ligand and CD40 ligand, have utility in treating a number of diseases, TNF-α has been proven to be an effective anti-cancer agent. The effect of TNF-α on cancer is multifactorial including the induction of apoptosis and tumor necrosis. Indirect systemic effects of TNF-α include activation of immune effector cells including induction of cytokine secretion and activation of the vascular coagulation system. TNF-α induces adhesiveness of vascular endothelium to neutrophils and platelets and decreases thrombomodulin production (Koga et al., Am. J. Physiol., 268, 1104-1113 (1995)). The result is clot formation in the tumor neovasculature and subsequent hemorrhagic necrosis of the tumors. A nucleic acid sequence encoding a human TNF-α is described in detail in U.S. Patent 4,879,226.

While it is preferred that the nucleic acid sequence encoding a human TNF-α is that set forth in U.S. Patent 4,879,226, which nucleotide sequence is set forth in SEQ ID NO:2, many modifications and variations of the nucleic acid sequence are possible and appropriate in the context of the invention. For example, the degeneracy of the genetic code allows for the substitution of nucleotides throughout polypeptide coding regions, as well as in the translational stop signal, without alteration of the encoded polypeptide. Such substitutable sequences can be deduced from the known amino acid sequence of a human TNF-α or nucleic acid sequence encoding human TNF-α (such as the sequence disclosed in U.S. Patent 4,879,226) and can be constructed by conventional synthetic or site-specific mutagenesis procedures. Synthetic DNA methods can be carried out in substantial accordance with the procedures of Itakura et al., Science, 198, 1056-1063 (1977), and Crea et al., Proc. Natl. Acad. Sci. USA, 75, 5765-5769 (1978). Site-specific mutagenesis procedures are described in Maniatis et al., Molecular Cloning: A Laboratory Manual, Cold Spring Harbor, NY (2d ed. 1989).

In addition, a nucleic acid sequence encoding a homolog of a human TNF-α, i.e., any protein that is more than about 70% identical (preferably more than about 80% identical, more preferably more than about 90% identical, and most preferably more than about 95% identical) to the protein at the amino acid level and displays the same level of anti-cancer activity of a human TNF-α, can be incorporated into the adenoviral vector (e.g., replication-deficient adenoviral vector). The degree of amino acid identity can be determined using any method known in the art, such as the BLAST sequence database. Furthermore, a homolog of the protein can be any peptide, polypeptide, or portion thereof, which hybridizes to the protein under at least moderate, preferably high, stringency conditions, and retains anti-cancer activity. Exemplary moderate stringency conditions include overnight incubation at 37° C in a solution comprising 20% formamide, 5x SSC (150 mM NaCl, 15 mM trisodium citrate), 50 mM sodium phosphate (pH 7.6), 5x Denhardt's solution, 10% dextran sulfate, and 20 mg/ml denatured sheared salmon sperm DNA, followed by washing the filters in 1x SSC at about 37-50° C, or substantially similar conditions, e.g., the moderately stringent conditions described in Sambrook et al., *supra.* High stringency conditions are conditions that use, for example (1) low ionic strength and high temperature for washing, such as 0.015 M sodium chloride/0.0015 M sodium citrate/0.1% sodium dodecyl sulfate (SDS) at 50° C, (2) employ a denaturing agent during hybridization, such as formamide, for example, 50% (v/v) formamide with 0.1% bovine serum albumin (BSA)/0.1% Ficoll/0.1% polyvinylpyrrolidone (PVP)/50 mM sodium phosphate buffer at pH 6.5 with 750 mM sodium chloride, 75 mM sodium citrate at 42° C, or (3) employ 50% formamide, 5x SSC (0.75 M Nad, 0.075 M sodium citrate), 50 mM sodium phosphate (pH 6.8), 0.1% sodium pyrophosphate, 5x Denhardt's solution, sonicated salmon sperm DNA (50 µg/ml), 0.1% SDS, and 10% dextran sulfate at 42° C, with washes at (i) 42° C in 0.2x SSC, (ii) at 55° C in 50% formamide and (iii) at 55° C in 0.1x SSC (preferably in combination with EDTA). Additional details and an explanation of stringency of hybridization reactions are provided in, e.g., Ausubel et al., *supra.*

The nucleic acid sequence can encode a functional portion of a human TNF-α, i.e., any portion of the protein that retains the biological activity of the naturally occurring, full-length protein at measurable levels. A functional TNF-α fragment produced by expression of the nucleic acid sequence of the adenoviral vector, preferably the replication-deficient adenoviral vector, can be identified using standard molecular biology and cell culture techniques, such as assaying the biological activity of the fragment in human cells transiently transfected with a nucleic acid sequence encoding the protein fragment.

Alternatively, the nucleic acid sequence encoding a human TNF-α can be manipulated to alter (preferably enhance) the activity of the TNF-α. The nucleic acid sequence can be manipulated to enhance secretion of the TNF-α protein, or can be manipulated to encode a TNF-α protein that remains bound to the cell surface. The nucleic acid sequence can be manipulated to enhance the stability of the TNF-α protein (e.g., retain proper three-dimensional conformation under adverse conditions), or to increase the potency of the TNF-α protein for activation the TNF receptor. As such, the invention is not limited to that nucleic acid sequence specifically in U.S. Patent 4,879,226, and modifications of the nucleic acid sequence are contemplated herein.

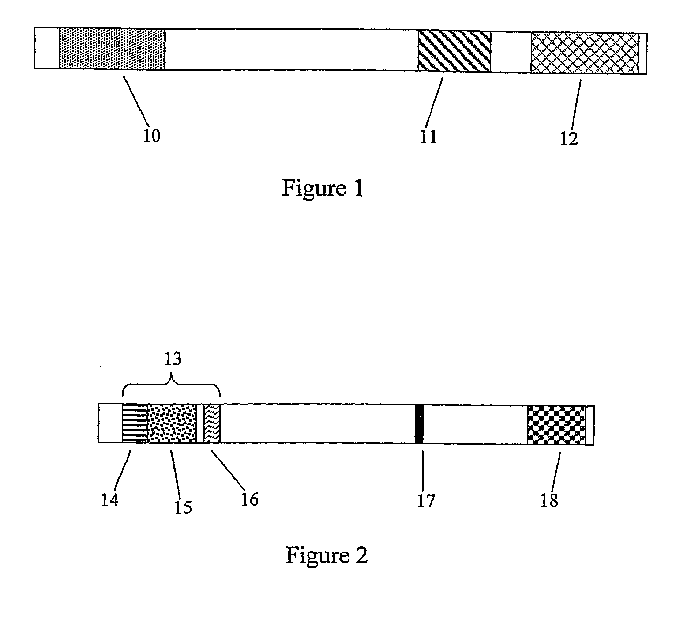

The nucleic acid sequence is desirably present as part of an expression cassette, i.e., a particular base sequence that possesses functions which facilitate subcloning and recovery of a nucleic acid sequence (e.g., one or more restriction sites) or expression of a nucleic acid sequence (e.g., polyadenylation or splice sites). The nucleic acid sequence coding for a human TNF-α is preferably located in the E1 region (e.g., replaces the E1 region in whole or in part) of the adenoviral genome. For example, the E1 region can be replaced by a promoter-variable expression cassette comprising a nucleic acid sequence encoding a human TNF-α, as shown in, for example, **Figures 1** and **2**, which depict an unmodified and modified adenoviral vector genome, respectively. The E1 region **(10)** can be replaced by an expression cassette **(13)** comprising a radiation-inducible promoter **(14),** a nucleic acid sequence encoding TNF-α **(15),** and a polyadenylation sequence **(16).** The expression cassette is preferably inserted in a 5'-3' orientation, e.g., oriented such that the direction of transcription of the expression cassette is the same as that of the surrounding adenoviral genome. In addition to the expression cassette comprising the nucleic acid sequence encoding a human TNF-α, the adenoviral vector can comprise other expression cassettes containing nucleic acid sequences encoding other products, which cassettes can replace any of the deleted regions of the adenoviral genome. The insertion of an expression cassette into the adenoviral genome (e.g., the E1 region of the genome) can be facilitated by known methods, for example, by the introduction of a unique restriction site at a given position of the adenoviral genome. As set forth above, preferably the E3 region of the adenoviral vector **(11)** is deleted **(17),** and the E4 region **(12)** is replaced by a spacer element **(18).**

Preferably, the nucleic acid sequence encoding a human TNF-α further comprises a transcription-terminating region such as a polyadenylation sequence located 3' of TNF-α coding sequence (in the direction of transcription of the coding sequence). Any suitable polyadenylation sequence can be used, including a synthetic optimized sequence, as well as the polyadenylation sequence of BGH (Bovine Growth Hormone), polyoma virus, TK (Thymidine Kinase), EBV (Epstein Barr Virus), and the papillomaviruses, including human papillomaviruses and BPV (Bovine Papilloma Virus). A preferred polyadenylation sequence is the SV40 (Human Sarcoma Virus-40) polyadenylation sequence.

Preferably, the nucleic acid sequence encoding a human TNF-α is operably linked to (i.e., under the transcriptional control of) one or more promoter and/or enhancer elements, for example, as part of a promoter-variable expression cassette. Techniques for operably linking sequences together are well known in the art. Any suitable promoter or enhancer sequence can be used in the context of the invention. Suitable viral promoters include, for instance, cytomegalovirus (CMV) promoters, such as the CMV immediate-early promoter (described in, for example, U.S. Patent 5,168,062), promoters derived from human immunodeficiency virus (HIV), such as the HIV long terminal repeat promoter, Rous sarcoma virus (RSV) promoters, such as the RSV long terminal repeat, mouse mammary tumor virus (MMTV) promoters, HSV promoters, such as the Lap2 promoter or the herpes thymidine kinase promoter (Wagner et al., Proc. Natl. Acad. Sci., 78, 144-145 (1981)), promoters derived from SV40 or Epstein Barr virus, an adeno-associated viral promoter, such as the p5 promoter, and the like. The regulatory sequences to which the nucleic acid sequence is operably linked also can comprise a tissue-specific promoter, i.e., a promoter that is preferentially activated in a given tissue and results in production of a gene product, particularly TNF-α, in the tissue where activated. For example, regulatory sequences selectively active in, for instance, endothelial cells or stromal cells are ideal for expressing an exogenous nucleic acid sequence in the environment of cancer cells.

Many of the above-described promoters are constitutive promoters. Instead of being a constitutive promoter, the promoter can be an inducible promoter, i.e., a promoter that is up- and/or down-regulated in response to an appropriate signal. For example, an expression control sequence up-regulated by a chemotherapeutic agent is particularly useful in cancer applications (e.g., a chemo-inducible promoter). In addition, an expression control sequence can be up-regulated by a radiant energy source or by a substance that distresses cells. For example, an expression control sequence can be up-regulated by ultrasound, light activated compounds, radiofrequency, chemotherapy, and cyofreezing. A preferred adenoviral vector according to the invention comprises a chemo-inducible or radiation-inducible promoter operably linked to the nucleic acid sequence encoding a human TNF-α. The use of a radiation-inducible promoter enables localized control of TNF-α production, for example, by the administration of radiation to a cell or host comprising the adenoviral vector, thereby minimizing systemic toxicity. Any suitable radiation-inducible promoter can be used in the context of the invention. A preferred radiation-inducible promoter for use in the context of the invention is the early growth region-1 (EGR-1) promoter, specifically the CArG domain of the EGR-1 promoter. The region of the EGR-1 promoter likely responsible for radiation-inducibility is located between nucleotides -550 bp and -50 bp. The EGR-1 promoter is described in detail in U.S. Patent 5,206,152 and International Patent Application WO 94/06916 and U.S. Patent 6,605,712. Another suitable radiation-inducible promoter is the c-Jun promoter, which is activated by X-radiation. The region of the c-Jun promoter likely responsible for radiation-inducibility is believed to be located between nucleotide -1.1 kb to 740 bp. The c-Jun promoter and the EGR-1 promoter are further described in, for instance, U.S. Patent 5,770,581. The promoter can be introduced into the adenoviral genome by any suitable method, such as by use of a unique restriction site in a given region of the adenoviral genome. Alternatively, the promoter can be inserted as part of the expression cassette comprising the nucleic acid sequence encoding a human TNF-α.

### Other Adenoviral Vector Considerations

The adenoviral vector described can comprise nucleic acid sequences other than that encoding a human TNF-α, e.g., a nucleic acid sequence to treat other similar or different diseases and/or afflictions including, but not limited to, chronic lung diseases, such as emphysema, asthma, adult respiratory distress syndrome, and chronic bronchitis, as well as coronary heart disease, common complications associated with cancer, and other afflictions suitably treated or prevented by gene therapy, vaccination, and the like. If the additional nucleic acid sequence (i.e., transgene) confers a prophylactic or therapeutic benefit, the nucleic acid sequence can exert its effect at the level of RNA or protein. Alternatively, the additional nucleic acid sequence can encode an antisense molecule, a ribozyme, a protein that affects splicing or 3' processing (e.g., polyadenylation), or a protein that affects the level of expression of another gene within the cell (i.e., where gene expression is broadly considered to include all steps from initiation of transcription through production of a processed protein), such as by mediating an altered rate of mRNA accumulation or transport or an alteration in post-transcriptional regulation. The additional nucleic acid sequence can encode a chimeric protein for combination therapy. The additional nucleic acid sequence also can encode a factor that acts upon a different target than TNF-α, thereby providing multifactorial cancer treatment. Alternatively, the additional nucleic acid sequence can encode a factor that enhances the effect of TNF-α. An example of such a factor is PKR, double-stranded RNA dependent protein kinase, which renders cancer cells more prone to apoptosis (see, for example, U.S. Patent 5,976,800).

The adenoviral vector can comprise an additional nucleic acid sequence encoding an anti-angiogenic substance other than TNF-α. An anti-angiogenic substance is any biological factor that prevents or ameliorates neovascularization. The anti-angiogenic substance can effect partial or complete prevention and amelioration of angiogenesis to achieve a therapeutic effect. An anti-angiogenic substance includes, for instance, an anti-angiogenic factor, an anti-sense molecule specific for an angiogenic factor, a ribozyme, a receptor for an angiogenic factor, and an antibody that binds a receptor for an angiogenic factor. To prevent vascular leakage into surrounding tissue, which can lead to tumor spread, the adenoviral vector can comprise an additional nucleic acid sequence that encodes a vessel maturation factor, such as, for example, an angiopoietin (Ang, e.g., Ang-1 and Ang-2), midkine (MK), COUP-TFII, and heparin-binding neurotrophic factor (HBNF, also known as heparin binding growth factor). A nucleic acid sequence encoding an immunosuppressor also can be incorporated into the adenoviral vector to reduce any inappropriate immune response thereto as a result of administering the adenoviral vector to a host.

The adenoviral vector can further comprise a nucleic acid sequence encoding a cytokine, such as an interferon (INF α, β, γ), a colony stimulating factor, or an interleukin (IL-1, IL-2, IL-3, IL-10, IL-12, IL-13, IL-15, etc.). The additional nucleic acid sequence can encode a pro-drug converting enzyme, such as HSV thymidine kinase (see, for example, International Patent Application WO 01/24684). The additional nucleic acid sequence preferably encodes a protein that is deleterious to the cancer cells of a mammal (i.e., a "deleterious protein"). Nucleic acid sequences that encode proteins that are deleterious to cancer cells are known in the art, examples of which include nucleic acid sequences that encode p53, Fas, Fas ligand (FasL), FasLM45 (a derivative of FasL that has a deletion in the protease cleavage site), Fas-associating protein with death domain (FADD; also known as mediator of receptor-induced toxicity (MORT-1)), a cell death-inducing coding sequence of Bcl-2 which comprises an N-terminal deletion, a cell death-inducing coding sequence of Bcl-x which comprises an N-terminal deletion, caspases (e.g., caspase 1 (interleukin-1 β-converting enzyme (ICE)), caspase 3, caspase 8 (MACH/FLICE (FADD-homologous ICE-CED3-like protease)/Mch5), and caspase 10 (Mch4)), receptor interacting protein (RIP), cell death abnormal (CED4), Apaf-1, RIP-associated ICH-1/CED-3-homologous protein with a death domain (RAIDD)/caspase and RIP adapter with death domain (CRADD), inhibitor ofNF-κB (IκB), DNA fragmentation factor (DFF), TNF-R1, TNF-related apoptosis-inducing ligand (TRAIL; apo2 ligand (Apo2L)), death receptor 4 (DR4), DR5, bax, bak, bid, bad, bik, bif-2, c-myc, JUN, a dominant negative JUN product, a protease, a protein kinase, a transcriptional activator, a signal transduction protein, protein kinase C, PITSLRE kinase, DAP kinase, JUN N-terminal kinase (JNK)/SAPK, Daxx, ras, Raf, NIK, MEEK1, ASK1, protein kinase R (PKR), Rb194, an adenoviral E1A product, an adenoviral E2F product, a synthetic cell cycle-independent form of an adenoviral E2F product, an adenoviral E4/ORF4 product, apoptin, an apoptotic-active portion of any of the foregoing, and a combination of two or more of the foregoing.

The additional nucleic acid sequence can encode a marker protein, such as green fluorescent protein or luciferase. Such marker proteins are useful in vector construction and determining vector migration. Marker proteins also can be used to determine points of injection in order to efficiently space injections of the pharmaceutical composition to provide a widespread area of treatment. Alternatively, the nucleic acid sequence can encode a selection factor (e.g., an antibiotic resistance factor), which also is useful in vector construction protocols. If desired, the additional nucleic acid sequence(s) can be part of an expression cassette(s).

Any of the nucleic acid sequences described herein can be altered from their native form to increase their therapeutic effect. For example, a cytoplasmic form of a therapeutic gene product can be converted to a secreted form by incorporating a signal peptide into the encoded gene product. A gene product also can be engineered to be taken up by neighboring cells by fusion of the peptide with VP22.

### Pharmaceutical Composition

The pharmaceutical composition of the invention comprises a pharmaceutically acceptable carrier and the adenoviral vector (e.g., a replication-deficient adenoviral vector) comprising the nucleic acid sequence encoding TNF-α. Any suitable pharmaceutically acceptable carrier can be used within the context of the invention, and such carriers are well known in the art. The choice of carrier will be determined, in part, by the particular site to which the pharmaceutical composition is to be administered and the particular method used to administer the pharmaceutical composition.

Suitable formulations include aqueous and non-aqueous solutions, isotonic sterile solutions, which can contain anti-oxidants, buffers, bacteriostats, and solutes that render the formulation isotonic with the blood or other bodily fluid of the intended recipient, and aqueous and non-aqueous sterile suspensions that can include suspending agents, solubilizers, thickening agents, stabilizers, and preservatives. Preferably, the pharmaceutically acceptable carrier is a liquid that contains a buffer and a salt. The formulations can be presented in unit-dose or multi-dose sealed containers, such as ampules and vials, and can be stored in a freeze-dried (lyophilized) condition requiring only the addition of the sterile liquid carrier, for example, water, immediately prior to use. When the formulation is presented in a vial, the vial can be constructed of any material suitable for housing a pharmaceutical composition. The vial desirably is constructed of glass or plastic. The viral preferably is a glass vial. Glass vials are typically constructed of clear glass or tinted glass (e.g., green, blue, or amber), so as to protect a light-sensitive pharmaceutical composition contained therein from degradation. Extemporaneous solutions and suspensions can be prepared from sterile powders, granules, and tablets. Preferably, the pharmaceutically acceptable carrier is a buffered saline solution.

More preferably, the pharmaceutical composition is formulated to protect the adenoviral vector from damage prior to administration. The particular formulation desirably decreases the light sensitivity and/or temperature sensitivity of the adenoviral vector. Indeed, the pharmaceutical composition will be maintained for various periods of time and, therefore should be formulated to ensure stability and maximal activity at the time of administration. Typically, the pharmaceutical composition is maintained at a temperature above 0° C, preferably at 4° C or higher (e.g., 4-10° C). In some embodiments, it is desirable to maintain the pharmaceutical composition at a temperature of 10° C or higher (e.g., 10-20° C), 20° C or higher (e.g., 20-25° C), or even 30° C or higher (e.g., 30-40° C). The pharmaceutical composition can be maintained at the aforementioned temperature(s) for at least 1 day (e.g., 7 days (1 week) or more), though typically the time period will be longer, such as at least 3, 4, 5, or 6 weeks, or even longer, such as at least 10, 11, or 12 weeks, prior to administration to a patient. During that time period, the adenoviral gene transfer vector optimally loses no, or substantially no, activity, although some loss of activity is acceptable, especially with relatively higher storage temperatures and/or relatively longer storage times. The activity of the adenoviral vector composition desirably decreases about 50% or less, preferably about 20% or less, more preferably about 10% or less, and most preferably about 5% or less, after any of the aforementioned time periods.

To this end, the pharmaceutical composition preferably comprises a pharmaceutically acceptable liquid carrier, such as, for example, those described above, and a stabilizing agent selected from the group consisting of polysorbate 80, L-arginine, polyvinylpyrrolidone, α-D-glucopyranosyl α-D-glucopyranoside dihydrate (commonly known as trehalose), and combinations thereof. More preferably, the stabilizing agent is trehalose, or trehalose in combination with polysorbate 80. The stabilizing agent can be present in any suitable concentration in the pharmaceutical composition. When the stabilizing agent is trehalose, the trehalose desirably is present in a concentration of about 1-25% (wt./vol.), preferably about 2-10% (wt./vol.), and more preferably about 4-6% (wt./vol.) of the pharmaceutical composition. When trehalose and polysorbate 80 are present in the pharmaceutical composition, the trehalose preferably is present in a concentration of about 4-6% (wt./vol.), more preferably about 5% (wt./vol.), while the polysorbate 80 desirably is present in a concentration of about 0.001-0.015% (wt./vol.), more preferably about 0.0025% (wt./vol.). When a stabilizing agent, e.g., trehalose, is included in the pharmaceutical composition, the pharmaceutically acceptable liquid carrier preferably contains a saccharide other than trehalose. The pharmaceutical composition can further comprise about 0.05-3 mM (e.g., about 0.05-2 mM, about 0.05-1.8 mM, about 0.05-1.5 mM, about 0.5-1.5 mM, or about 0.05-1 mM), preferably about 1 mM or about 2 mM, of a divalent metal salt and/or a cationic polymer to further stabilize the pharmaceutical composition. Desirably, the divalent metal salt is MgCl₂. In addition, the pharmaceutical composition can further comprise a suitable amount of NaCl, such as about 50-150 mM, preferably about 75 mM NaCl. The liquid carrier typically is water, preferably water suitable for infection (WFI). Suitable formulations of the pharmaceutical composition are further described in U.S. Patents 6,225,289 and 6,514,943, U.S. Patent Application Publication Nos. 2002/0019041 A1 and 2003/0153065 A1, and International Patent Applications WO 00/34444 and WO 03/59292. The adenoviral vector most preferably is dispersed in an aqueous formulation buffer containing 5% (wt./vol.) trehalose, 0.0025% (wt./vol.) polysorbate 80, 1 mM MgCl₂, 75 mM NaCl, and 10 mM Tris HCl (pH 7.6 ± 0.02).

The pharmaceutical composition can further be formulated to reduce adherence loss of the adenoviral vector on devices used to prepare, store, or administer the expression vector, such as glassware, syringes, catheters, or needles. Use of such a pharmaceutical composition will extend the shelf life of the pharmaceutical composition, facilitate administration, and increase the efficacy of the inventive method. In this regard, the pharmaceutical composition also can be formulated to enhance the spread of the adenoviral vector throughout the target tissue and/or enhance transduction efficiency. To this end, the pharmaceutical composition also can comprise hyaluronidase, which has been shown to enhance uptake of adenoviral vectors. Addition of proteases to the pharmaceutical composition can enhance the spread of the adenoviral vector throughout the tumor. It is desirable to formulate the composition such that the adenoviral vector (e.g., replication-deficient adenoviral vector) remains in the target tissue and does not leak into surrounding normal tissue, i.e., to increase retention of the pharmaceutical composition in the target tissue. Accordingly, agents that increase viscosity of the pharmaceutical composition, such as stimuli-sensitive polymers, can be included. Alternatively, the adenoviral vectors of the pharmaceutical composition can be bound to biocompatible solid carriers, such as particulate carriers (e.g., beads, wafers, etc.), that remain in the target tissue due to size, or incorporated into a matrix, such as gel or foam.

In addition, the pharmaceutical composition can comprise additional therapeutic or biologically-active agents. For example, therapeutic factors useful in the treatment of a particular indication can be present. Factors that control inflammation, such as ibuprofen or steroids, can be part of the pharmaceutical composition to reduce swelling and inflammation associated with *in vivo* administration of the adenoviral vector and physiological distress. Immune system suppressors can be administered with the pharmaceutical composition to reduce any immune response to the adenoviral vector itself or associated with a disorder. Alternatively, immune enhancers can be included in the pharmaceutical composition to upregulate the body's natural defenses against disease. Moreover, cytokines other than TNF-α can be administered with the pharmaceutical composition to attract immune effector cells to the tumor site.

Anti-angiogenic factors, such as soluble growth factor receptors (e.g., sflt), growth factor antagonists, i.e., angiotensin, and the like, also can be part of the pharmaceutical composition. Similarly, vitamins and minerals, anti-oxidants, and micronutrients can be co-administered with the pharmaceutical composition. Antibiotics, i.e., microbicides and fungicides, can be present to reduce the risk of infection associated with gene transfer procedures and other disorders. The addition of chemotherapeutic agents to the pharmaceutical composition can provide an additional mechanism of effecting tumor reduction.

### Target Tissue

The inventive method can be used to deliver a nucleic acid sequence encoding a human TNF-α to any target tissue (e.g., tumor tissue) able to be transduced by adenoviral vectors, such as those adenoviral vectors described herein. Preferably, the target tissue comprises TNF-α receptors, such that TNF-α can exert its biological activity on the tissue. Ideally, the target tissue is a tumor, e.g., a solid tumor or a tumor associated with soft tissue (i.e., soft tissue sarcoma), in a human. The term "tumor" refers to both tumor cells and associated stromal cells. The tumor can be associated with cancers of (i.e., located in) the oral cavity and pharynx, the digestive system, the respiratory system, bones and joints (e.g., bony metastases), soft tissue, the skin (e.g., melanoma), breast, the genital system, the urinary system, the eye and orbit, the brain and nervous system (e.g., glioma), or the endocrine system (e.g., thyroid) and is not necessarily the primary tumor. Tissues associated with the oral cavity include, but are not limited to, the tongue and tissues of the mouth. Cancer can arise in tissues of the digestive system including, for example, the esophagus, stomach, small intestine, colon, rectum, anus, liver, gall bladder, and pancreas. Cancers of the respiratory system can affect the larynx, lung, and bronchus and include, for example, non-small cell lung carcinoma. Tumors can arise in the uterine cervix, uterine corpus, ovary vulva, vagina, prostate, testis, and penis, which make up the male and female genital systems, and the urinary bladder, kidney, renal pelvis, and ureter, which comprise the urinary system. The target tissue also can be associated with lymphoma (e.g., Hodgkin's disease and Non-Hodgkin's lymphoma), multiple myeloma, or leukemia (e.g., acute lymphocytic leukemia, chronic lymphocytic leukemia, acute myeloid leukemia, chronic myeloid leukemia, and the like).

The tumor can be at any stage. The term "tumor stage" is used in the art to describe the tumor type and the degree of tumor spread. Several tumor staging systems are known in the art, and any suitable staging system can be used to determine the stage of the tumor to be treated. For example, the TNM (Tumor, Node, Metastasis) system is an internationally accepted system that is used frequently to determine tumor stage. In this regard "T" describes the size, depth, and area of the primary tumor. "TX" indicates that the primary tumor cannot be assessed. "T0" indicates that there is no evidence of a primary tumor. "Tis" indicates carcinoma in situ (i.e., the malignant cells are confined to the epithelial layer of the tissue). "T1" indicates a localized tumor two centimeters (cm) or less in diameter and confined to the organ of origin. "T2" indicates a localized tumor less than 5 cm in diameter that extends into adjacent tissue of the same organ. "T3" indicates an advanced tumor greater than 5 cm in diameter with greater involvement of adjacent tissue of the same organ, and "T4" indicates a massive tumor that extends into nerves, blood vessels, bone, or another organ. "N" describes whether the cancer has spread to regional lymph nodes. In this respect, "NX" indicates that regional lymph nodes cannot be assessed. "N0" indicates that there is no evidence of metastases to regional lymph nodes, and stages "N1," "N2," and "N3" indicate increasing involvement of regional lymph nodes. The "M" classification describes the presence or absence of distant metastases. In this regard, "MX" indicates that distant metastases cannot be assessed. "M0" indicates that there is no evidence of metastases, and "M1" indicates the presence of distant metastases. Once a tumor has been classified according to the TNM system, each classification can be combined, and an overall stage of I, II, III, or IV can then be assigned to the tumor. While the above description of the TNM system applies generally to most tumor types, the specific definition of each level can vary depending on the type of cancer. Further information about tumor staging is described in, for example, AJCC Cancer Staging Manual, 6th ed., American College of Surgeons, Lippincott-Raven. Philadelphia (2002).

The inventive method also is useful in treating tumors of any grade. The "grade" of a tumor refers to the degree of differentiation of tumor cells. Tumor grade is typically assessed by histological characterization of a tumor sample and determination of the growth rate of the tumor cells (such as by measuring the mitotic index). In general, tumor cells that are well-differentiated resemble normal cells and are of a lower grade (e.g., Grade 1 or 2), while undifferentiated tumor cells are typically more aggressive and are of a higher grade (e.g., Grade 3 or 4).

The tumor can be subject to different therapies. In this regard, the inventive method is useful in treating tumors (i.e., destruction of tumor cells or reduction in tumor size) that have been proven to be resistant to other forms of cancer therapy, such as radiation-resistant tumors. The tumor also can be of any size. Indeed, the invention is predicated, in part, on the surprising ability of the inventive method to significantly reduce the size of initially large tumors (e.g., 42 cm² or 4400 cm³). Ideally, in treating the human for cancer, the inventive method results in cancerous (tumor) cell death and/or reduction in tumor size. It will be appreciated that tumor cell death can occur without a substantial decrease in tumor size due to, for instance, the presence of supporting cells, vascularization, fibrous matrices, etc. Accordingly, while reduction in tumor size is preferred, it is not required in the treatment of cancer.

The target tissue can be a tumor that is amenable to surgical removal (i.e., "resection"). In this respect, the inventive method can be used following surgical resection, as described herein, to eliminate any residual tumor cells. Alternatively, the target tissue can be a tumor that is surgically unresectable. In this case, the inventive method can be used to effect shrinkage of the tumor, thereby facilitating surgical resection. In one embodiment of the invention, the target tissue preferably is a tumor associated with unresectable, locally advanced pancreatic cancer (LAPC), which is defined as a pancreatic tumor having vascular involvement and impingement of major blood vessels, but having no involvement of colon, adrenals, or kidneys, peritoneal seeding (as observed radiographically), and no metastatic disease. The inventive method also is suitable for the treatment of locally advanced resectable esophageal cancer, which is defined as a stage II or III resectable adenocarcinoma or squamous cell carcinoma of the esophagus. In addition, the inventive method can be used to treat locally advanced resectable rectal cancer, which is defined as an adenocarcinoma of the rectum located below the peritoneal reflection, and excluding Paget's disease of the anus. The inventive method also is suitable for the treatment of recurrent glioblastoma multiforme (GBM), which also is referred to as grade IV astrocytoma and develops from glial (supportive) tissue of the brain. The inventive method, however, is not limited to these particular tumor types. Indeed, as discussed above, the inventive method is useful for treating any tumor type.

### Methods of Administration

The inventive method comprises administering the pharmaceutical composition to a human for the treatment of cancer. The use of TNF-α protein in humans as an anti-cancer/anti-tumor agent has been limited by severe systemic effects arising as a result of treatment. Therefore, in order to attenuate or prevent such negative side effects, it is desirable to administer the nucleic acid sequence encoding a human TNF-α only to the tissue which requires it (i.e., the tumor) or, at the very least, to promote expression of the nucleic acid sequence predominantly in tumor tissue. Accordingly, the pharmaceutical composition can be administered by any means such that TNF-α is produced within, or in close proximity to, the target tissue.

The invention preferably involves contacting target tissue by administering the pharmaceutical composition comprising the adenoviral vector (e.g., the replication-deficient adenoviral vector) comprising a nucleic acid sequence encoding a human TNF-α in a localized manner to the target tissue. While any suitable means of administering the pharmaceutical composition to the target tissue (e.g., a tumor) can be used within the context of the invention, preferably localized administration to the tumor is accomplished by directly injecting the pharmaceutical composition comprising the adenoviral vector comprising the nucleic acid sequence encoding a human TNF-α into a tumor. By the term "injecting," it is meant that the pharmaceutical composition is forcefully introduced into the target tissue. Preferably, the pharmaceutical composition is injected percutaneously (i.e., through the skin). The particular route used to inject the pharmaceutical composition into the target tissue will depend on the location of the target tissue in the body. In this regard, for example, the pharmaceutical composition preferably is administered by a transabdominal route when the target tissue is a pancreatic tumor. In addition, the pharmaceutical composition preferably is administered by an endorectal or a transperineal route when the target tissue is a rectal tumor. Of course, administration of the pharmaceutical composition can be accomplished via any route that efficiently delivers the adenoviral vector to the target tissue.

The pharmaceutical composition is desirably administered in a uniform manner across the tumor mass. Thus, when administering the pharmaceutical composition via injection, the injection is performed to maximize distribution throughout the tumor. For example, the needle of the injection device punctures the surface of the tumor tissue (commonly at a depth of about 1 cm to about 5 cm, preferably about 2 cm to about 4 cm (e.g., 3 cm) depending on tumor volume), and the pharmaceutical composition is expelled from the delivery device as the needle is slowly removed from the tissue, such that vector is released along the track of the needle. In addition, timing of each injection can be controlled to evenly deliver the pharmaceutical composition to the tumor. In other words, the pharmaceutical composition can be expelled from the injection device slowly (e.g., about 5 seconds per injection, preferably about 10 seconds per injection, and more preferably about 15 seconds per injection) for efficient uptake of the adenoviral vector by the surrounding tissue. In one embodiment, from about 1 µl to about 10 µl of pharmaceutical composition is administered to the tumor over about 10 minutes to about 30 minutes (e.g., over about 10 minutes, over about 20 minutes, or over about 30 minutes). Alternatively, the pharmaceutical composition can be quickly and forcefully expelled from the injection device (e.g., in a "spray") in order to force vector uptake. Indeed, the pharmaceutical composition can be administered such that the pharmaceutical composition transverses normal tissue prior to contacting target tissue. Of course, homogenous distribution of the pharmaceutical composition across the target tissue is not required.

In instances wherein a tumor is not readily visible through the skin, a clinician can use medical equipment to visualize the tumor, estimate tumor volume, and guide injection placement. Imaging technology for cancer diagnosis is quite advanced. Ultrasound and computed tomography (CT) equipment are commonly used to detect tumor masses. However, more advanced imaging techniques have been developed and include positron emission tomography (PET), which reveals the metabolic activity of tissues, single photon emission computed tomography (SPECT), spiral CT, magnetic resonance imaging (MRI), and endoscopic ultrasonagraphy (EUS), which employs a fiber-optic endoscope fitted with a probe.

Any suitable injection device can be used within the context of the invention.
For example, the common medical syringe can be used to directly inject the pharmaceutical composition into a subcutaneous tumor. Medical syringes can be fitted with any suitable injection needle. Suitable injection needles include, but are not limited to, Winston-Cook injection needles (e.g., the Varices Injector) (Winston-Salem, North Carolina), the EchoTip^{®} Ultrasound needle, and the Quadra-Fuse injection needle (Rex Medical, Conshohocken, PA). Of course, standard medical syringes can be used to inject target tissue (e.g., tumors) not directly beneath the skin. If the tumor is not readily accessible, the tumor can be exposed during a surgical procedure to allow injection. However, minimally invasive delivery devices allow administration of the pharmaceutical composition without invasive medical procedures. Such devices are capable of accessing target tissues not directly accessible through the skin, for example, via small incisions of less than 5 inches. Minimally invasive injection devices can comprise injector tips which are flexible and steerable to allow access via small incisions to the curved outer surface of an organ, e.g., the liver. An alternative means of non-invasive injection comprises the use of a needleless injection device, such as the Biojector 2000 Needle-Free Injection Management System® available from Bioject, Inc. The pharmaceutical composition can be administered to a target tissue using a catheter. For example, transcatheter chemoembolization involves insertion of a catheter into the target tissue through a small incision in the skin. Angiography is used to guide the catheter into the vasculature feeding the target tissue, whereupon the therapeutic agent is released together with particulate matter that blocks the flow of blood to the target tissue. The therapeutic agent does not escape into surrounding tissues, and the tumor is starved from nutrients. Endoscopy is similar to catheterization while permitting visualization of the target tissue while administering the pharmaceutical composition. Catheters are useful in both releasing the pharmaceutical composition in to the blood stream that feeds the tumor, as well as injecting the pharmaceutical composition into tumor tissue. To allow for multiple injections with a specific geometry, a marking system can be employed so that the sites of previous injections are well delineated. Enhanced tumor and injection visualization is especially warranted when the pharmaceutical composition is administered to tumor tissue via multiple applications in a distinct geometrical pattern.

In addition to directly injecting the pharmaceutical composition into tumor tissue, the pharmaceutical composition can be topically applied to tumor tissue. For example, the tumor tissue can be bathed in the pharmaceutical composition, thereby effecting gene transfer to the tissue surface and surrounding environment. Of course, more than one route of administration can be used such that, for example, the pharmaceutical composition is injected and topically administered to a tumor tissue. Topical administration comprising bathing the tumor in the pharmaceutical composition allows exposure of the relevant tissue to the adenoviral vector for a longer period of time than commonly allowed by injection. Exposure of the tissue to the pharmaceutical composition for a prolonged period of time can increase the level of uptake in target cells and supporting cells.
Likewise, the target tissue can be perfused with the pharmaceutical composition over a period of time using any suitable delivery device, e.g., a catheter. A tumor can be perfused with the pharmaceutical composition over any period of time so long as transduction occurs, e.g., from about 1 minute to about 20 hours (e.g., from about 1 minute to about 12 hours), preferably from about 15 minutes to about 10 hours (e.g., from about 15 minutes to about 6 hours), more preferably from about 30 minutes to about 5 hours (e.g., from about 30 minutes to about 3 hours), and more preferably from about 1-2 hours.

While less preferred, other routes of administration can be used to administer the pharmaceutical composition. Indeed, although more than one route can be used to administer the pharmaceutical composition, a particular route can provide a more immediate and more effective reaction than another route. For example, while not particularly preferred, the pharmaceutical composition can be applied or instilled into body cavities, absorbed through the skin, inhaled, or administered parenterally via, for instance, intramuscular, intravenous, peritoneal, or intraarterial administration. Preferably, the adenoviral vector of the pharmaceutical composition parenterally administered to a patient is specifically targeted to particular cells, e.g., cancer cells. For regional delivery, the pharmaceutical composition can be administered intraarterially or intravenously, e.g., via the hepatic artery for delivery to the liver or the carotid artery for delivery to the brain. For administration to the brain, the pharmaceutical composition can be introduced into tumor tissue using an intratumoral delivery catheter, ventricular shunt catheter attached to a reservoir (e.g., Ommaya reservoir), infusion pump, or introduced into a tumor resection cavity (such as Gliasite, Proxima Therapeutics). Tumor tissue in the brain also can be contacted by administering the pharmaceutical composition via convection using a continuous infusion catheter or through cerebrospinal fluid. Alternatively, the pharmaceutical composition can be administered to the pleural cavity for delivery to the lung or the peritoneal cavity for mesothelioma. In addition, the lymphatic system can be accessed, for example, following intratumoral injection, for delivery to lymph nodes. For regional delivery, the pharmaceutical composition is preferably delivered directly into an artery feeding the target tissue. As discussed herein, an adenoviral vector can be modified to alter the binding specificity or recognition of the adenoviral vector for a receptor on a potential host cell. With respect to adenovirus, such manipulations can include deletion of regions of the fiber, penton, or hexon, insertions of various native or non-native ligands into portions of the coat protein, and the like. Parenteral administration can require large doses or multiple administrations to effectively deliver the adenoviral vector of the pharmaceutical composition to the appropriate host cells.

In some instances, the pharmaceutical composition need not contact the target tissue directly to realize a therapeutic effect. For example, the pharmaceutical composition can be administered to supporting tissue, whereby either the adenoviral vector or resulting TNF-α is transmitted to target tissue wherein it exerts its biologic effect. For example, it has been surprisingly observed that the administration of the pharmaceutical composition directly to tumor tissue in accordance with the inventive method can promote treatment lesions located in other regions of the body. In other words, delivery of the pharmaceutical composition to a tumor can promote regression of additional (i.e., one or more other) tumors located elsewhere in the body, e.g., metastasis. Accordingly, the inventive method can treat a human for multiple (i.e., two or more) tumors, preferably by reducing the size of multiple tumors in the human body. In particular, multiple tumors located in different regions of the body (e.g., a first tumor in the shoulder and a second tumor in the abdomen) can be treated by delivery of the pharmaceutical composition to a first tumor. In this embodiment, the pharmaceutical composition is administered to a first tumor, which allows treatment of the first tumor and, additionally, one or more other tumors which were not directly administered the pharmaceutical composition. Preferably, tumor cells within the first tumor and one or more additional tumors are destroyed. Also preferably, the size of the first tumor and one or more additional tumors is reduced. A systemic biological effect can, therefore, be achieved via local (direct) or regional delivery (e.g., delivery directly into the regional lymph or blood supply) of the pharmaceutical composition.

The pharmaceutical composition can be administered in or on a device that allows controlled or sustained release of the adenoviral vector, such as a sponge, biocompatible meshwork, mechanical reservoir, or mechanical implant. Implants (see, e.g., U.S. Patent 5,443,505), devices (see, e.g., U.S. Patent 4,863,457), such as an implantable device, e.g., a mechanical reservoir or an implant or a device comprised of a polymeric composition, are particularly useful for administration of the adenoviral vector. The pharmaceutical composition of the inventive method also can be administered in the form of sustained-release formulations (see, e.g., U.S. Patent 5,378,475) comprising, for example, gel foam, hyaluronic acid, gelatin, chondroitin sulfate, a polyphosphoester, such as bis-2-hydroxyethyl-terephthalate (BHET), and/or a polylactic-glycolic acid.

The pharmaceutical composition also can be incorporated into or coat other materials, such as glass or magnetic beads, that are subsequently administered to a patient. For example, the FDA has approved study of the use of radioactive glass beads to deliver concentrated radiation therapy to hidden tissues (e.g., invasive cancers, tumors deep within the body cavity, etc.). Such beads can contain or be coated with the pharmaceutical composition. Alternatively, magnetic beads coated with the pharmaceutical composition can be administered to a patient, and directed to target tissue by placement of a magnet in the vicinity of a tumor. While beads are likely to remain lodged in the body unless forcibly removed, biodegradable delivery devices dissolve into non-toxic end products that are naturally removed from the body. The Gliadel® wafer, for example, is currently used to deliver chemotherapeutic drugs to the site of glioblastoma. Such biodegradation matrices, once implanted, can release the adenoviral vector encoding TNF-α through degradation of the matrix, thereby delivering the therapeutic agent to the relevant site without clinician intervention.

### Multiple Applications of a Single Dose

While administration of a dose of the pharmaceutical composition can be accomplished through a single application (e.g., a single injection or a single topical application to the target tissue), preferably a single dose is administered via multiple applications of the pharmaceutical composition to different points of the target tumor. The multiple applications can number anywhere from about 2 applications to about 50 applications or more (including all integers between 2 and 50), depending on the size and location of the tumor. For example, a single dose can be administered to relevant tissue in 40 separate injections or more. A single dose of pharmaceutical composition preferably is administered in 2, 3, 4, 5, 6, 7, 8, 9, 10 or more applications (e.g., 15, 20, 25, 30, 35, 40, or 45 applications), more preferably about 2-8 applications, and even more preferably about 2-5 applications. The number of applications depends on the tumor location, tumor size, tumor type, and the like. For example, if administering to soft tissue sarcoma, which is typically very large (e.g., 78 cm in its largest dimension), the single dose preferably is administered in about 3-8 applications. For smaller or more compact solid tumors, only 2-5 applications are preferred. Multiple applications provide an advantage over single applications in that they can be manipulated by such parameters as a specific geometry defined by the location on the tumor where each application is administered. The administration of a single dose of the pharmaceutical composition is better controlled using multiple applications, and the effectiveness with which any given dose is administered can be maximized. In this way, too, the adenoviral vector and, ultimately TNF-α production, can be uniformly distributed throughout the tumor.

The specific geometry of the multiple applications is defined by the location on the target tissue, either in two- or three-dimensional space, where each application of the pharmaceutical composition is administered. The location of each application will be dictated by tumor type and volume. The pattern of applications is selected to effect a broad distribution of the replication-deficient adenoviral vector and, ultimately the produced TNF-α protein, to the tumor. With respect to the specific geometry of the multiple applications in two-dimensional space, the specific geometry is defined by a plane (i.e., a cross-section of the target tissue) in which the multiple applications lie. The plane defined by the multiple applications can lie at a constant distance from the surface of the target tissue (i.e., substantially parallel to the surface of the target tissue), or, alternatively, the plane can lie at an angle with respect to the surface of the target tissue. Furthermore, the multiple applications can define any suitable pattern or specific geometry. Therefore, for example, in two-dimensional space, the multiple applications can define a square whereas in three-dimensional space the multiple applications can define a cube. In that the inventive method comprises multiple rounds of therapy (i.e., administration of multiple doses of the pharmaceutical composition over a period of time (therapeutic period), as discussed below), the geometry of the multiple applications desirably is rotated or shifted to achieve maximal distribution of the pharmaceutical composition over the therapeutic period. To aid in consistent, patterned application of the pharmaceutical composition, a grid or template overlay will aid in guiding injections, marking devices can record previous injection points, and imaging techniques can be used to visualize the target tissue.

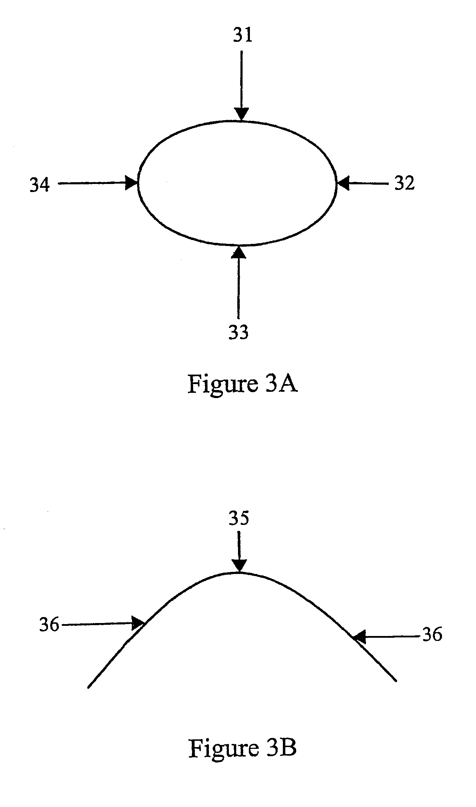

When administering the pharmaceutical composition directly to a solid tumor, a single dose of pharmaceutical composition preferably is administered via multiple applications around the circumference of the tumor, especially wherein the administration device, e.g., a needle, is inserted to a depth of about 1 cm to about 5 cm in the tumor mass, preferably at a depth of about 2 cm to about 4 cm (e.g., about 3 cm). Ideally, the multiple applications are administered to the tumor at equally spaced intervals around the periphery (circumference), e.g., in a clockwise or counter-clockwise pattern (e.g., at the 12 o'clock, 3 o'clock, 6 o'clock, and 9 o'clock positions), and an anterior-posterior or dorsal-caudal position, as illustrated in **Figure 3A** and **Figure 3B**, which depict an end-on view and side-view of a solid tumor, respectively, with an illustration of a preferred pattern for multiple applications of a single dose of pharmaceutical composition to the solid tumor. The geometry of the multiple applications is then shifted in subsequent dose administrations.
For example, in an especially preferred embodiment, the inventive method comprises administering the pharmaceutical composition in the following pattern: (a) 12 o'clock **(31),** 3 o'clock **(32), 6** o'clock **(33),** and 9 o'clock **(34)** positions and an anterior-posterior or dorsal-caudal position **(35)** for each administration in week 1, (b) 1 o'clock, 4 o'clock, 7 o'clock, and 10 o'clock positions and an anterior-posterior or dorsal-caudal position for the first administration in week 2, (c) 2 o'clock, 5 o'clock, 8 o'clock, and 11 o'clock positions and an anterior-posterior or dorsal-caudal position for the second administration in week 2, and (d) repeating (a) - (c) around the periphery of the tumor, using parallel injections **(36),** with each subsequent dose administration.

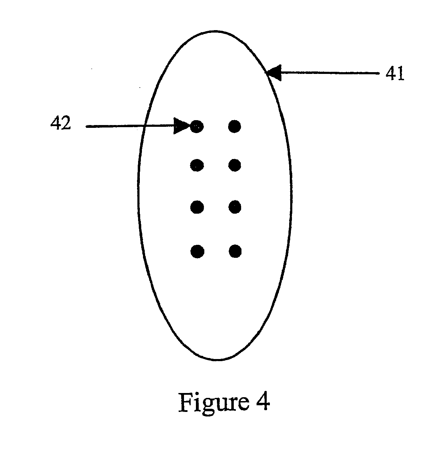

When administering the pharmaceutical composition to a diffuse or large tumor, such as that associated with soft tissue sarcoma, injection into the circumference of the tumor is not possible. Accordingly, the injections are administered in a pattern comprising a series of parallel lines, as illustrated in **Figure 4****,** which depicts an end-on view of a soft tissue sarcoma **(41)** with an illustration of a preferred pattern for multiple applications **(42)** of a single dose of pharmaceutical composition to the soft tissue sarcoma. Alternatively, the injections can be administered in concentric circles. In either case, the specific geometry of applications is selected with the goal of widespread delivery of the adenoviral vector (e.g., replication-deficient adenoviral vector). In some embodiments, a dose of pharmaceutical composition is not administered directly to the tumor tissue, but is locally administered to the surrounding cells/tissue of the tumor, to prevent tumor spread.

Another parameter of the multiple applications which can be manipulated is the time differential between each application. Preferably, each of the multiple applications is administered within about 10 minutes (e.g., about 0.5-10 minutes) of each other, more preferably within about 8 minutes (e.g., about 1-8 minutes) of each other, and even more preferably within about 6 minutes (e.g., about 3-6 minutes) of each other. Most preferably, all of the multiple applications of the single dose are administered within the aforesaid time frames.

### Dosage

The dose of the pharmaceutical composition, and particularly the amount of the adenoviral vector comprising the nucleic acid sequence encoding TNF-α, will depend on a number of factors, including tumor size, the extent of any side-effects, the particular route of administration, and the like. The dosage should be such that the negative side-effects associated with TNF-α are minimized. Desirably, a single dose of the pharmaceutical composition comprises at least about 1x10⁵ particles (which also is referred to as particle units) to at least about 1x10¹³ particles of the adenoviral vector. The dose preferably is at least about 1x10⁶ particles (e.g., about 4x10⁶-4x10¹² particles), more preferably at least about 1x10⁷ particles, more preferably at least about 1x10⁸ particles (e.g., about 4x10⁸-4x10¹¹ particles), and most preferably at least about 1x10⁹ particles to at least about 1x10¹⁰ particles (e.g., about 4x10⁹-4x10¹⁰ particles) of the adenoviral vector. Alternatively, the dose of the pharmaceutical composition comprises no more than about 1x10¹⁴ particles, preferably no more than about 1x10¹³ particles, even more preferably no more than about 1x10¹² particles, even more preferably no more than about 1x10¹¹ particles, and most preferably no more than about 1x10¹⁰ particles (e.g., no more than about 1x10⁹ particles).
In other words, a single dose of pharmaceutical composition can comprise about 1x10⁶ particle units (pu), 4x10⁶ pu, 1x10⁷ pu, 4x10⁷ pu, 1x10⁸ pu, 4x10⁸ pu, 1x10⁹ pu, 4x10⁹ pu, 1x10¹⁰ pu, 4x10¹⁰ pu, 1x10¹¹ pu, 4x10¹¹ pu 1x10¹¹ pu, 4x10¹¹ pu, 1x10¹² pu, or 4x10¹² pu of the adenoviral vector (e.g., the replication-deficient adenoviral vector). Each application of a multiple application protocol for a single dose will include the approximate fraction of the total such that the aggregation of the individual applications equals a single dose as described above. Thus, if there are five applications of a dose of the pharmaceutical composition, the amount of adenoviral vector (e.g., the replication-deficient adenoviral vector) in each application is desirably one-fifth of a single dose as described above.

The dose as described herein is suitable for a volume of targeted tissue of about 3 cm² to about 300 cm² (e.g., about 5 cm², 7 cm², 8 cm², or 9 cm²), typically about 10 cm² to about 200 cm² (e.g., about 15 cm², 20 cm², 25 cm², 30 cm², 35 cm², 40 cm², 45 cm², or 50 cm²), more typically about 50 cm² to about 125 cm² (e.g., 75 cm², 80 cm², 85 cm², 90 cm², or 110 cm²), although tumors smaller than 3 cm² or larger than 300 cm² can be treated with the inventive method. When injecting the pharmaceutical composition directly into a target tissue, a single application can comprise about 100 µl to about 20 ml of the pharmaceutical composition, preferably about 250 µl to about 10 ml, more preferably about 500 µl to about 5 ml (e.g., 1 ml, 2 ml, or 3 ml). Ideally, when administering multiple applications of a single dose via injection, each application contains about 1 ml of pharmaceutical composition.

Thus, in a single dose of the pharmaceutical composition involving, for example, an E1A/E1B/E3/E4-deficient adenoviral vector comprising the nucleic acid sequence encoding a human TNF-α under the control of the EGR-1 promoter (such as that described in International Patent Application WO 02/00906 and U.S. Patent 6,579,522), about 1x10⁸-1x10¹² adenoviral particles are administered to a tumor with an estimated volume of about 3 cm² to about 150 cm². Under these conditions, a substantial level of TNF-α production is achieved in the target tissue without producing the negative side effects associated with systemic administration of the TNF-α protein.

### Timing of Administration

The proliferative nature of cancer impedes the complete elimination of the disease from the body. No matter the treatment, a single dose of a therapeutic agent is not likely to accomplish a complete anti-cancer effect, as surviving cancer cells replicate quickly. Indeed, as with most chronic diseases, prolonged treatment involving multiple doses of a therapeutic agent may be required. Accordingly, in one embodiment, the inventive method comprises delivering multiple doses of pharmaceutical composition over a period of time (i.e., a therapeutic period).

Two timing issues exist with respect to the invention: the total therapeutic period, in other words, the entire length of time over which treatment occurs, and the time between the administration of the doses of pharmaceutical composition and/or adjuvant therapies. Ideally, the therapeutic period is not longer than about 10 weeks in length, such as a therapeutic period comprising about 1-10 weeks in length, although it can be shorter than 1 week (e.g., 1, 2, 3, 4, 5, or 6 day(s)) or longer than 10 weeks (e.g., 12, 14, 16, or 18 weeks). Preferably, the therapeutic period is from about 2-9 weeks (i.e., about 2, 3, 4, 5, 6, 7, 8, or 9 weeks in length), more preferably about 3-8 weeks (e.g., 4-7 weeks), and most preferably about 5-6 weeks in length (e.g., about 5½ weeks). A therapeutic period of 5-7 weeks provides sufficient time to effectively attack the cancer while not being so long as to jeopardize patient compliance. Ideally, the pharmaceutical composition, the dose of ionizing radiation, and the dose of one or more chemotherapeutics are administered concurrently throughout the therapeutic period.

With respect to the administration of multiple doses of the pharmaceutical composition, preferably a dose is administered about once a week throughout the therapeutic period. The start of therapy can require more frequent dosing, e.g., a single dose is administered two days/week, three days/week, four days/week, five days/week, six days/week, or seven days/week (daily administration). In one embodiment, at least two doses are administered during week 1 of the therapeutic period and, if necessary, also during week 2. Thereafter, a single dose of the pharmaceutical composition can be administered each remaining week of the therapeutic period. Ideally, the therapeutic period comprises up to six weeks, and the method comprises administering one dose of pharmaceutical composition in each week of the therapeutic period.

However, as the timing of administrations will rely on several factors, such as tolerance of the patient to the treatment, the extent of the disease, the availability of the clinician, etc., it is acceptable for more than one week to pass between doses. For example, a dose can be administered every 10 days, bi-weekly, every 18 days, tri-weekly, every 25 days, once a month, or bi-monthly. Preferably, the multiple doses of the pharmaceutical composition are administered such that the level of TNF-α remains above background levels during the therapeutic period.

Indeed, the timing of administration is selected such that gene expression (i.e., expression of the human TNF-α coding sequence) occurs, and the desired biological effect is realized. Accordingly, the timing of administration can be optimized for reducing a particular tumor type. For instance, if the tumor is large and diffuse, such as soft tissue sarcoma, the therapeutic period desirably comprises up to five weeks, with two doses of pharmaceutical composition administered in week 1, and one dose of the pharmaceutical composition administered in each subsequent week (e.g., weeks 2-5). For smaller, compact solid tumors, the therapeutic period desirably comprises up to six weeks, with two doses of pharmaceutical composition administered in weeks 1-2, and one dose of pharmaceutical composition administered in each subsequent week (e.g., weeks 3-6). In either case, when two doses of pharmaceutical administration are administered in one week, the doses preferably are administered on day 1 and day 4 of treatment.

In some embodiments, it may be advantageous to employ a method of administering the pharmaceutical composition wherein a dose is continuously administered to the target tissue over a prolonged period of time. For example, continuous perfusion of the target tissue with the pharmaceutical composition may be desirable. Accordingly, the inventive method can comprise contacting the target tissue (e.g., tumor) at least once during the therapeutic period. In other words, a single administration of a dose of pharmaceutical composition is delivered over a prolonged period of time such as, for example, 1-10 weeks in length, although it can be shorter than 1 week (e.g., 1 day, 2 days, 3 days, 4 days, 5 days, 6 days, 2 weeks, 3 weeks, 4 weeks, 5 weeks, 6 weeks, 7 weeks, 8 weeks, or 9 weeks) or longer than 10 weeks (e.g., 12, 14, 16, or 18 weeks). Such a delivery strategy can be accomplished through, for instance, incorporation of the pharmaceutical composition in a sustained-release device or reservoir and implantation into the patient.

### Radiation Therapy

A typical course of treatment for most types of cancer is radiation therapy. Radiation therapy uses a beam of high-energy particles or waves, such as X-rays and gamma rays, to eradicate cancer cells by inducing mutations in cellular DNA. In that cancer cells divide more rapidly than normal cells, tumor tissue is more susceptible to radiation than normal tissue. Radiation also has been shown to enhance exogenous DNA expression in exposed cells. In a preferred embodiment, the inventive method further comprises administering a dose of radiation to a patient over the therapeutic period. Intratumoral delivery of the nucleic acid sequence encoding TNF-α and confocal radiation to the tumor site results in localized delivery of two potent anti-cancer treatment modalities. When the nucleic acid sequence encoding TNF-α is operably linked to a radiation-inducible promoter, radiation potentiates TNF-α production and maintains therapeutic levels of TNF-α at the tumor site continuously throughout the period of radiation therapy, in addition to the additive or synergistic effect of radiation and TNF-α observed in eradicating tumor cells (see, for example, Hersh et al., Gene Therapy, 2, 124-131 (1995), and Kawashita et al., Human Gene Therapy, 10, 1509-1519 (1999)).

Any type of radiation can be administered to a patient, so long as the dose of radiation is tolerated by the patient without significant negative side-effects. Suitable types of radiotherapy include, for example, ionizing (electromagnetic) radiotherapy (e.g., X-rays or gamma rays) or particle beam radiation therapy (e.g., high linear energy radiation). Ionizing radiation is defined as radiation comprising particles or photons that have sufficient energy to produce ionization, i.e., gain or loss of electrons (as described in, for example, U.S. Patent 5,770,581). The effects of radiation can be at least partially controlled by the clinician. The dose of radiation is preferably fractionated for maximal target cell exposure and reduced toxicity. Radiation can be administered concurrently with radiosensitizers that enhance the killing of tumor cells, or with radioprotectors (e.g., IL-1 or IL-6) that protect healthy tissue from the harmful effects of radiation. Similarly, the application of heat, i.e., hyperthermia, or chemotherapy can sensitize tissue to radiation.

The source of radiation can be external or internal to the patient. External radiation therapy is most common and involves directing a beam of high-energy radiation to a tumor site through the skin using, for instance, a linear accelerator. While the beam of radiation is localized to the tumor site, it is nearly impossible to avoid exposure of normal, healthy tissue. However, external radiation is usually well tolerated by patients. Internal radiation therapy involves implanting a radiation-emitting source, such as beads, wires, pellets, capsules, and the like, inside the body at or near the tumor site. Such implants can be removed following treatment, or left in the body inactive. Types of internal radiation therapy include, but are not limited to, brachytherapy, interstitial irradiation, and intracavity irradiation. Internal radiotherapy is particularly suited for cancer treatment using adenoviral vectors in that the pharmaceutical composition of the inventive method can be incorporated in or on the implanted structures, thereby allowing administration of two forms of cancer therapy simultaneously. A less common form of internal radiation therapy is radioimmunotherapy wherein tumor-specific antibodies bound to radioactive material is administered to a patient. The antibodies seek out and bind tumor antigens, thereby effectively administering a dose of radiation to the relevant tissue.

No matter the method of administration, the total dose of radiation administered to a patient in the context of the invention preferably is about 5 Gray (Gy) to about 70 Gy. More preferably, about 10 Gy to about 65 Gy (e.g., about 15 Gy, 20 Gy, 25 Gy, 30 Gy, 35 Gy, 40 Gy, 45 Gy, 50 Gy, 55 Gy, or 60 Gy) or about 10 Gy to about 40 Gy (e.g., about 15 Gy, 20 Gy, 25 Gy, 30 Gy, or 35 Gy) are administered over the course of treatment. While a complete dose of radiation can be administered over the course of one day, the total dose is ideally fractionated and administered over several days, e.g., one fraction of the dose of ionizing radiation is administered each day over the course of several days. Desirably, radiotherapy is administered over the course of at least about 3 days, e.g., at least 4, 5, 7, 10, 14, 17, 21, 25, 28, 32, 35, 38, 42, 46, 52, or 56 days (about 1-8 weeks). Accordingly, a daily dose of radiation will comprise approximately 1-5 Gy (e.g., about 1 Gy, 1.5 Gy, 1.8 Gy, 2 Gy, 2.5 Gy, 2.8 Gy, 3 Gy, 3.2 Gy, 3.5 Gy, 3.8 Gy, 4 Gy, 4.2 Gy, 4.5 Gy, or 5 Gy), preferably 3-5 Gy (e.g., about 3-4 Gy or 4-5 Gy), and more preferably 1-2 Gy (e.g., 1.5-2 Gy). The daily dose of radiation should be sufficient to induce expression of the nucleic acid sequence if operably linked to a radiation-inducible promoter. If stretched over a period of time, radiation preferably is not administered every day, thereby allowing the patient to rest and the effects of the therapy to be realized. For example, radiation desirably is administered on 5 consecutive days, and not administered on 2 days, for each week of treatment, thereby allowing 2 days of rest per week. However, radiation can be administered 1 day/week, 2 days/week, 3 days/week, 4 days/week, 5 days/week, 6 days/week, or all 7 days/week, depending on the response of the patient to therapy and any potential side effects. Radiation therapy can be initiated at any time in the therapeutic period. Preferably, radiation is initiated in week 1 or week 2, and is administered for the remaining duration of the therapeutic period. For example, radiation is administered in weeks 1-6 or in weeks 2-6 of a therapeutic period comprising 6 weeks for treating, for instance, a solid tumor. Alternatively, radiation is administered in weeks 1-5 or weeks 2-5 of a therapeutic period comprising 5 weeks. The treatment of some types of cancer may require an additional dose of ionizing radiation (a "booster" dose) after completion of the initial radiation treatment. In such cases, the booster dose can be any dose suitable to enhance a therapeutic effect, but preferably comprises about 5 to about 10 Gy, more preferably about 5.4 to about 9 Gy.

In some embodiments, such as those instances wherein the nucleic acid sequence encoding TNF-α is operably-linked to a radiation-inducible promoter, the inventive method comprises exposing the tumor to radiation immediately following administration of the pharmaceutical composition in a dose sufficient to upregulate the activity of the promoter, although up to two months can pass between radiation exposure and administration of the pharmaceutical compositions in a human are further described in Cancer Facts and Figures 2001, American Cancer Society, New York, NY, and International Patent Application WO 01/24684. Accordingly, a clinician can use standard tests to determine the efficacy of the various embodiments of the inventive method in treating cancer. However, in addition to tumor size and spread, the clinician also should consider quality of life and survival of the patient in evaluating efficacy of treatment.

Care should be taken such that wounds resulting from the tumor or administration of the pharmaceutical composition (i.e., ulcers) are healed or shielded from excessive radiation exposure. Desirably, at least one application of radiation is administered on the day the pharmaceutical composition is administered. Most preferably, the target site is exposed to radiation about 30 minutes to about 8 hours (e.g., about 3 hours), preferably at least about 4 hours (e.g., about 4-6 hours) following administration of the pharmaceutical composition, especially when the adenoviral vector (e.g., the replication-deficient adenoviral vector) comprises a nucleic acid sequence encoding a human TNF-α, which is operably linked to a radiation-inducible promoter, so as to maximally upregulate activity of the radiation-inducible promoter and the consequent production of TNF-α. Of course, when internal sources of radiation are employed, e.g., brachytheraphy or radioimmunotherapy, the exposure time typically will increase, with a corresponding decrease in the intensity of radiation.

### Chemotherapy

Like radiation, chemotherapy is a standard treatment for most cancer types. In a preferred embodiment, the inventive method further comprises administering a dose of one or more chemotherapeutics to a patient over the therapeutic period. Any suitable chemotherapeutic can be used in the inventive method. Suitable chemotherapeutics include, but are not limited to, adriamycin, asparaginase, bleomycin, busulphan, cisplatin, carboplatin, carmustine, capecitabine, chlorambucil, cytarabine, cyclophosphamide, camptothecin, dacarbazine, dactinomycin, daunorubicin, dexrazoxane, docetaxel, doxorubicin, etoposide, floxuridine, fludarabine, fluorouracil, gemcitabine, hydroxyurea, idarubicin, ifosfamide, irinotecan, lomustine, mechlorethamine, mercaptopurine, meplhalan, methotrexate, mitomycin, mitotane, mitoxantrone, nitrosurea, paclitaxel, pamidronate, pentostatin, plicamycin, procarbazine, rituximab, streptozocin, teniposide, thioguanine, thiotepa, vinblastine, vincristine, vinorelbine, taxol, transplatinum, anti-vascular endothelial growth factor compounds ("anti-VEGFs"), anti-epidermal growth factor receptor compounds ("anti-EGFRs"), 5-fluorouracil, and the like. A dose of one or more chemotherapeutics can be administered according to the inventive method. The type and number of chemotherapeutics used in the inventive method will depend on the standard chemotherapeutic regimen for a particular tumor type. In other words, while a particular cancer may be routinely treated with a single chemotherapeutic agent, another may be routinely treated with a combination of chemotherapeutic agents. Preferably, the inventive method comprises administration of 5-fluorouracil (5-FU), cisplatin, paclitaxel, gemcitabine, cyclophosphamide, capecitabine, and/or doxorubicin. Any suitable dose of the one or more chemotherapeutics can be administered to the human. Suitable doses of the chemotherapeutics described above are known in the art, and are described in, for example, U.S. Patent Application Publication No. 2003/0082685 A1. In embodiments where a dose of 5-FU is administered to the patient, the dose preferably comprises about 50 mg per m² of body surface area of the patient per day (i.e., mg/m²/day) to about 1500 mg/m²/day (e.g., about 100 mg/m²/day, about 500 mg/m²/day, and about 1000 mg/m²/day). More preferably, the dose of 5-FU comprises about 100 mg/m²/day to about 300 mg/m²/day (e.g., 200 mg/m²/day) or about about 900 mg/m²/day to about 1100 mg/m²/day (e.g., about 1000 mg/m²/day). When a dose of cisplatin is administered to the patient, the dose preferably comprises about 25 mg/m²/day to about 500 mg/m²/day (e.g., about 50 mg/m²/day, about 100 mg/m²/day, or about 300 mg/m²/day). More preferably, the dose of cisplatin is about 50-100 mg/m²/day, most preferably 75 mg/m²/day. When a dose of capecitabine is administered to the patient, the dose preferably comprises about 500 mg/m²/day to about 1500 mg/m²/day (e.g., about 700 mg/m²/day, about 800 mg/m²/day, or about 900 mg/m²/day). More preferably, the dose of capecitabine comprises about 800 mg/m²/day to about 1000 mg/m²/day (e.g., about 900 mg/m²/day).

As with radiation, if stretched over a period of time, chemotherapy is not administered every day, thereby allowing the patient to rest and the effects of the therapy to be realized. For example, chemotherapy desirably is administered on 5 consecutive days, and not administered on 2 days, for each week of treatment, thereby allowing 2 days of rest per week. However, chemotherapy can be administered 1 day/week, 2 days/week, 3 days/week, 4 days/week, 5 days/week, 6 days/week, or all 7 days/week, depending on the response of the patient to therapy and any potential side effects. For example, in one embodiment of the inventive method, the administration of the one or more chemotherapeutic agents begins on each of two separate days of the therapeutic period. In this regard, the dose of the one or more therapeutics can begin on any two days of the therapeutic period. In a particularly preferred embodiment, a dose of 5-FU and cisplatin are administered to the patient beginning on day 1 and day 29 of a 5 week therapeutic period.

Chemotherapy can be initiated at any time in the therapeutic period. Preferably, chemotherapy is initiated in week 1 on the same day radiation therapy is initiated (denoted "day 1") and is administered for the duration of the therapeutic period. For example, chemotherapy is administered in weeks 1-6 or in weeks 2-6 of a therapeutic period comprising 6 weeks for treating, for instance, a solid tumor. Alternatively, chemotherapy is administered in weeks 1-5 or weeks 2-5 of a therapeutic period comprising 5 weeks. The dose of the one or more chemotherapeutic agents is administered after the dose of ionizing radiation. Most preferably, the patient is exposed to chemotherapy within about 24 hours (e.g., about 12 hours before or after) of exposure to the dose of ionizing radiation.

In some embodiments, it may be advantageous to employ a method of administering the one or more chemotherapeutics wherein a dose is continuously administered to the patient over a prolonged period of time. For example, continuous infusion of the patient with the chemotherapeutic may be desirable. In this regard, the duration of the administration of the dose of the one or more chemotherapeutics may be any suitable length of time. Standard infusion rates for the chemotherapeutics described herein are known in the art and can be used in the inventive method, or modified in any suitable manner according to the nature of the disease. For example, when the inventive method is practiced using 5-FU as a chemotherapeutic, a typical infusion rate is about 96 hours per treatment week (i.e., 5 days per week). Other aspects of cancer chemotherapy and dosing schedules are described in, for example, Bast et al. (eds.), Cancer Medicine, 5th edition, BC. Decker Inc., Hamilton, Ontario (2000).

### Surgical Resection

In one embodiment, the pharmaceutical composition is administered before, during, or after surgical resection of a tumor. Complete surgical removal of tumor tissue is often complicated by invasion of the tumor tissue into surrounding tissues and indefinite margins of the mass. As described herein, treatment of a tumor using the inventive method leads to tumor shrinkage, which will facilitate resection. Ideally, surgical resection of a tumor is performed after completion of the therapeutic period. Surgical resection of a tumor can be performed at any time after completion of the therapeutic period, so long as the patient is allowed sufficient time to recover from the administration of the pharmaceutical composition, ionizing radiation, and chemotherapy. Desirably, surgical resection of a tumor is performed at least 1 week after completion of the therapeutic period. Preferably, surgical resection of a tumor is performed about 3-15 weeks (e.g., about 4 weeks, 5 weeks, 6 weeks, 7 weeks, 8 weeks, 9 weeks, 10 weeks, 11, weeks, 12 weeks, 13 weeks, or 14 weeks) after completion of the therapeutic period. More preferably, surgical resection of a tumor is performed about 3-6 weeks (e.g., about 4 weeks or 5 weeks) or about 4-10 weeks (e.g., about 6 weeks, 7 weeks, or 8 weeks) after completion of the therapeutic period.

Often, post-surgical administration of the pharmaceutical composition (using the inventive method) can eliminate any residual tumor cells remaining after surgical resection of a tumor. Adjuvant radiation and/or chemotherapy are also often part of standard therapeutic regimens following surgical resection of tumor tissue. Thus, post-surgical administration of the pharmaceutical composition, a dose of ionizing radiation, and/or a dose of one or more chemotherapeutics (using the inventive method) also can be used to eliminate any residual tumor cells. Adjuvant radiation and/or chemotherapy can be administered at any time following surgical resection of the tumor, so long as the patient is allowed sufficient recovery time after surgery. In one embodiment, adjuvant chemotherapy is administered to the patient at least 1 week following surgical resection of the tumor. Preferably, adjuvant chemotherapy is administered about 1 week to about 10 weeks (e.g., about 3 weeks, about 5 weeks, or about 7 weeks) following surgical resection of a tumor, more preferably about 2 weeks to about 4 weeks (e.g., about 3 weeks) following surgical resection of a tumor. Any one or combination of chemotherapeutics, such as those described herein, can be administered to the patient in any suitable dose as part of adjuvant chemotherapy following surgical resection of a tumor. In one embodiment, adjuvant chemotherapy comprises administration of 5-FU and the folic acid derivative leucovorin to the patient.

### Evaluation of Treatment Efficacy

The inventive method provides an efficient and safe therapeutic regimen for delivering TNF-α to a human for the prophylactic or therapeutic treatment of disease. Ideally, the inventive method promotes inhibition of tumor cell proliferation, the inhibition of tumor vascularization, the eradication of tumor cells, and/or a reduction in the size of at least one tumor such that a human is treated for cancer. By "treatment of cancer" is meant alleviation of cancer in whole or in part. Several parameters to be considered in administering a human TNF-α to a human patient using adenoviral-mediated gene transfer are discussed herein. The proper combination of parameters for a particular situation can be established by the clinician. The progress of the inventive method in treating cancer (e.g., reducing tumor size or eradicating cancerous cells) can be ascertained using any suitable method, such as those methods currently used in the clinic to track tumor size and cancer progress. The primary efficacy parameter used to evaluate the treatment of cancer by the inventive method preferably is a reduction in the size of a tumor. Tumor size can be figured using any suitable technique, such as measurement of dimensions, or estimation of tumor volume using available computer software, such as FreeFlight software developed at Wake Forest University that enables accurate estimation of tumor volume. Tumor size can be determined by tumor visualization using, for example, CT, ultrasound, SPECT, spiral CT, MRI, photographs, and the like. In embodiments where a tumor is surgically resected after completion of the therapeutic period, the presence of tumor tissue and tumor size can be determined by gross analysis of the tissue to be resected, and/or by pathological analysis of the resected tissue.

Desirably, the growth of a tumor is stabilized (i.e., tumor growth does not increase or decrease) as a result of the inventive method. Preferably, the inventive method reduces the size of a tumor at least about 5% (e.g., at least about 10%, 15%, 20%, or 25%). More preferably, tumor size is reduced at least about 30% (e.g., at least about 35%, 40%, 45%, 50%, 55%, 60%, or 65%). Even more preferably, tumor size is reduced at least about 70% (e.g., at least about 75%, 80%, 85%, 90%, or 95%). Most preferably, the tumor is completely eliminated.

The effect of the inventive method on tumor size can also be classified in terms of tumor response. In this regard, a more than 50% reduction in the volume of a tumor treated in accordance with the inventive method (i.e., a "treated tumor") indicates a "partial response" (PR). The growth of a treated tumor to more than 125% of the size of the tumor before the therapeutic period indicates "progressive disease" (PD). By "before the therapeutic period" is meant a time shortly or immediately before commencement of the therapeutic period. The complete disappearance of a treated tumor for at least four weeks after the observation of the disappearance of the tumor indicates a "complete response" (CR). When the size of a treated tumor is between 75% and 125% of the size of the tumor before the therapeutic period, the tumor response is referred to as "stable disease" (SD). A "marginal response" (MR) is indicated by a reduction in treated tumor size of between 50% and 75% of the size of the tumor before the therapeutic period. Thus, the primary efficacy parameter for the inventive method can also be described as an "objective tumor response," which is defined as a partial response or a complete response. While a partial response or a complete response is preferred, treatment of a cancer in a patient by the inventive method also can be evidenced by stable disease.

When a tumor is subject to surgical resection following completion of the therapeutic period, the efficacy of the inventive method in reducing tumor size can be determined by measuring the percentage of resected tissue that is necrotic (i.e., dead). In this regard, a cancer is treated if the necrosis percentage of the resected tissue is greater than about 50% (e.g., about 60%, 70%, 80%, 90%, or 100%), more preferably about 90% or greater (e.g., about 90%, 95%, or 100%). Most preferably, the necrosis percentage of the resected tissue is 100%, that is, no tumor tissue is present.

A number of secondary parameters can be employed to determine the efficacy of the inventive method. Examples of secondary parameters include, but are not limited to, detection of new tumors, detection of tumor antigens or markers (e.g., CEA, PSA, or CA-125), biopsy, surgical downstaging (i.e., conversion of the surgical stage of a tumor from unresectable to resectable), PET scans, survival, disease progression-free survival, time to disease progression, quality of life assessments such as the Clinical Benefit Response Assessment, and the like, all of which can point to the overall progression (or regression) of cancer in a human. Biopsy is particularly useful in detecting the eradication of cancerous cells within a tissue. Radioimmunodetection (RAID) is used to locate and stage tumors using serum levels of markers (antigens) produced by and/or associated with tumors ("tumor markers" or "tumor-associated antigens"), and can be useful as a pre-treatment diagnostic predicate, a post-treatment diagnostic indicator of recurrence, and a post-treatment indicator of therapeutic efficacy. Examples of tumor markers or tumor-associated antigens that can be evaluated as indicators of therapeutic efficacy include, but are not limited to, carcinembryonic antigen (CEA) prostate-specific antigen (PSA), CA-125, CA19-9, ganglioside molecules (e.g., GM2, GD2, and GD3), MART-1, heat shock proteins (e.g., gp96), sialyl Tn (STn), tyrosinase, MUC-1, HER-2/neu, c-erb-B2, KSA, PSMA, p53, RAS, EGF-R, VEGF, MAGE, and gp100. Other tumor-associated antigens are known in the art. RAID technology in combination with endoscopic detection systems also efficiently distinguishes small tumors from surrounding tissue (see, for example, U.S. Patent 4,932,412).

Desirably, in accordance with the inventive method, the treatment of cancer in a human patient is evidenced by one or more of the following results: (a) the complete disappearance of a tumor (i.e., a complete response), (b) about a 25% to about a 50% reduction in the size of a tumor for at least four weeks after completion of the therapeutic period as compared to the size of the tumor before the therapeutic period, (c) at least about a 50% reduction in the size of a tumor for at least four weeks after completion of the therapeutic period as compared to the size of the tumor before the therapeutic period, and (d) at least a 2% decrease (e.g., about a 5%, 10%, 20%, 30%, 40%, 50%, 60%, 70%, 80% or 90% decrease) in a specific tumor-associated antigen level at about 4-12 weeks after completion of the therapeutic period as compared to the tumor-associated antigen level before the therapeutic period. While at least a 2% decrease in a tumor-associated antigen level is preferred, any decrease in the tumor-associated antigen level is evidence of treatment of a cancer in a patient by the inventive method. For example, with respect to unresectable, locally advanced pancreatic cancer, treatment can be evidenced by at least a 10% decrease in the CA19-9 tumor-associated antigen level at 4-12 weeks after completion of the therapeutic period as compared to the CA19-9 level before the therapeutic period. Similarly, with respect to locally advanced rectal cancer, treatment can be evidenced by at least a 10% decrease in the CEA tumor-associated antigen level at 4-12 weeks after completion of the therapeutic period as compared to the CEA level before the therapeutic period.

With respect to quality of life assessments, such as the Clinical Benefit Response Criteria, the therapeutic benefit of the treatment in accordance with the invention can be evidenced in terms of pain intensity, analgesic consumption, and/or the Karnofsky Performance Scale score. The Karnofsky Performance Scale allows patients to be classified according to their functional impairment. The Karnofsky Performance Scale is scored from 0-100. In general, a lower Karnofsky score is predictive of a poor prognosis for survival. Thus, the treatment of cancer in a human patient alternatively, or in addition, is evidenced by (a) at least a 50% decrease (e.g., at least a 60%, 70%, 80%, 90%, or 100% decrease) in pain intensity reported by a patient for any consecutive four week period in the 12 weeks after completion of the therapeutic period, as compared to the pain intensity reported by the patient before the therapeutic period, (b) at least a 50% decrease (e.g., at least a 60%, 70%, 80%, 90%, or 100% decrease) in analgesic consumption reported by a patient for any consecutive four week period in the 12 weeks after completion of the therapeutic period as compared to the analgesic consumption reported by the patient before the therapeutic period, and/or (c) at least a 20 point increase (e.g., at least a 30 point, 50 point, 70 point, or 90 point increase) in the Karnofsky Performance Scale score reported by a patient for any consecutive four week period in the 12 weeks after completion of the therapeutic period as compared to the Karnofsky Performance Scale score reported by the patient before the therapeutic period.

The treatment of a cancer in a human patient desirably is evidenced by one or more (in any combination) of the foregoing results, although alternative or additional results of the referenced tests and/or other tests can evidence treatment efficacy.

In accordance with the invention, tumor size is reduced as a result of the inventive method preferably without significant adverse events in the human. Adverse events are categorized or "graded" by the Cancer Therapy Evaluation Program (CTEP) of the National Cancer Institute (NCI), with Grade 0 representing minimal adverse side effects and Grade 4 representing the most severe adverse events. The NCI toxicity scale (published April 1999) and Common Toxicity Criteria Manual (updated August 1999) is available through the NCI, e.g., through the NCI internet website at http://www.ctep.info.nih.gov or in the *Investigator's Handbook* for participants in clinical trials of investigational agents sponsored by the Division of Cancer Treatment and Diagnosis, NCI (updated March 1998). Desirably, the inventive method is associated with minimal adverse events, e.g. Grade 0, Grade 1, or Grade 2 adverse events, as graded by the CTEP/NCI. However, as discussed herein, reduction of tumor size, although preferred, is not required in that the actual size of tumor may not shrink despite the eradication of tumor cells. Eradication of cancerous cells is sufficient to realize a therapeutic effect. Likewise, any reduction in tumor size is sufficient to realize a therapeutic effect.

Detection, monitoring, and rating of various cancers in a human are further described in Cancer Facts and Figures 2001, American Cancer Society, New York, NY, and International Patent Application WO 01/24684. Accordingly, a clinician can use standard tests to determine the efficacy of the various embodiments of the inventive method in treating cancer. However, in addition to tumor size and spread, the clinician also should consider quality of life and survival of the patient in evaluating efficacy of treatment.

### Other Considerations

The inventive method can be performed in combination with other therapeutic methods to achieve a desired biological effect in a patient. In one embodiment, drugs such as H2 blockers and/or proton pump inhibitors can be administered to the patient during the therapeutic period to protect the gastrointestinal system (e.g., the stomach) from side effects of ionizing radiation. The inventive method also can be performed alongside hormone therapy, which is the manipulation of hormone levels in the body to treat disease. Many cancers are somehow affected by the levels of hormones in the body and, as such, typical therapeutics associated with hormone therapy, e.g., tamoxifen, work to reduce the level circulating hormones and/or interrupt the binding of hormones to hormone receptors.

Hyperthermia, by definition, is increasing the body's temperature as a means of therapy. Studies show that hyperthermia is an effective adjuvant therapy by enhancing the effects of chemotherapy, radiotherapy, and immunotherapy (Ito et al., Cancer Gene Therapy, 8(9), 649-654 (2001)). The temperature of specific region of the body, such as a tumor site, can be raised by a heating device (e.g., a device that emits microwaves) or by administering a toxin to induce fever or inflammation. Particles that are induced to emit heat also can be administered to the patient such that the area of treatment is localized and internal. Magnetic particles have been shown to generate heat under an alternating magnetic field (AMF) by hysteresis loss (Ito et al, *supra*). Submicron particles such as these can be administered to the target tissue and activated by AMF. If desired, the nucleic acid sequence encoding TNF-α can be operably linked to a promoter responsive to heat or AMF irradiation, thereby providing an additional safety measure in that TNF-α production is localized to target tissue.

The following examples further illustrate the invention but, of course, should not be construed as in any way limiting its scope.

### EXAMPLE 1

This example demonstrates the safety and efficacy of the pharmaceutical composition of the invention comprising (i) a pharmaceutically acceptable carrier and (ii) an adenoviral vector comprising a nucleic acid sequence encoding TNF-α operably linked to a radiation-inducible promoter, wherein the dose comprises from about 4 x 10⁹ to about 4 x 10¹¹ particle units (pu) of adenoviral vector, in a clinically-relevant animal model.

Preclinical toxicology studies were performed in nude mice (nu/nu) bearing a human SQ-20B squamous cell carcinoma zenograft (n=80) and Balb/c mice (n=100). Ten nu/nu mice/sex/group in four groups totaling 80 mice were used in the study. The four groups consisted of: (1) vehicle control, (2) vehicle control plus radiation, (3) pharmaceutical composition comprising 4 x 10⁹ pu of adenoviral vector plus radiation, and (4) pharmaceutical composition comprising 4 x 10¹⁰ pu of adenoviral vector plus radiation. Doses of vehicle or pharmaceutical composition comprising adenoviral vectors were administered by intratumoral injection (20 µl into 2 sites, 40 µl total) into established SQ-20B human squamous cell tumors from 0.4 cm to 0.6 cm in diameter. Ten Balb/c mice/sex/group with 5 groups totaling 100 mice also were used in the study. The groups included: (1) vehicle control, (2) vehicle plus radiation control, (3) pharmaceutical composition comprising 4 x 10⁹pu of adenoviral vector plus radiation, (4) pharmaceutical composition comprising 4 x 10¹⁰ pu of adenoviral vector plus radiation, and (5) pharmaceutical composition comprising 4 x 10¹¹ pu of adenoviral vector plus radiation. Doses of vehicle or pharmaceutical composition were administered by (i) subcutaneous (s.c.) injection into the right hind limb of each Balb/c mouse or (ii) intratumorally in nu/nu mice on days 0, 3, 7, and 10 in conjunction with radiation doses of 5 Gy/fraction administered daily on Days 0 through 4 and Days 7 through 11 (total dose 50 Gy) starting 4 hr after injection. Radiation was to (i) the right hind limb of Balb/c mice or (ii) the tumor site of nu/nu mice with the rest of the body shielded.

The adenoviral vectors comprised an E1A/E1B/E3/E4-deficient adenoviral serotype 5 viral backbone comprising the coding sequence for human TNF-α located in the E1 region and operably linked to the EGR-1 promoter. The adenoviral vector further comprised a spacer sequence in the E4 region. Clinical cage-side observations, food consumption, hematology and chemistry, and clinical pathology were evaluated. Subjects were necropsied on days 14 and 28.

In addition, a pharmacokinetics study in nude mice (n=33) was performed. The mice were intratumorally injected with a dose of pharmaceutical composition comprising 4x10⁹ pu of the adenoviral vector on days 0-2 or daily 5 days/week for two weeks, with or without radiation therapy as described above. Subjects were sacrificed on days 3 or 28. TNF-α levels in plasma and tumor tissue were quantified by ELISA.

Doses of up to 350 times the maximum dose recommended for clinical studies (on a per kg weight basis) were well tolerated without significant toxicity. Erythema of the skin at the injection site in all dose groups was observed in both Balb/c and nu/nu mice and is consistent with the erythema reported after s.c. administration of soluble recombinant human TNF in humans. The majority of the other toxicities observed were found in the highest dose group (4 x 10¹¹ pu) and were independent of radiation. These toxicities consisted of a local reaction including ulceration, alopecia, and skin discoloration. Any observed systemic reactions were statistically significantly different when comparing the treatment group to the vehicle-treated controls; however, the changes in these parameters compared to normal levels were not considered of toxicological significance. No mice died or had significant toxicities when administered pharmaceutical composition comprising 4 x 10⁹ or 4 x 10¹⁰ pu of adenoviral vector in combination with 50 Gy of radiation. In addition, intratumoral delivery of the pharmaceutical composition in combination with radiation therapy resulted in significant and sustained levels of TNF-α in tumor homogenates without "spill over" into plasma, and demonstrated pronounced anti-neoplastic activity. Indeed, radiation increased intra-tumoral levels of TNF-α 12-fold.

The above-described results demonstrate the safety and efficacy of the inventive pharmaceutical composition and method in animal models deemed in the art as reasonably predictive of success in humans.

### EXAMPLE 2

This example illustrates use of the inventive method to therapeutically treat cancer in a human as indicated by size reduction of tumor mass.

Patients with solid tumors accessible for repeated intratumoral injections were selected for treatment using the inventive method. These patients had failed one or more prior therapies. Patients were injected intra-tumorally with a pharmaceutical composition comprising one of five dose levels of the TNF-α coding sequence-containing adenoviral vector described in Example 1 (4x10⁹ - 4x10¹¹ pu in ½ log increments) over a maximum 6-week therapeutic period. Several patients comprised a lesion treated by radiation only, which served as a control. A single dose of pharmaceutical composition was administered via multiple injections to the tumor on day 1 and day 4 of weeks 1 and 2 of the therapeutic period, and once weekly for weeks 3-6. For each dose, the multiple injections were administered in a pattern such that the injections were equally spaced around the periphery of the tumor. For example, injections were administered to the tumor in the following pattern: 12 o'clock, 3 o'clock, 6 o'clock, and 9 o'clock positions and an anterior-posterior or dorsal-caudal position for each administration in week 1, (b) 1 o'clock, 4 o'clock, 7 o'clock, and 10 o'clock positions, and an anterior-posterior or dorsal-caudal position for the first administration in week 2, (c) 2 o'clock, 5 o'clock, 8 o'clock, and 11 o'clock positions, and an anterior-posterior or dorsal-caudal position for the second administration in week 2, and repeating (a) - (c) around the periphery of the tumor with each subsequent dose administration. Concomitant radiation therapy started on week 2 and was administered for five consecutive days, and not administered for two days, for each remaining week of the therapeutic period, achieving a total dose of 30-70 Gy. Tumor size was calculated at the end of treatment and, if possible, 2-3 months post-treatment

| | | | Tumor Response | |
|---|---|---|---|---|
| Patient | Index Cancer | Lesion Site | % change of tumor size | Response Index |
| 1 | NSCLC | Breast | -62% | PR |
| 2 | Breast | Chest Wall | -100% | CR |
| 3 | Pancreas | Pancreas Bed | -62% | PR |
| 4 | Colon | Peritoneum | -17% | SD |
| 5 | Breast | Supraventrical | -23% | SD |
| 6 | Head/Neck | Mandibular | -17% | SD |
| 7 | Rectal | Rectal | -100% | CR |
| 8 | Head/Neck | Head/Neck | Not Evaluable | Not Evaluable |
| 9 | Breast | Breast | Not Evaluable | Not Evaluable |
| 10 | Pancreas | Pancreas | -29 | MR |
| 11 | Colon | Groin | -38 | MR |
| 12 | NSCLC | Supraclavicular | -60 | PR |
| 13 | Melanoma | Axilla | -100 | CR |
| 14 | Sarcoma | Skin | Not Evaluable | Not Evaluable |
| 16 | Head/Neck | Oropharynx | Not Evaluable | Not Evaluable |
| 17 | Sarcoma | Mediastinum | -47 | MR |
| 18 | Bladder | Axilla | -32 | MR |
| 19 | NSCLC | Axilla | -56 | PR |
| 20 | Pancreas | Pancreas | >125 | PD |
| 21 | Melanoma | Pelvis | | |
| 22 | Breast | Thorax | 0 | SD |
| 23 | Rectal | Rectum | -59 | PR |
| 24 | Colorectal | Pelvis | 0 | SD |
| 25 | Lung | Kidney | >-50 | PR |
| 26 | Melanoma | Axilla | +6 | SD |
| 27 | Pancreas | Jejenum | -50 | PR |

Regarding safety, no dose-limiting toxicity was observed over the range of doses of adenoviral vectors tested. No drug-related serious adverse events were experienced by the patients, while minimal adverse events included injection site pain and chills. Significant increases in TNF-α levels in serum above baseline were not observed (1-50 pg/ml) at all time points tested. No virus was detected in cultures taken from blood or urine, and antibody titer against the vector did not increase in seven of eight patients tested.

Regarding efficacy of the treatment protocol, a summary of the tumor responses is provided in the table above, wherein the following abbreviations have the indicated meanings: NSCLC - Non-small cell lung cancer; PR - partial response, more than 50% reduction in volume of the injected lesion; PD - progressive disease, lesion has grown to more than 125% of pre-study value; CR - complete response, complete disappearance of a lesion for at least four weeks from date of documentation; SD - stable disease, tumor is between 75% and 125% of pre-treatment value; MR - reduction to between 50% and 75% of pre-treatment value. PR and CR are routinely regarded as "objective tumor responses."

An objective tumor response was observed in 57% of patients tested, with 28.5% of patients displaying a complete response and 28.5% of patients displaying a partial response. Some tumor shrinkage was achieved in all patients, averaging a decrease in size of 54% in injected lesions. No shrinkage was observed in control lesions. These results prove the effectiveness of the inventive method to treat a wide range of solid tumors, regardless of location in the body.

### EXAMPLE 3

This example illustrates use of the inventive method to reduce the size of a tumor associated with soft tissue sarcoma.

Patients with extremity soft tissue sarcomas are selected for treatment using the inventive method. Patients are injected intratumorally with a pharmaceutical composition comprising one of five dose levels of the TNF-α coding sequence-containing adenoviral vector described in Example 1 (4x10⁹ - 4x10¹² pu in 1 - ½ log increments) over a 5-week therapeutic period. A single dose of pharmaceutical composition is administered via multiple injections to the tumor on day 1 and day 4 of week 1 of the therapeutic period, and once weekly for weeks 2-5. For each dose, the multiple injections are administered to the tumor in parallel lines. Concomitant radiation therapy is initiated in week 1 and administered for five consecutive days, and not administered for two days, for each week of the therapeutic period, achieving a total dose of 30-70 Gy.

### EXAMPLE 4

This example illustrates use of the inventive method to reduce the size of a tumor associated with unresectable, locally advanced pancreatic cancer (LAPC).

Patients with tumors associated with newly diagnosed unresectable, locally advanced pancreatic cancer were selected for treatment using the inventive method. Patients were injected intra-tumorally via percutaneous transabdominal administration (PTA) (either CT- or ultrasound-guided), or via endoscopic ultrasound (EUS)-guided fine needle injection administration with a pharmaceutical composition as described in Example 1. In particular, patients were administered one of three dose levels of the human TNF-α coding sequence-containing adenoviral vector described in Example 1 (4x10⁹, 4x10¹⁰, or 4x10¹¹ pu in 2 mL volume) over a 5.5 week therapeutic period. A single dose of the pharmaceutical composition was administered to the tumor once weekly for five weeks of the therapeutic period. For PTA administration, the entire 2 mL volume of the dose of the pharmaceutical composition was administered with a single needle pass at a single tumor site at each administration session. For EUS-guided delivery, the 2 mL volume of the dose of the pharmaceutical composition was administered via up to four needle passes and four injections in a fan-like pattern at the tumor site.

Ionizing radiation was administered to each patient beginning at about 4 hours after administration of the pharmaceutical composition on day 1 of the therapeutic period. A total radiation dose of 50.4 Gy was delivered via 28 fractions of 1.8 Gy each. Each radiation fraction was administered for five consecutive days, and not administered for two days, for each week of the therapeutic period. On the first day of each week of the therapeutic period, 5-fluoruracil (5-FU) was administered prior to that day's radiation treatment. 5-FU was administered as a continuous infusion of 200 mg/m²/day for five consecutive days of each week of the therapeutic period, resulting in a total weekly infusion time of about 96 hours. Administration of 5-FU continued throughout the duration of radiation therapy, and was discontinued following conclusion of radiation therapy.

The above-described treatment was well tolerated. At four weeks after completion of the therapeutic period, approximately 56% of the patients exhibited decreases in levels of the pancreatic tumor-associated antigen CA19-9, while 16% of treated patients exhibited stable CA19-9 levels. Results of CA19-9 testing indicated some correlation between increased CA19-9 levels and disease progression. At three months after completion of the therapeutic period, inhibition of overall disease progression (defined as an objective tumor response or stabilized disease plus no metastatic disease) was observed in 25% of the patients treated with the 4x10⁹ pu dose of the pharmaceutical composition, and in 50% of the patients treated with the 4x10¹⁰ pu dose of the pharmaceutical composition. Stabilized disease or reduction in tumor size was observed in 67% of the patients treated with 4x10⁹ pu dose of the pharmaceutical composition and in 70% of the patients treated with 4x10¹⁰ pu dose of the pharmaceutical composition.

These results prove the effectiveness of the inventive method to treat unresectable, locally advanced pancreatic cancer.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Of course, variations of those preferred embodiments will become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subj ect matter recited in the claims appended hereto as permitted by applicable law. Moreover, any combination of the above-described elements in all possible variations thereof is encompassed by the invention unless otherwise indicated herein or otherwise clearly contradicted by context.

In particular, the afore described invention relates to the following preferred embodiments:
1. Embodiment: A method for treating unresectable, locally advanced pancreatic cancer (LAPC) in a human comprising:
   (a) administering to the human a dose of a pharmaceutical composition comprising (i) a pharmaceutically acceptable carrier and (ii) an adenoviral vector comprising a nucleic acid sequence encoding a human TNF-α and operably linked to a promoter, wherein the dose comprises about 4 X 10⁷ to about 4 x 10¹² particle units (pu) of adenoviral vector, at least once in a therapeutic period comprising up to about 10 weeks,
   (b) administering a dose of ionizing radiation to the human over the duration of the therapeutic period, and
   (c) administering a dose of one or more chemotherapeutics to the human over the duration of the therapeutic period, whereby the unresectable, locally advanced pancreatic cancer in the human is treated.
2. Embodiment: A method for treating locally advanced resectable esophageal cancer in a human comprising:
   (a) administering to the human a dose of a pharmaceutical composition comprising (i) a pharmaceutically acceptable carrier and (ii) an adenoviral vector comprising a nucleic acid sequence encoding a human TNF-α and operably linked to a promoter, wherein the dose comprises about 4 X 10⁷ to about 4 x 10¹² particle units (pu) of adenoviral vector, at least once in a therapeutic period comprising up to about 10 weeks,
   (b) administering a dose of ionizing radiation to the human over the duration of the therapeutic period, and
   (c) administering a dose of one or more chemotherapeutics to the human over the duration of the therapeutic period, whereby the esophageal cancer in the human is treated.
3. Embodiment: A method for treating locally advanced resectable rectal cancer in a human comprising:
   (a) administering to the human a dose of a pharmaceutical composition comprising (i) a pharmaceutically acceptable carrier and (ii) an adenoviral vector comprising a nucleic acid sequence encoding a human TNF-α and operably linked to a promoter, wherein the dose comprises about 4 X 10⁷ to about 4 x 10¹² particle units (pu) of adenoviral vector, at least once in a therapeutic period comprising up to about 10 weeks,
   (b) administering a dose of ionizing radiation to the human over the duration of the therapeutic period, and
   (c) administering a dose of one or more chemotherapeutics to the human over the duration of the therapeutic period, whereby the locally advanced rectal cancer in the human is treated.
4. Embodiment: The method of any of Embodiments 1-3, wherein the adenoviral vector, the dose of ionizing radiation, and the dose of the one or more chemotherapeutics are administered to the human concurrently over the duration of the therapeutic period.
5. Embodiment: The method of any of Embodiments 1-4, wherein the pharmaceutical composition contacts a tumor, and tumor cells within the tumor are destroyed.
6. Embodiment: The method of Embodiment 5, wherein the size of the tumor is reduced.
7. Embodiment: The method of any of Embodiments 1-6, wherein the adenoviral vector is replication-deficient.
8. Embodiment: The method of Embodiment 7, wherein the promoter is an inducible promoter.
9. Embodiment: The method of Embodiment 8, wherein the promoter is chemo-inducible.
10. Embodiment: The method of Embodiment 8, wherein the promoter is radiation-inducible.
11. Embodiment: The method of Embodiment 10, wherein the radiation-inducible promoter is the EGR-1 promoter.
12. Embodiment: The method of any of Embodiments 5-11, wherein a dose of pharmaceutical composition is administered to the tumor about once a week in a therapeutic period comprising about three to seven weeks.
13. Embodiment: The method of any of Embodiments 5-12, wherein the therapeutic period comprises up to six weeks, and the method comprises administering one dose of pharmaceutical composition in each week of the therapeutic period.
14. Embodiment: The method of any of Embodiments 5-13, wherein the pharmaceutical composition is administered to the tumor by percutaneous transabdominal administration, endoscopic administration, or by endoscopic ultrasound-guided administration.
15. Embodiment: The method of any of Embodiments 5-14, wherein the pharmaceutical composition is administered to the tumor via 2-5 injections.
16. Embodiment: The method of any of Embodiments 1-15, wherein the dose of ionizing radiation comprises about 40 Gray (Gy) to about 60 Gy administered over the duration of the therapeutic period.
17. Embodiment: The method of Embodiment 16, wherein the method comprises administering one fraction of the dose of ionizing radiation per day for five days in each week of the therapeutic period.
18. Embodiment: The method of Embodiment 17, wherein the fraction of the dose of ionizing radiation comprises about 1-2 Gy.
19. Embodiment: The method of Embodiment 17 or Embodiment 18, wherein the fraction of the dose of ionizing radiation is administered to the human at least four hours after administration of the pharmaceutical composition.
20. Embodiment: The method of any of Embodiments 1-19, wherein the one or more chemotherapeutics are selected from the group consisting of adriamycin, asparaginase, bleomycin, busulphan, cisplatin, carboplatin, carmustine, capecitabine, chlorambucil, cytarabine, cyclophosphamide, camptothecin, dacarbazine, dactinomycin, daunorubicin, dexrazoxane, docetaxel, doxorubicin, etoposide, floxuridine, fludarabine, fluorouracil, gemcitabine, hydroxyurea, idarubicin, ifosfamide, irinotecan, lomustine, mechlorethamine, mercaptopurine, melphalan, methotrexate, mitomycin, mitotane, mitoxantrone, nitrosurea, paclitaxel, pamidronate, pentostatin, plicamycin, procarbazine, rituximab, streptozocin, teniposide, thioguanine, thiotepa, vinblastine, vincristine, vinorelbine, taxol, transplatinum, and 5-fluorouracil.
21. Embodiment: The method of Embodiment 19, wherein the one or more chemotherapeutics are selected from the group consisting of 5-fluorouracil, capecitabine, and cisplatin, or combinations thereof.
22. Embodiment: The method of Embodiment 21, wherein the chemotherapeutic is 5-fluoruracil.
23. Embodiment: The method of Embodiment 22, wherein the dose of 5-fluorouracil comprises about 100 mg/m²/day to about 300 mg/m²/day.
24. Embodiment: The method of Embodiment 21, wherein the chemotherapeutic is capecitabine.
25. Embodiment: The method of Embodiment 24, wherein the dose of capecitabine comprises about 800 mg/m²/day to about 1000 mg/m²/day.
26. Embodiment The method of Embodiment 21, wherein the therapeutic period comprises five weeks and the method comprises administering about 50-100 mg/m² of cisplatin and about 900-1100 mg/m² of 5-fluorouracil beginning on each of two separate days of the therapeutic period.
27. Embodiment: The method of Embodiment 26, wherein the method comprises administering about 75 mg/m² of cisplatin and about 1000 mg/m² of 5-fluoruracil beginning on day 1 and day 29 of the therapeutic period.
28. Embodiment: The method of any of Embodiments 20-27, wherein the one or more chemotherapeutic agents are administered within 24 hours of the administration of the dose of ionizing radiation.
29. Embodiment: The method of any of Embodiments 5-28, wherein the method further comprises surgical resection of the tumor after completion of the therapeutic period.
30. Embodiment: The method of Embodiment 29, wherein the method comprises surgical resection of the tumor 4-10 weeks after completion of the therapeutic period.
31. Embodiment: The method of Embodiment 30, wherein the method comprises surgical resection of the tumor 3-6 weeks after completion of the therapeutic period.
32. Embodiment: The method of any of Embodiments 29-31, wherein the method comprises administering adjuvant chemotherapy following surgical resection of the tumor.
33. Embodiment: The method of Embodiment 32, wherein the adjuvant chemotherapy is administered 2-4 weeks following surgical resection of the tumor.
34. Embodiment: The method of Embodiment 3, wherein the stage of the locally advanced resectable rectal cancer is T3 or T4.
35. Embodiment: The method of Embodiment 2, wherein the stage of the locally advanced resectable esophageal cancer is stage III or stage IV.
36. Embodiment: The method of any of Embodiments 1-35, which method further comprises administering a dose of an H2 blocker or a proton pump inhibitor.
37. Embodiment: The method of any of Embodiments 16-19, wherein the method further comprises administering a booster dose of ionizing radiation comprising about 5-10 Gy to the tumor.
38. Embodiment: The method of any of Embodiments 29-31, whereby the treatment of the locally advanced resectable esophageal cancer or the locally advanced rectal cancer is evidenced by the lack of tumor tissue in the resected tissue.
39. Embodiment: The method of any of Embodiments 29-31, whereby the treatment of the locally advanced resectable rectal cancer is evidenced by the resected tumor comprising a necrosis percentage of at least about 90%.
40. Embodiment: The method of Embodiment 5, whereby the treatment of the locally advanced resectable rectal cancer is evidenced by at least a 10% decrease in the carcinoembryonic antigen (CEA) level at 4-12 weeks after completion of the therapeutic period as compared to the CEA level before the therapeutic period.
41. Embodiment: The method of Embodiment 5, whereby the treatment of the unresectable, locally advanced pancreatic cancer is evidenced by about a 25% to 50% reduction in the size of the tumor for at least four weeks after completion of therapeutic period as compared to the area of the tumor before the therapeutic period.
42. Embodiment: The method of Embodiment 5, whereby the treatment of the unresectable, locally advanced pancreatic cancer is evidenced by at least about a 50% reduction in the size of the tumor for at least four weeks after completion of the therapeutic period as compared to the area of the tumor before the therapeutic period.
43. Embodiment: The method of Embodiment 5, whereby the treatment of the unresectable, locally advanced pancreatic cancer is evidenced by disappearance of the tumor that is sustained for at least four weeks.
44. Embodiment: The method of Embodiment 5, whereby the treatment of the unresectable, locally advanced pancreatic cancer is evidenced by at least a 10% decrease in the CA19-9 tumor antigen level at 4-12 weeks after completion of the therapeutic period as compared to the CA19-9 tumor antigen level before the therapeutic period.
45. Embodiment: The method of Embodiment 5, whereby the treatment of the unresectable, locally advanced pancreatic cancer is evidenced by the conversion of the surgical stage of the tumor from unresectable to resectable at 12 weeks after completion of the therapeutic period.
46. Embodiment: The method of Embodiment 5, whereby the treatment of the unresectable, locally advanced pancreatic cancer is evidenced by at least a 50% decrease in pain intensity as assigned by the Clinical Benefit Response Criteria for any consecutive four-week period in the 12 weeks after completion of the therapeutic period as compared to the pain intensity before the therapeutic period.
47. Embodiment: The method of Embodiment 5, whereby the treatment of the unresectable, locally advanced pancreatic cancer is evidenced by at least a 50% decrease in analgesic consumption as assigned by the Clinical Benefit Response Criteria for any consecutive four week period in the 12 weeks after completion of the therapeutic period as compared to the analgesic consumption before the therapeutic period.
48. Embodiment: The method of Embodiment 5, whereby the treatment of the unresectable, locally advanced pancreatic cancer is evidenced by at least a 20 point increase in the Karnofsky Performance Scale score as assigned by the Clinical Benefit Response Criteria for any consecutive four week period in the 12 weeks after completion of the therapeutic period as compared to the score before the therapeutic period.
49. Embodiment: A method of treating glioma in a human comprising:
   (a) administering to the human a dose of a pharmaceutical composition comprising (i) a pharmaceutically acceptable carrier and (ii) an adenoviral vector comprising a nucleic acid sequence encoding a human TNF-α and operably linked to a promoter, wherein the dose comprises about 4 x 10⁷ to about 4 x 10¹² particle units (pu) of adenoviral vector, at least once in a therapeutic period comprising up to about 10 weeks, and
   (b) administering a dose of ionizing radiation to the human over the duration of the therapeutic period, whereby the glioma in the human is treated..
50. Embodiment: The method of Embodiment 49, wherein the glioma is recurrent glioblastoma multiforme.
51. Embodiment: The method of Embodiment 49 or Embodiment 50, wherein the dose of ionizing radiation comprises about 10 Gray (Gy) to about 40 Gy administered over the duration of the therapeutic period.
52. Embodiment: The method of Embodiment 51, wherein the method comprises administering one fraction of the dose of ionizing radiation per day.
53. Embodiment: The method of Embodiment 52, wherein the fraction of the dose of ionizing radiation comprises about 4-5 Gy.
54. Embodiment: The method of Embodiment 52 or Embodiment 53, wherein the fraction of the dose of ionizing radiation is administered to the human at least three days after administration of the pharmaceutical composition.

## Claims

1. Use of an adenoviral vector, comprising a nucleic acid sequence encoding a human TNF-α and being operably linked to a promoter, in the manufacturing of a pharmaceutical composition for treating locally advanced resectable esophageal cancer in a human, wherein
(a) a dose of the pharmaceutical composition comprising (i) a pharmaceutically acceptable carrier and (ii) about 4 x 10⁷ to about 4 x 10¹² particle units (pu) of said adenoviral vector is to be administered to the human at least once in a therapeutic period comprising up to about 10 weeks;
(b) a dose of ionizing radiation is to be administered to the human over the duration of the therapeutic period; and
(c) a dose of one or more chemotherapeutics is to be administered to the human over the duration of the therapeutic period.

2. The use of claim 1, wherein the adenoviral vector, the dose of ionizing radiation, and the dose of the one or more chemotherapeutics are to be administered to the human concurrently over the duration of the therapeutic period.

3. The use of claim 1 or claim 2, wherein the pharmaceutical composition is to be contacted with a tumor, such that tumor cells within the tumor are destroyed.

4. The use of claim 3, wherein the pharmaceutical composition is to be contacted with a tumor, such that the size of the tumor is reduced.

5. The use of any of claims 1-4, wherein the adenoviral vector is replication-deficient.

6. The use of any of claims 1-5, wherein the promoter is an EGR-1 promoter.

7. The use of any of claims 3-6, wherein a dose of pharmaceutical composition is to be administered to the tumor about once a week in a therapeutic period comprising about three to seven weeks.

8. The use of any of claims 3-7, wherein the pharmaceutical composition is to be administered to the tumor via 2-5 injections.

9. The use of any of claims 1-8, wherein the dose of ionizing radiation comprises about 40 Gray (Gy) to about 60 Gy and is to be administered over the duration of the therapeutic period.

10. The use of any of claims 1-9, wherein the one or more chemotherapeutics are selected from the group consisting of adriamycin, asparaginase, bleomycin, busulphan, cisplatin, carboplatin, carmustine, capecitabine, chlorambucil, cytarabine, cyclophosphamide, camptothecin, dacarbazine, dactinomycin, daunorubicin, dexrazoxane, docetaxel, doxorubicin, etoposide, floxuridine, fludarabine, fluorouracil, gemcitabine, hydroxyurea, idarubicin, ifosfamide, irinotecan, lomustine, mechlorethamine, mercaptopurine, melphalan, methotrexate, mitomycin, mitotane, mitoxantrone, nitrosurea, paclitaxel, pamidronate, pentostatin, plicamycin, procarbazine, rituximab, streptozocin, teniposide, thioguanine, thiotepa, vinblastine, vincristine, vinorelbine, taxol, transplatinum, and 5-fluorouracil.

11. The use of claim 10, wherein the one or more chemotherapeutics are selected from the group consisting of 5-fluorouracil, capecitabine, and cisplatin, or combinations thereof.

12. The use of claim 10 or claim 11, wherein the one or more chemotherapeutic agents are to be administered within 24 hours of the administration of the dose of ionizing radiation.

13. The use of any of claims 3-12, wherein further the tumor is to be surgically resected after completion of the therapeutic period.

14. The use of claim 13, wherein the tumor is to be surgically resected 4-10 weeks after completion of the therapeutic period.

15. The use of claim 13 or claim 14, wherein adjuvant chemotherapy is to be administered following surgical resection of the tumor.

16. The use of claim 15, wherein the adjuvant chemotherapy is to be administered 2-4 weeks following surgical resection of the tumor.

17. The use of claim 16, wherein the locally advanced resectable esophageal cancer is stage III or stage IV.

18. The use of any of claims 1-17, whereby the treatment of the locally advanced resectable esophageal cancer is to be evidenced by the lack of tumor tissue in the resected tissue.
